# EUROPEAN PATENT SPECIFICATION

(11) **EP 3 288 969 B1**
(45) Date of publication and mention of the grant of the patent: **19.02.2020**
(21) Application number: 16721210.9
(22) Date of filing: 26.04.2016
(51) Int. Cl.: C07K 14/705

(54) **NUCLEIC ACID CONSTRUCT FOR EXPRESSING MORE THAN ONE CHIMERIC ANTIGEN RECEPTOR**
NUKLEINSÄUREKONSTRUKT ZUR EXPRESSION VON MEHRERE CHIMÄRE ANTIGENREZEPTOREN
CONSTRUCTION D'ACIDE NUCLÉIQUE POUR L'EXPRESSION DE PLUSIEURS RECEPTEURS D'ANTIGENES CHIMERIQUES.

(30) Priority: 27.04.2015 GB 201507111
(43) Date of publication of application: 07.03.2018
(73) Proprietor: UCL Business Ltd, London W1T 4TP (GB)
(72) Inventor: PULÉ, Martin, London W12 7RP (GB); CORDOBA, Shaun, London W12 7RP (GB)
(74) Representative: D Young & Co LLP
(86) International application number: PCT/GB2016/051161
(87) International publication number: WO 2016/174405

(56) References cited:
- WO-A1-2013/059593
- WO-A1-2013/123061
- WO-A1-2014/127261
- WO-A1-2015/052538
- WO-A1-2015/075468
- US-A1- 2014 271 635
- ZAKARIA GRADA ET AL: "TanCAR: A Novel Bispecific Chimeric Antigen Receptor for Cancer Immunotherapy", MOLECULAR THERAPY-NUCLEIC ACIDS, vol. 2, no. 7, 1 July 2013 (2013-07-01), page e105, XP055152438, DOI: 10.1038/mtna.2013.32 cited in the application
- MAY C.I. VAN SCHALKWYK ET AL: "Design of a Phase I Clinical Trial to Evaluate Intratumoral Delivery of ErbB-Targeted Chimeric Antigen Receptor T-Cells in Locally Advanced or Recurrent Head and Neck Cancer", HUMAN GENE THERAPY CLINICAL DEVELOPMENT, vol. 24, no. 3, 1 September 2013 (2013-09-01), pages 134-142, XP055285985, ISSN: 2324-8637, DOI: 10.1089/humc.2013.144
- SHANNON L. MAUDE ET AL: "Chimeric Antigen Receptor T Cells for Sustained Remissions in Leukemia", NEW ENGLAND JOURNAL OF MEDICINE, vol. 371, no. 16, 16 October 2014 (2014-10-16), pages 1507-1517, XP055188099, ISSN: 0028-4793, DOI: 10.1056/NEJMoa1407222
- M. SADELAIN ET AL: "The Basic Principles of Chimeric Antigen Receptor Design", CANCER DISCOVERY, vol. 3, no. 4, 1 April 2013 (2013-04-01), pages 388-398, XP055287277, US ISSN: 2159-8274, DOI: 10.1158/2159-8290.CD-12-0548
- ELEANOR J. CHEADLE ET AL: "CAR T cells: driving the road from the laboratory to the clinic", IMMUNOLOGICAL REVIEWS, vol. 257, no. 1, 13 December 2013 (2013-12-13), pages 91-106, XP055177342, ISSN: 0105-2896, DOI: 10.1111/imr.12126
- SHAUN CORDOBA ET AL: "Chimeric Antigen Receptor Logical AND Gate Based on CD45/CD148 Phosphatases", MOLECULAR THERAPY, vol. 22, no. Suppl. 1, 1 May 2014 (2014-05-01), page S59, XP055170616, ISSN: 1525-0016

## Description

### FIELD OF THE INVENTION

The present invention relates to constructs and approaches for expressing more than one chimeric antigen receptor (CAR) at the surface of a cell. The cell may be capable of specifically recognising a target cell, due to a differential pattern of expression (or non-expression) of two or more antigens by the target cell. The constructs of the invention enable modulation of the relative expression of the two or more CARs at the cell surface by a method involving co-expression of the CARs from a single vector.

### BACKGROUND TO THE INVENTION

A number of immunotherapeutic agents have been described for use in cancer treatment, including therapeutic monoclonal antibodies (mAbs), immunoconjugated mAbs, radioconjugated mAbs and bi-specific T-cell engagers.

Typically these immunotherapeutic agents target a single antigen: for instance, Rituximab targets CD20; Myelotarg targets CD33; and Alemtuzumab targets CD52.

However, it is relatively rare for the presence (or absence) of a single antigen effectively to describe a cancer.

A particular problem in the field of oncology is provided by the Goldie-Coldman hypothesis: which describes that the sole targeting of a single antigen may result in tumour escape by modulation of said antigen due to the high mutation rate inherent in most cancers. This modulation of antigen expression may reduce the efficacy of known immunotherapeutics.

Tumour heterogeneity describes the observation that different tumour cells can show distinct morphological and phenotypic profiles, including cellular morphology, gene expression, metabolism, proliferation and metastatic potential. This phenomenon occurs both between tumours and within tumours. Tumour heterogeneity has been observed in leukaemias, breast, prostate, colon, brain, head and neck, bladder and gynecological cancers, for example. Tumour heterogeneity may result in the expression of different antigens on the surface of cells within a tumour or between tumours. This heterogeneity of cancer cells introduces significant challenges in designing effective treatment strategies.

There is thus a need for immunotherapeutic agents which are capable of targeting more than one antigen to reflect the complex pattern of marker expression that is associated with many cancers.

### Chimeric Antigen Receptors (CARs)

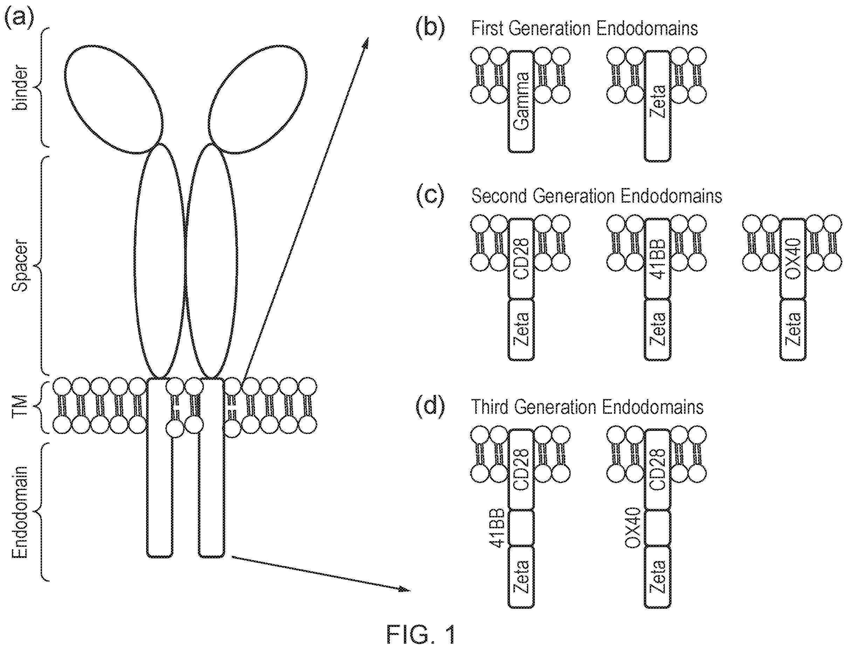

Chimeric antigen receptors are proteins which graft the specificity of a monoclonal antibody (mAb) to the effector function of a T-cell. Their usual form is that of a type I transmembrane domain protein with an antigen recognizing amino terminus, a spacer, a transmembrane domain all connected to a compound endodomain which transmits T-cell survival and activation signals (see Figure 1A).

The most common form of these molecules are fusions of single-chain variable fragments (scFv) derived from monoclonal antibodies which recognize a target antigen, fused via a spacer and a trans-membrane domain to a signaling endodomain. Such molecules result in activation of the T-cell in response to recognition by the scFv of its target. When T cells express such a CAR, they recognize and kill target cells that express the target antigen. Several CARs have been developed against tumour associated antigens, and adoptive transfer approaches using such CAR-expressing T cells are currently in clinical trial for the treatment of various cancers.

It has been observed that using a CAR approach for cancer treatment, tumour heterogeneity and immunoediting can cause escape from CAR treatment. For example, in the study described by Grupp et al (2013; New Eng. J. Med 368:1509-1518) CAR-modified T cell approach was used for the treatment of acute lymphoid leukemia. It was found that one patient of the anti-CD19 CAR trial relapsed with CD19-negative disease two months after treatment.

### Expression of two CAR binding specificities

Bispecific CARs known as tandem CARs or TanCARs have been developed in an attempt to target multiple cancer specific markers simultaneously. In a TanCAR, the extracellular domain comprises two antigen binding specificities in tandem, joined by a linker. The two binding specificities (scFvs) are thus both linked to a single transmembrane portion: one scFv being juxtaposed to the membrane and the other being in a distal position.

Grada et al (2013, Mol Ther Nucleic Acids 2:e105 describes a TanCAR which includes a CD19-specific scFv, followed by a Gly-Ser linker and then a HER2-specific scFv. The HER2-scFv was in the juxta-membrane position, and the CD19-scFv in the distal position. The Tan CAR was shown to induce distinct T cell reactivity against each of the two tumour restricted antigens. This arrangement was chosen because the respective lengths of HER2 (632 aa/125Å) and CD19 (280aa, 65Å) lends itself to that particular spatial arrangement. It was also known that the HER2 scFv bound the distal-most 4 loops of HER2.

The problem with this approach is that the juxta-membrane scFv may be inaccessible due to the presence of the distal scFv, especially which it is bound to the antigen. In view of the need to choose the relative positions of the two scFvs in view of the spatial arrangement of the antigen on the target cell, it may not be possible to use this approach for all scFv binding pairs. Moreover, it is unlikely that the TanCar approach could be used for more than two scFvs, a TanCAR with three or more scFvs would be a very large molecule and the scFvs may well fold back on each other, obscuring the antigen-binding sites. It is also doubtful that antigen-binding by the most distal scFv, which is separated from the transmembrane domain by two or more further scFvs, would be capable of triggering T cell activation.

An alternative option would be to express two or more CARs from the same vector.

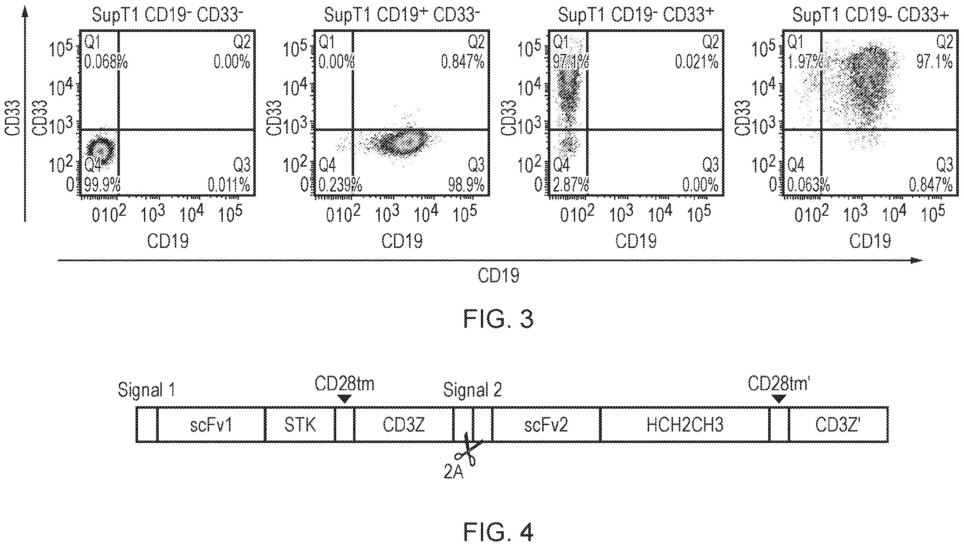

Various different methods have been developed to allow co-expression of two proteins from a single vector (see Figure 3).

Initial attempts used two different promoters within the same cassette. This results in two separate transcripts each of which code for a separate protein. This is a difficult approach for a number of reasons. A key problem is "promoter interference" whereby one promoter dominates and causes silencing of the second promoter. In addition, different promoters work differently in different cellular contexts and this makes consistent "tuning" of the relative expression of each transgene difficult to achieve.

An alternative approach is to use an Internal Ribosome Entry sequence (IRES). Here, a single transcript is generated. The IRES sequence in the transcript is placed between the open reading frames for the two transgenes and mimics an mRNA cap structure. Hence, the ribosome either initiates translation at the 5' cap or the IRES resulting in expression of two separate proteins. A key limitation with this approach is the inability to control relative expression. The 3' transcript is typically expressed less than the 5' one, but the ratio of expression is difficult to predict and tune.

A further approach has been provided following characterization of the role of foot-and-mouth-disease virus (FMDV) 2A peptide in allowing FMDV (and related viruses) to express multiple proteins from a single open reading frame (ORF) (Donnelly et al; J. Gen. Virol.; 82, 1027-1041 (2001)). The 2A peptide (and homologs) cleaves at very high efficiency immediately after translation of the ORF, enabling the expression of multiple peptides from a single ORF. A problem with the use of the 2A peptide to cleave between different peptides in the same ORF is that expression is limited to a 1:1 ratio.

The relative expression of different tumour antigens is highly variable. In order to tailor recognition of a tumour cell by a cell expressing two or more CARs, it would be highly advantageous if the relative expression of the CARs at the cell surface reflected the relative expression of the antigens on the tumour cell.

Thus there is a need for alternative methods for expressing more than one CAR from a single vector which enable such "tailoring" of antigen recognition.

### SUMMARY OF ASPECTS OF THE INVENTION

The present inventors have developed a panel of "logic-gated" chimeric antigen receptor pairs which, when expressed by a cell, such as a T cell, are capable of detecting a particular pattern of expression of at least two target antigens. If the at least two target antigens are arbitrarily denoted as antigen A and antigen B, the three possible options are as follows:
"OR GATE" - T cell triggers when either antigen A or antigen B is present on the target cell
"AND GATE" - T cell triggers only when both antigens A and B are present on the target cell
"AND NOT GATE" - T cell triggers if antigen A is present alone on the target cell, but not if both antigens A and B are present on the target cell

Engineered T cells expressing these CAR combinations can be tailored to be exquisitely specific for cancer cells, based on their particular expression (or lack of expression) of two or more markers.

The present inventors have also found that, when a CAR is co-expressed with a second CAR as a polyprotein which after translation is subsequently cleaved to separate the two CARs, it is possible to modulate the relative cell surface expression of the two CARs by reducing trafficking to the cell surface of one or both CAR(s) and/or by reducing its half-life at the cell surface. This need not be limited to a pair of CARs, but may be used to allow control of the relative expression of multiple proteins initially translated as a polyprotein.

As used herein, 'polyprotein' refers to a polypeptide sequence translated from a single nucleic acid construct as a single entity, but which comprises polypeptide sequences which are subsequently separated and which function as discrete entities (e.g. separate CARs).

The present invention relates to an OR logic gate, in which the relative expression of the two CARs is modulated by incorporation of an intracellular retention signal in one or both CAR(s) and/or by altering the signal peptide of one CAR in order to remove or replace hydrophobic amino acids.

Thus in a first aspect the present invention provides a nucleic acid construct comprising the following structure:

A-X-B

in which
A and B are nucleic acid sequences encoding a first chimeric antigen receptor (CAR) and a second CAR; and
X is a nucleic acid sequence which encodes a cleavage site,
wherein
the first CAR and second CAR recognise different antigens;
the first CAR and second CAR each comprise an activating endodomain; and wherein
   (a) the first CAR and/or second CAR comprises an intracellular retention signal selected from the following group: an endocytosis signal; a Golgi retention signal; a *trans-*Golgi network (TGN) recycling signal; an endoplasmic reticulum (ER) retention signal; and a lysosomal sorting signal; and/or
   (b) the signal peptide of the first or second CAR comprises one or more mutation(s) such that the signal peptide of the first CAR and second CAR differ in their number of hydrophobic amino acids and wherein one signal peptide comprises up to five more hydrophobic amino acids than the other signal peptide.

For (b) the mutated signal peptide may have fewer hydrophobic amino acids than the "wild-type" signal peptide sequence from which it is derived. The mutated signal peptide may have fewer hydrophobic amino acids than the signal peptide of the other CAR.

Each CAR may comprise:
(i) an antigen-binding domain;
(ii) a spacer; and
(iii) a trans-membrane domain.

The spacer of the first CAR may be different to the spacer of the second CAR. For example, the spacer of the first CAR may have a different length and/or configuration from the spacer of the second CAR, such that each CAR is tailored for recognition of its respective target antigen.

The two or more CARs may have portions of high homology, for example the transmembrane and/or intracellular signalling domains may be highly homologous. If the same or similar linkers are use for the two CARs, then they will also be highly homologous. In order to avoid homologous recombination between the sequences, portions of sequence encoding areas of high homology may be "codon-wobbled". Codon wobbling involves using alternative codons in regions of sequence encoding the same or similar amino acid sequences.

In a particular embodiment, one CAR binds CD19 and the other CAR binds CD20. In another embodiment, one CAR binds CD19 and the other CAR binds CD22.

The present invention relates to a method for modulating the relative expression of two (or more) CARs in an OR logic gate, by one or both of the following approaches (a) by incorporation of an intracellular retention signal in one or both CAR(s) and (b) by altering the signal peptide of one CAR in order to remove or replace hydrophobic amino acids.

For approach (a), the endodomain of the CAR may comprise the intracellular retention signal.

The intracellular retention signal may direct the CAR away from the secretory pathway and/or to a membrane-bound intracellular compartment such as a lysozomal, endosomal or Golgi compartment.

The intracellular retention signal may, for example, be a tyrosine-based sorting signal, a dileucine-based sorting signal, an acidic cluster signal, a lysosomal avoidance signal, an NPFX'(1,2)D-Type signal, a KDEL, a KKX'X' or a KX'KX'X' signal (wherein X' is any amino acid).

The intracellular retention signal may comprise a sequence selected from the group of: NPX'Y, YX'X'Z', [DE]X'X'X'L[LI], DX'X'LL, DP[FW], FX'DX'F, NPF, LZX'Z[DE], LLDLL, PWDLW, KDEL, KKX'X' or KX'KX'X';
wherein X' is any amino acid and Z' is an amino acid with a bulky hydrophobic side chain.

The intracellular retention signal may comprise any of the sequences shown in Tables 7 to 11.

The intracellular retention signal may comprise the Tyrosinase-related protein (TYRP)-1 intracellular retention signal. The intracellular retention signal may comprise the TYRP-1 intracellular domain. The intracellular retention signal may comprise the sequence NQPLLTD (SEQ ID No. 1).

The intracellular retention signal may comprise the Adenoviral E3/19K intracellular retention signal. The intracellular retention signal may comprise the E3/19K cytosolic domain. The intracellular retention signal may comprise the sequence KYKSRRSFIDEKKMP (SEQ ID No. 2); or DEKKMP (SEQ ID No. 3).

The intracellular retention signal may be proximal or distal to a transmembrane domain of the CAR.

For approach (b), the signal peptides of the first and second CAR are different. They may differ in their number of hydrophobic amino acids. One signal peptide may comprise one or more mutation(s) such that it has fewer hydrophobic amino acids either a) than the wild-type sequence from which it was derived; or b) than the other signal peptide.

The signal peptides may be different and the sequence of one signal peptide may be altered such that the hydrophobic amino acids are removed or replaced. Alternatively, the first signal peptide and the second signal peptide may be derivable from the same sequence, but one signal peptide may comprise one or more amino acid deletions/substitutions to remove/replace one or more hydrophobic amino acids compared to the other signal peptide.

The hydrophobic amino acid(s) removed or replaced may be selected from the group: Alanine (A); Valine (V); Isoleucine (I); Leucine (L); Methionine (M); Phenylalanine (P); Tyrosine (Y); Tryptophan (W) or the group Valine (V); Isoleucine (I); Leucine (L); and Tryptophan (W).

For approach (b) one signal peptide may comprise one, two, three, four or five mutations, such that it has one, two, three, four or five fewer hydrophobic amino acids than: the wild-type signal sequence from which it is derived and/or the other signal peptide.

In the nucleic acid construct of the present invention, the nucleic acid sequence of X may be a nucleic acid sequence encoding a self-cleaving peptide, a furin cleavage site or a Tobacco Etch Virus cleavage site.

The nucleic acid sequence of X may be a nucleic acid sequence encoding a 2A self-cleaving peptide from an aphtho- or a cardiovirus or a 2A-like peptide.

The nucleic acid construct may comprise a third nucleic acid sequence encoding a protein of interest (POI). The POI may be a transmembrane protein.

The POI may be selected from a list of: excitatory receptors such as 41BB, OX40, CD27, CD28 and related molecules; or inhibitory receptors such as PD1, CTLA4, LAIR1, CD22 and related molecules; or cytokine receptor molecules such as IL1R, IL2R, IL7R, IL15R and related molcules; or homing molecules such as N-CAM, V-CAM, L1-CAM, LFA-1, CDH1-3, Selectins or Integrins. The POI may be a third CAR.

The POI may be a synthetic protein such as a suicide gene or a marker gene.

The amount of a CAR which comprises an intracellular retention signal and/or which has an aleterd signal peptide which is expressed at the cell surface may be, for example, less than 90%, 70%, 50% or 30% compared to a CAR expressed from the same nucleic acid construct which does not comprise an intracellular retention signal and which has an unaltered signal peptide.

The invention also provides a nucleic acid construct comprising the following structure:

A-X-B-Y-C

in which
A, B and C are nucleic acid sequences encoding a first, second and third polypeptides of interest (POls); and
X and Y are nucleic acid sequences which may be the same or different, each of which encodes a cleavage site,
wherein at least two of the POls are chimeric antigen receptors (CARs) which comprise an intracellular retention signal; and/or differ in the number of hydrophobic amino acids in their signal peptides.

In this embodiment, the at least two POls which are CARs and which comprise an intracellular retention signal may:
(a) comprise different intracellular retention signals; and/or
(b) have the intracellular retention signal located at a different position in the CAR,
such that when the nucleic acid is expressed in a cell, there is differential relative expression of the at least two CARs at the cell surface.

The third POI may be a polypeptide which enables selection of transduced cells and/or enables cells expressing the polypeptide to be deleted.

The third POI may be another CAR. Thus the present invention provides a nucleic acid construct which, when expressed by a cell, causes the cell to express two or more CARs at the cell surface, such that it is specifically stimulated by a cell, such as a tumour cell, bearing a distinct pattern of two or more antigens. The expression of each CAR at the cell surface may be tailored to reflect the expression of each antigen on the target cell.

In a second aspect the present invention provides a vector comprising a nucleic acid construct according to the first aspect of the invention.

The vector may be a retroviral vector or a lentiviral vector or a transposon.

In a third aspect the present invention provides a cell comprising a nucleic acid construct according to the first aspect of the invention or a vector according to the second aspect of the invention.

The cell may be an immune cell such as a T cell or a natural killer (NK) cell.

In a fourth aspect, the present invention provides a method for making a cell according to the third aspect of the invention, which comprises the step of introducing: a nucleic acid construct according to the first aspect of the invention or a vector according to the second aspect of the invention, into a cell *ex vivo.*

The cell may be part of or derived from a sample isolated from a subject.

The cell used in the method of the fourth aspect of the invention may be from a sample isolated from a patient, a related or unrelated haematopoietic transplant donor, a completely unconnected donor, from cord blood, differentiated from an embryonic cell line, differentiated from an inducible progenitor cell line, or derived from a transformed cell line.

In a fifth aspect the present invention provides a pharmaceutical composition comprising a plurality of cells according to the forth aspect of the invention. The composition may be an autologous T and/or NK cell composition.

Described herein is a method for treating and/or preventing a disease, which comprises the step of administering a pharmaceutical composition according to the fifth aspect of the invention to a subject.

The method may comprise the following steps:
(i) isolation of a T and/or NK cell-containing sample from a subject;
(ii) transduction or transfection of the T and/or NK cells with: a nucleic acid construct according to the first aspect of the invention or a vector according to the second aspect of the invention; and
(iii) administering the T and/or NK cells from (ii) to a the subject.

In a sixth aspect, the present invention provides a pharmaceutical composition according to the fifth aspect of the invention for use in treating and/or preventing a disease.

There is also described herein the use of a cell according to the third aspect of the invention in the manufacture of a medicament for treating and/or preventing a disease.

The invention further relates to method for modulating the relative cell surface expression of a first chimeric antigen receptor (CAR) expressed from a single nucleic acid construct with a second CAR by (a) including an intracellular retention signal in the nucleic acid sequence which encodes the first and/or second CAR(s) and/or (b) altering the nucleic acid sequence which encodes the signal peptide of one CAR in order to remove or replace one or more hydrophobic amino acids in comparison with the signal peptide of the other CAR. The nucleic acid construct may be as defined in the first aspect of the invention.

By providing two or more CARs on the surface of the T cell, it is possible to target multiple cancer markers simultaneously, providing better therapeutic efficacy for heterogeneic tumours and avoiding the problem of cancer escape.

Because the CARs are expressed on the surface of the T cell as separate molecules, this approach overcomes the spatial and accessibility issues associated with TanCARs. T-cell activation efficiency is also improved. As each CAR has its own spacer, it is possible to tailor the spacer and therefore the distance that the binding domain projects from the T cell surface and its flexibility etc to the particular target antigen. This choice is unfettered by the design considerations associated with TanCARs, i.e. that one CAR needs to be juxta-posed to the T cell membrane and one CAR needs to be distal, positioned in tandem to the first CAR.

By providing a single nucleic acid which encodes the two CARs separated by a cleavage site, it is possible to engineer T cells to co-express the two CARs using a simple single transduction procedure. A double transfection procedure could be used with CAR-encoding sequences in separate constructs, but this would be more complex and expensive and requires more integration sites for the nucleic acids. A double transfection procedure would also be associated with uncertainty as to whether both CAR-encoding nucleic acids had been transduced and expressed effectively. This is especially true for a multiple CAR approach where three or more CARs are introduced to the cell.

The inclusion of an intracellular retention signal in a CAR, or the alteration of the signal peptide of the CAR to reduce the number of hydrophobic amino acids, reduces the amount of the CAR expressed on the cell surface. As such, the relative expression level of two CARs expressed from a single construct can be modulated. As a CAR is only active at the cell surface, reducing the relative cell surface expression of the CAR also reduces its relative activity.

Thus the present invention provides a nucleic acid construct encoding an OR gate, in which the relative level of expression of the two or more CARs may be finely tuned, depending on the relative level of expression of the respective antigens on the target cell.

This invention can be extended to modulate the relative expression of three or more proteins expressed as a concatenated polypeptide, separated by cleavage sites and relative surface expression dictated by retention signals or signal peptides of differing activity.

### DESCRIPTION OF THE FIGURES

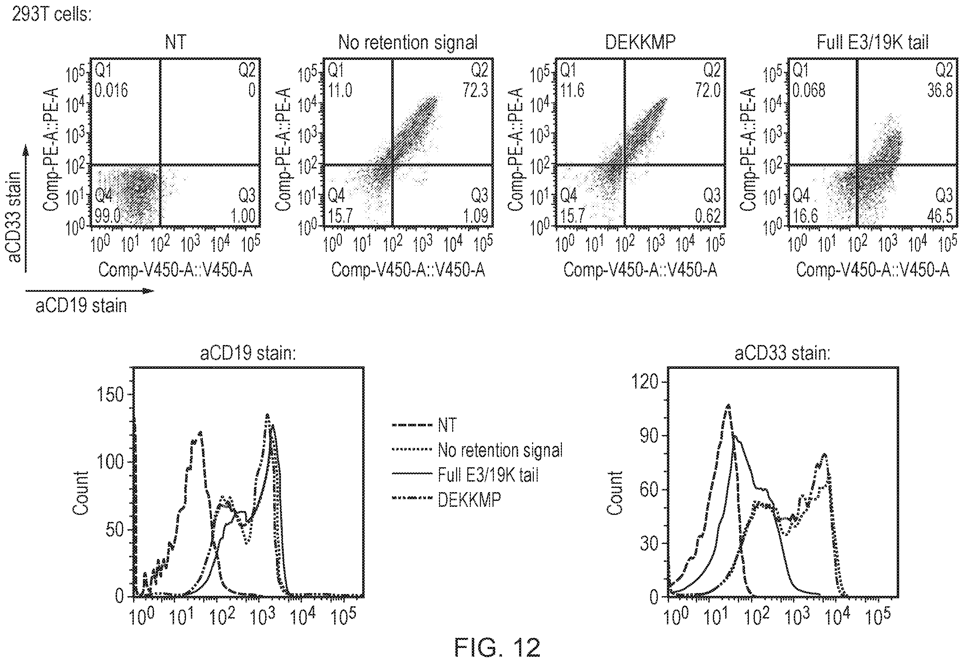

**Figure 1****:** (a) Generalized architecture of a CAR: A binding domain recognizes antigen; the spacer elevates the binding domain from the cell surface; the trans-membrane domain anchors the protein to the membrane and the endodomain transmits signals. (b) to (d): Different generations and permutations of CAR endodomains: (b) initial designs transmitted ITAM signals alone through FcεR1-γ or CD3ζ endodomain, while later designs transmitted additional (c) one or (d) two co-stimulatory signals in cis.

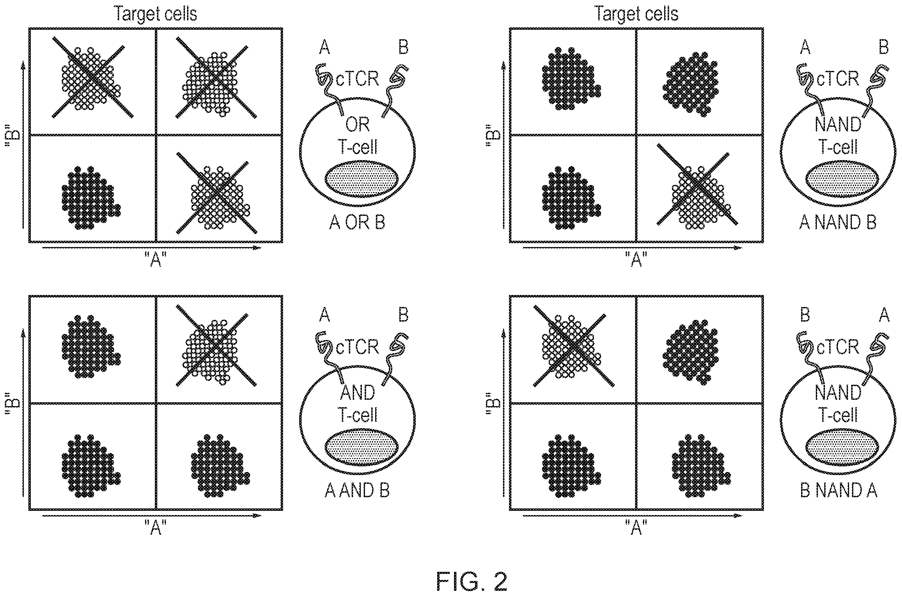

**Figure 2****:** Schematic diagram illustrating Logic Gates
   The invention relates to engineering T-cells to respond to logical rules of target cell antigen expression. This is best illustrated with an imaginary FACS scatter-plot. Target cell populations express both, either or neither of antigens "A" and "B". Different target populations (marked in red) are killed by T-cells transduced with a pair of CARs connected by different gates. With OR gated receptors, both single-positive and double-positive cells will be killed. With AND gated receptors, only double-positive target cells are killed. With AND NOT gating, double-positive targets are preserved while single-positive targets will be killed.
**Figure 3****:** Creation of target cell populations
   SupT1 cells were used as target cells. These cells were transduced to express either CD19, CD33 or both CD19 and CD33. Target cells were stained with appropriate antibodies and analysed by flow cytometry.
**Figure 4****:** Cassette design for an OR gate
   A single open reading frame provides both CARs with an in-frame FMD-2A sequence resulting in two proteins. Signal1 is a signal peptide derived from IgG1 (but can be any effective signal peptide). scFv1 is the single-chain variable segment which recognizes CD19 (but can be a scFv or peptide loop or ligand or in fact any domain which recognizes any desired arbitrary target). STK is the CD8 stalk but may be any suitable extracellular domain. CD28tm is the CD28 trans-membrane domain but can by any stable type I protein transmembrane domain and CD3Z is the CD3 Zeta endodomain but can be any endodomain which contains ITAMs. Signal2 is a signal peptide derived from CD8 but can be any effective signal peptide which is different in DNA sequence from signal1. scFv recognizes CD33 but as for scFv1 is arbitrary. HC2CH3 is the hinge-CH2-CH3 of human IgG1 but can be any extracellular domain which does not cross-pair with the spacer used in the first CAR. CD28tm' and CD3Z' code for the same protein sequence as CD28tm and CD3Z but are codon-wobbled to prevent homologous recombination.

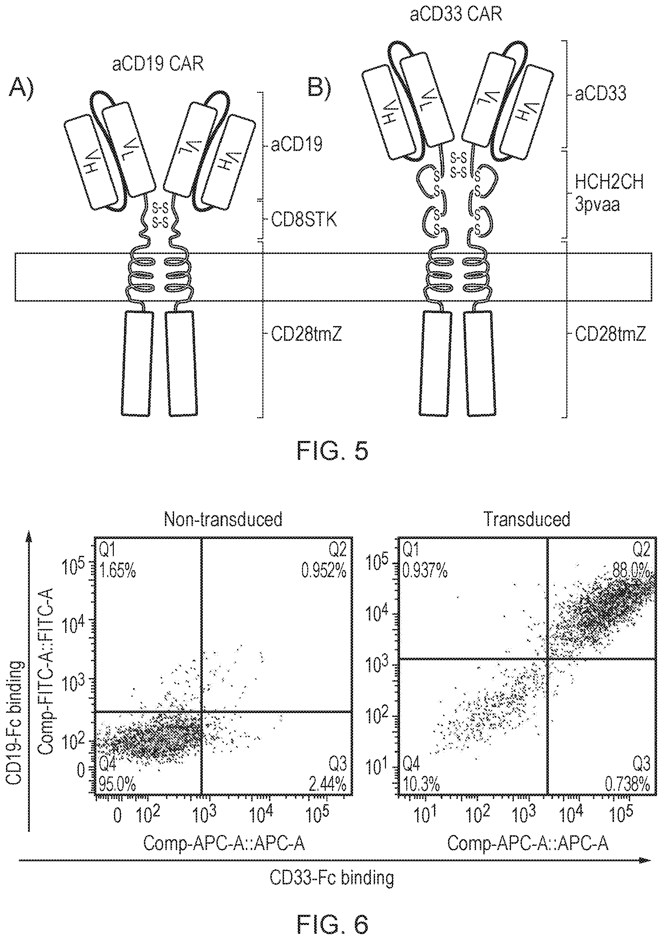

**Figure 5****:** Schematic representation of the chimeric antigen receptors (CARs) for an OR gate Stimulatory CARs were constructed consisting of either an N-terminal A) anti-CD19 scFv domain followed by the extracellular hinge region of human CD8 or B) anti-CD33 scFv domain followed by the extracellular hinge, CH2 and CH3 (containing a pvaa mutation to reduce FcR binding) region of human IgG1. Both receptors contain a human CD28 transmembrane domain and a human CD3 Zeta (CD247) intracellular domain. "S" depicts the presence of disulphide bonds.
**Figure 6****:** Expression data showing co-expression of both CARs on the surface of one T-cell.

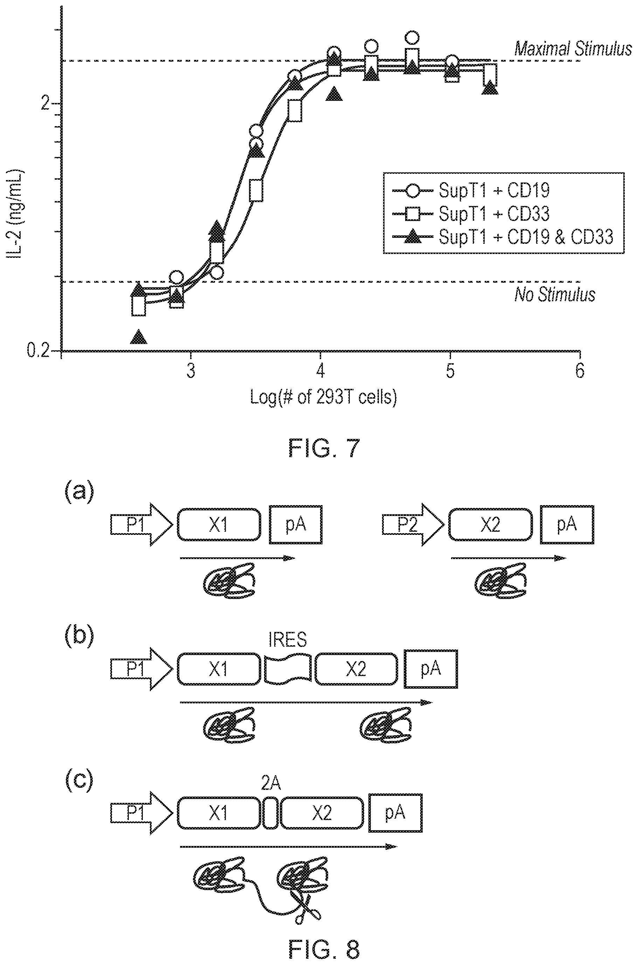

**Figure 7****:** Functional analysis of the OR gate
   Effector cells (5x10^4 cells) expressing the OR gate construct were co-incubated with a varying number of target cells and IL-2 was analysed after 16 hours by ELISA. The graph displays the average maximum IL-2 secretion from a chemical stimulation (PMA and lonomycin) of the effector cells alone and the average background IL-2 from effector cells without any stimulus from three replicates.
**Figure 8****:** Methods utilised to express different proteins from the same vector
   (a) Two different promoters within the same cassette result in two different transcripts which each give rise to separate proteins. (b) Use of an Internal Ribosome Entry sequence (IRES) leads to a single transcript which is translated into two separate proteins. (c) Use of the FMDV 2A peptide results in a single transcript, and a single polyprotein which rapidly cleaves into two separate proteins.

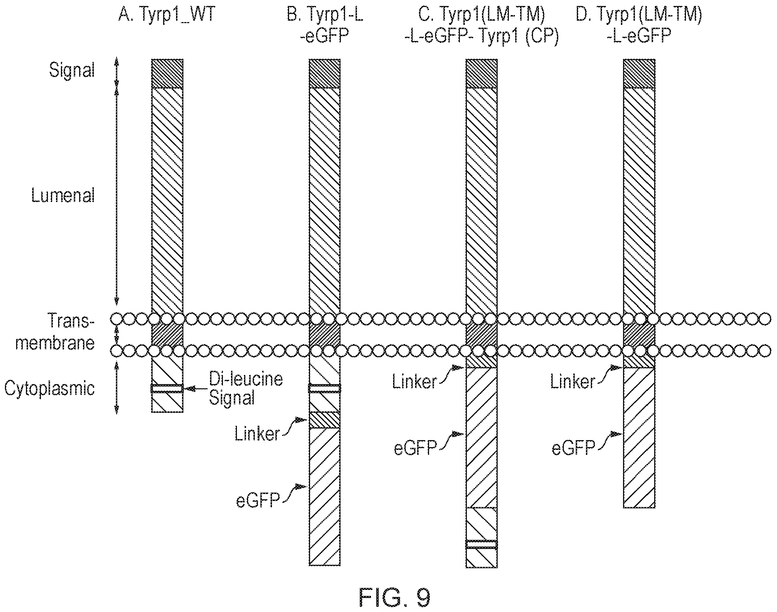

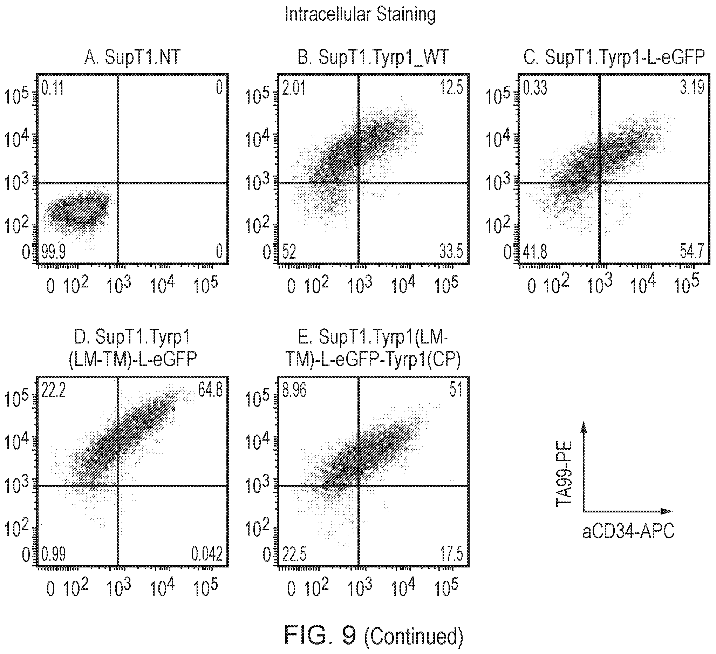

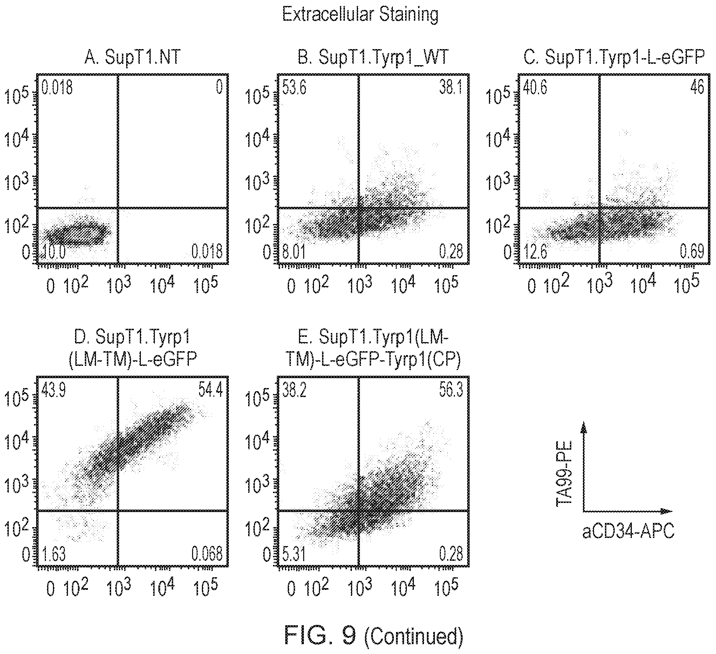

**Figure 9****:** TYRP1 endodomain is able to direct the retention of a transmembrane protein with a complex endodomain
   Tyrp1 is a type I transmembrane protein, 537aa long. The di-leucine motif, which retains the protein in the intracellular compartment, is indicated as a black rectangle on the cytoplasmic domain. (A) Tyrp1 (wt). Wild type Tyrp1 consists of a peptide signal, a luminal domain, a transmembrane domain, and a cytoplasmic domain. The cytoplasmic domain contains the di- leucine retention signal. (B) Tyrp1 (wt)-SG Linker- eGFP. This construct contains the wild type Tyrp1 simply fused to eGFP via a serine-glycine-glycine-glycine-serine linker. The Tyrp1-L-eGFP represents the cytoplasmic- proximal Tyrp1. (C) Tyrp1 Lumenal (LM)-Transmembrane (TM)- SG Linker- eGFP- Tyrp1 Cytoplasmic (CP). This construct constitutes the cytoplasmic- distal Tyrp1, since SG linker- eGFP interposes between the transmembrane and cytoplasmic domains. D: Tyrp1 Lumenal (LM)- Transmembrane (TM)-SG Linker- eGFP. This construct serves as the positive control, as the cytoplasmic domain containing the retention signal has been excluded. All constructs are co-expressed with IRES.CD34. Staining of transduced SupT1 cells is shown with intracellular and surface staining bottom left / right respectively.

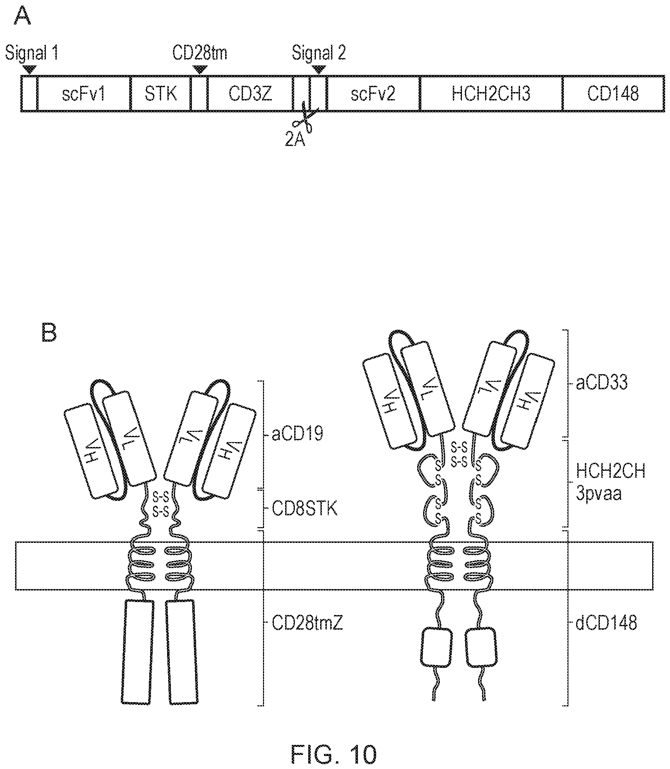

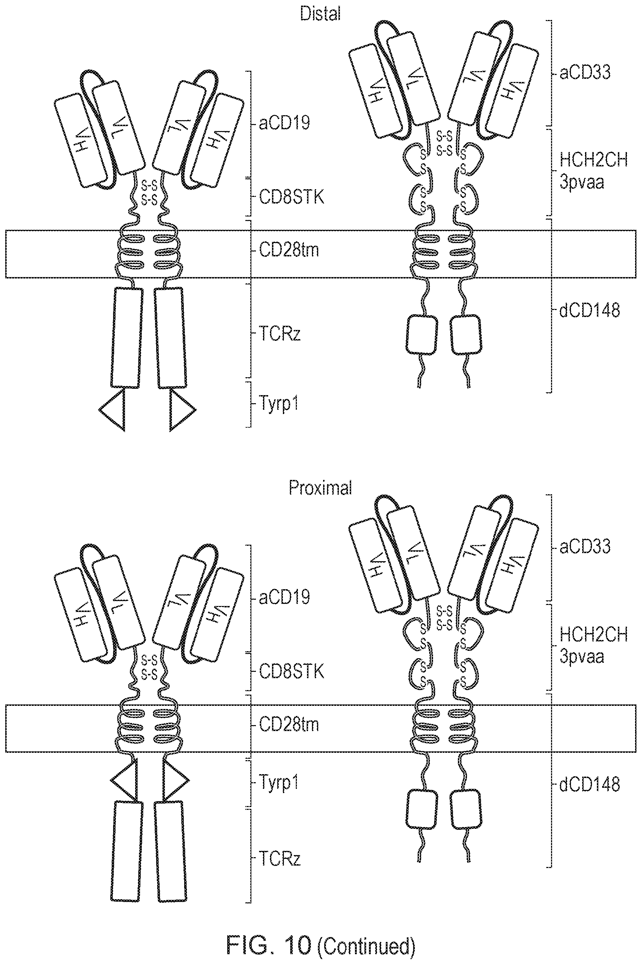

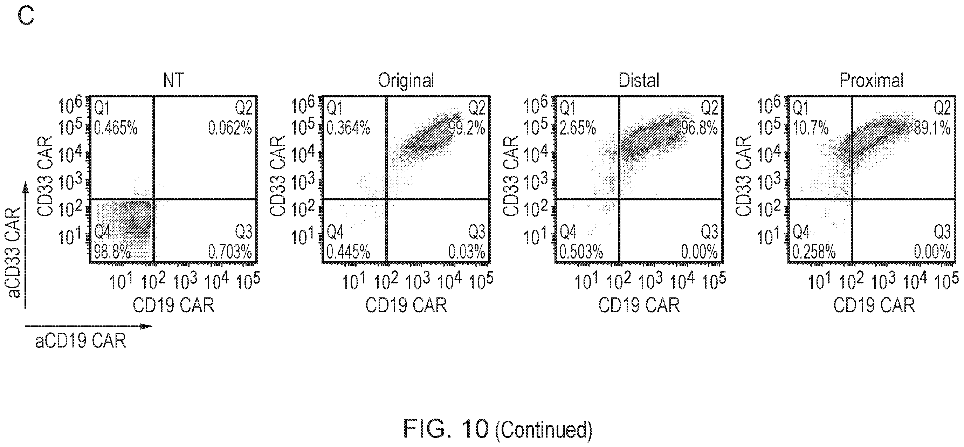

**Figure 10****:** Functionality of the TYRP1 retention signal in primary cells
   A construct was generated which co-express an anti-CD19 and an anti-CD33 CAR using a FMD-2A like peptide. Two variants of this construct were also generated: in the first variant,the di-leucine motif from TYRP1 was inserted into the anti-CD19 CAR endodomain just proximal to the TM domain; In the second variant the same TYRP1 di-lecuine motif was attached to the carboxy-terminus of the anti-CD19 CAR endodomain. PBMCs were isolated from blood and stimulated using PHA and IL-2. Two days later the cells were transduced on retronectin coated plates with retro virus containing the different CD19:CD33 CAR constructs. On day 5 the expression level of the two CARs translated by the construct was evaluated via flow cytometry using recombinant CD19-Fc and CD33-Fc fusions. A. Shows cartoon of the synthetic gene constructed to allow co-expression; B. Shows a cartoon of the subsequent pairs of proteins generated by the three construcs; C. Shows expression of the two receptors by flow-cytometry. In the original construct, both CARs are equally expressed. With incorporation of the di-leucine motif distally in the endodomain of the anti-CD19 CAR, the CD33 CAR expression remains constant but the CD19 expression drops to intermediate levels. With incorporation of the di-leucine motif proximally in the endodomain of the anti-CD19 CAR, the CD33 CAR expression remains constant, but the CD19 expression drops to low levels.

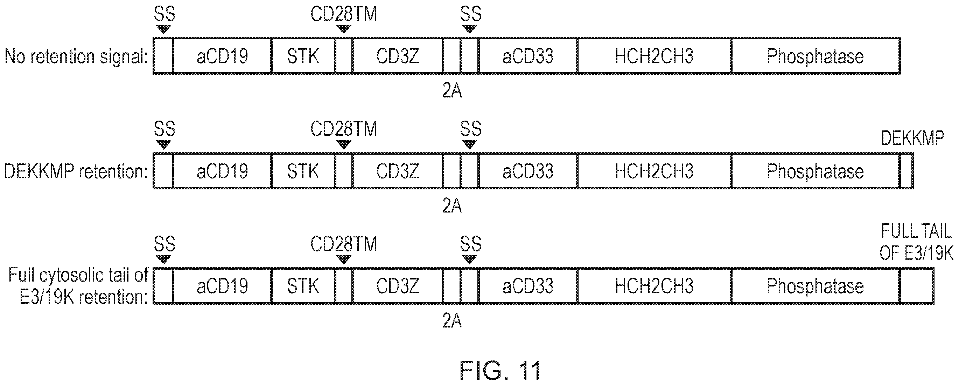

**Figure 11****:** Retention signal from cytosolic tail of E3/19K
   A construct was generated which co-expresses an anti-CD19 and an anti-CD33 CAR using a FMD-2A like peptide. Two variants of this construct were also generated: in the first variant, the last 6aa from E3/19K (DEKKMP), which were found to be critical for its Golgi/ER retention ability, were attached to the carboxy-terminus of the anti-CD33 CAR endodomain; in the second variant, the entire cytosolic tail of adenovirus E3/19K protein was attached to the carboxy-terminus of the anti-CD33 CAR endodomain
**Figure 12****:** Functionality of E3/19K retention signal
   The constructs shown in Figure 4 were transfected into 293T cells and the expression level of the two CARs translated by the construct was evaluated via flow cytometry using recombinant CD19-Fc and CD33-Fc fusions. A clear retention was observed when the full length adenovirus E3/19K protein, or the DEKKMP motif was placed on the anti-CD33 receptor. The anti-CD19 receptor expression levels were unaffected.

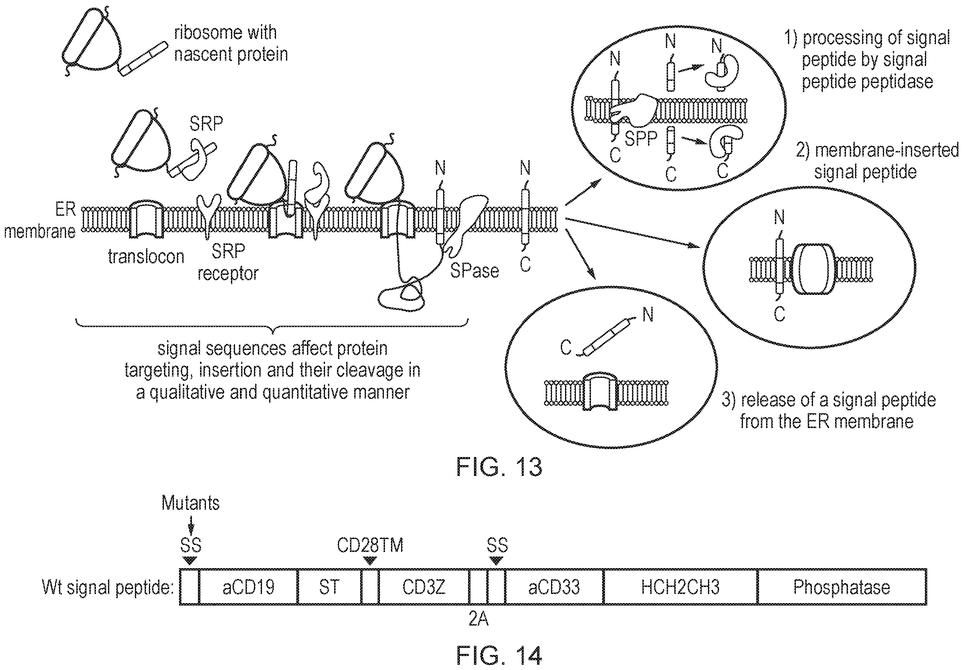

**Figure 13****:** Schematic diagram illustrating the function of signal sequences in protein targeting
**Figure 14****:** Schematic diagram of nucleic acid construct encoding two CARs

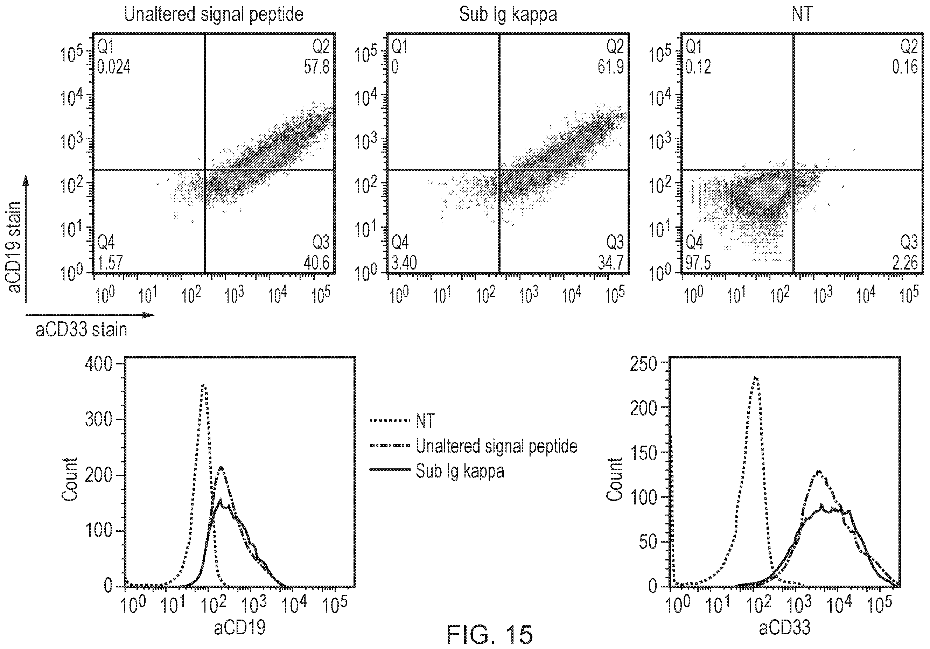

**Figure 15****:** Verifying the function of a substituted signal sequence.
   PCT/GB2014/053452 describes vector system encoding two chimeric antigen receptors (CARs), one against CD19 and one against CD33. The signal peptide used for the CARs in that study was the signal peptide from the human CD8a signal sequence. For the purposes of this study, this was substituted with the signal peptide from the murine Ig kappa chain V-III region, which has the sequence: METD**TLILWVLLLLV**PGSTG (hydrophobic residues hightlited in bold). In order to establish that the murine Ig kappa chain V-III signal sequence functioned as well as the signal sequence from human CD8a, a comparative study was performed. For both signal sequences, functional expression of the anti-CD33 CAR and the anti-CD19 CAR was observed.

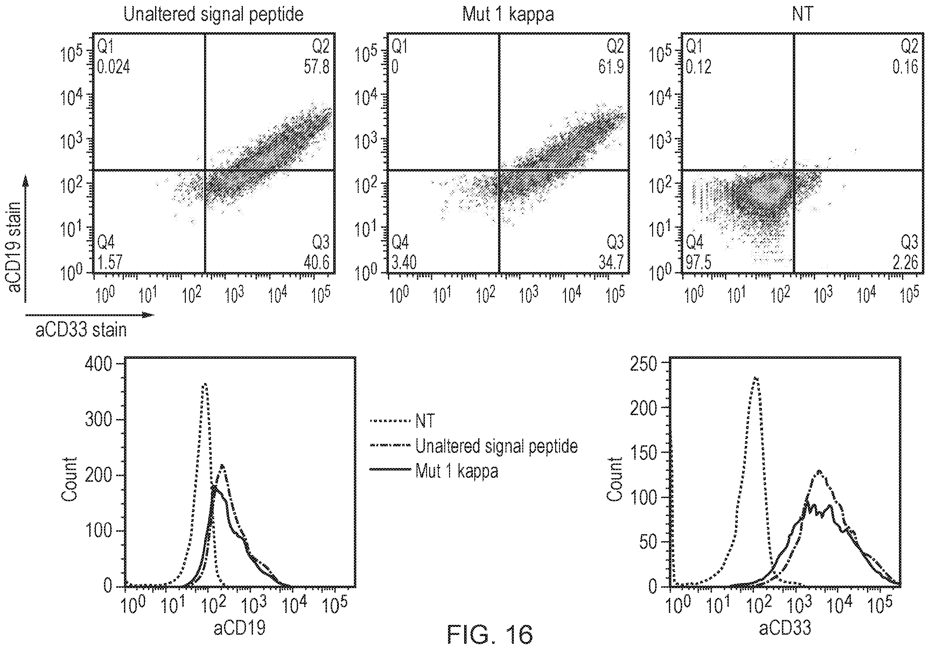

**Figure 16****:** Testing the effect of one amino acid deletion in the murine Ig kappa chain V-III. Mutant 1 kappa chain was created with the following deletion (shown in grey) in the h-region METD**TLILWVLLLLV**PGSTG and the relative expression on the anti-CD33 CAR and the anti-CD19 CAR was observed.

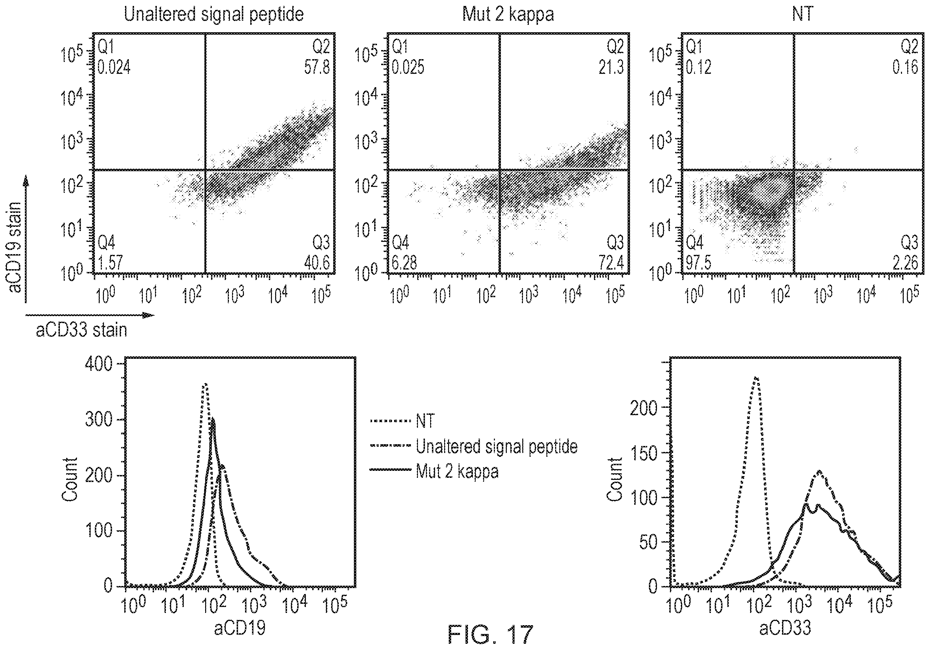

**Figure 17****:** Testing the effect of two amino acid deletions in the murine Ig kappa chain V-III. Mutant 2 kappa chain was created with the following deletions (shown in grey) in the h-region METD**TLILWVLLLLV**PGSTG and the relative expression on the anti-CD33 CAR and the anti-CD19 CAR was observed.

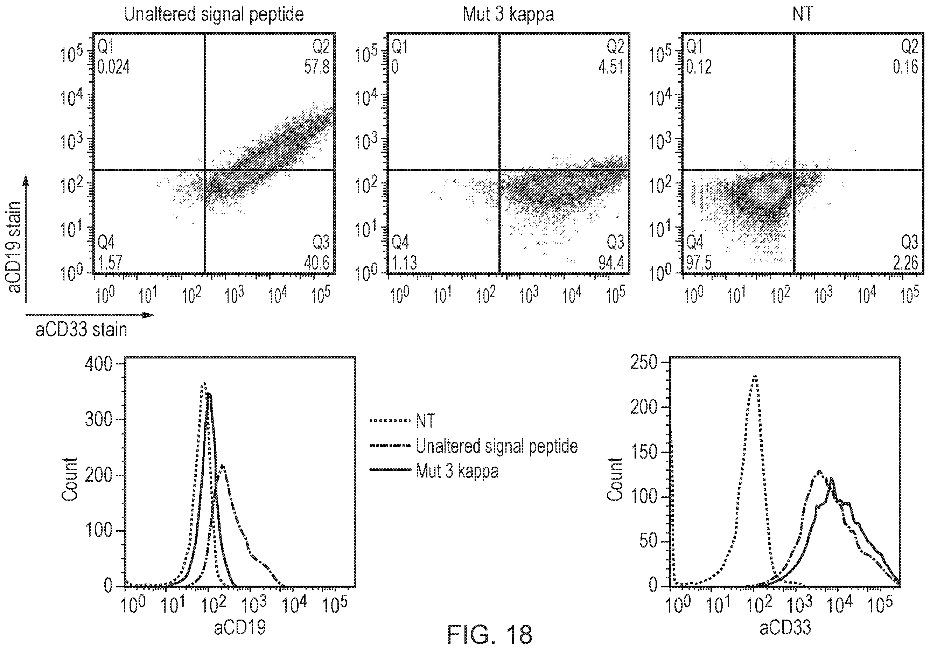

**Figure 18****:** Testing the effect of three amino acid deletions in the murine Ig kappa chain V-III. Mutant 2 kappa chain was created with the following deletions (shown in grey) in the h-region METD**TLILWVLLLLV**PGSTG and the relative expression on the anti-CD33 CAR and the anti-CD19 CAR was observed.

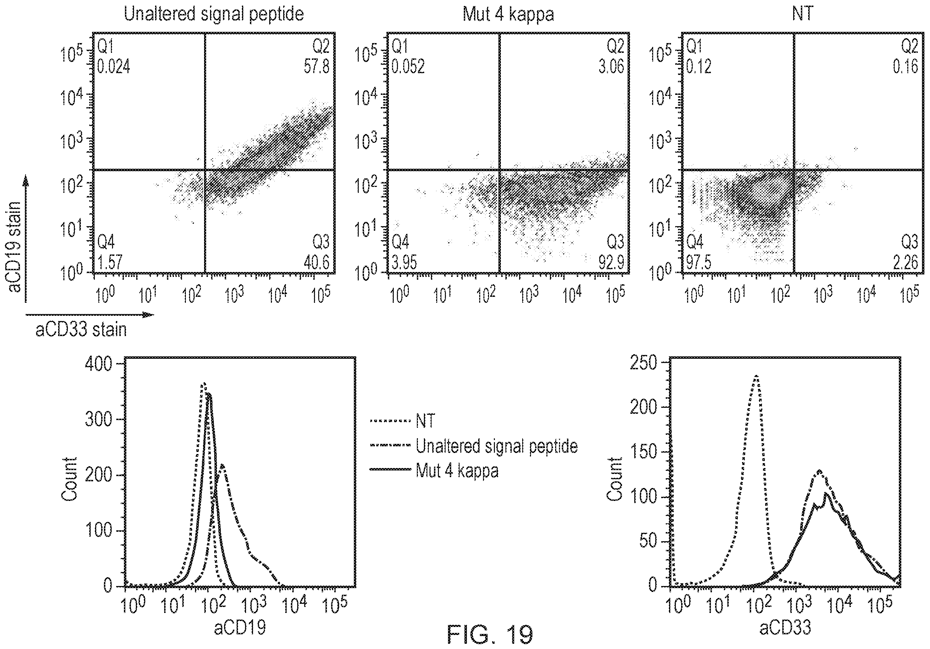

**Figure 19****:** Testing the effect of five amino acid deletions in the murine Ig kappa chain V-III. Mutant 2 kappa chain was created with the following deletions (shown in grey) in the h-region METD**T**LI**LWVLLL**LVPGSTG and the relative expression on the anti-CD33 CAR and the anti-CD19 CAR was observed

### DETAILED DESCRIPTION

### CHIMERIC ANTIGEN RECEPTORS (CARs)

The present invention provides a nucleic acid construct which expresses two or more chimeric antigen receptors (CARs).

CARs, which are shown schematically in Figure 1, are chimeric type I trans-membrane proteins which connect an extracellular antigen-recognizing domain (binder) to an intracellular signalling domain (endodomain). The binder is typically a single-chain variable fragment (scFv) derived from a monoclonal antibody (mAb), but it can be based on other formats which comprise an antibody-like antigen binding site. A spacer domain is usually necessary to isolate the binder from the membrane and to allow it a suitable orientation. A common spacer domain used is the Fc of IgG1. More compact spacers can suffice e.g. the stalk from CD8a and even just the IgG1 hinge alone, depending on the antigen. A trans-membrane domain anchors the protein in the cell membrane and connects the spacer to the endodomain.

Early CAR designs had endodomains derived from the intracellular parts of either the γ chain of the FcεR1 or CD3ζ. Consequently, these first generation receptors transmitted immunological signal 1, which was sufficient to trigger T-cell killing of cognate target cells but failed to fully activate the T-cell to proliferate and survive. To overcome this limitation, compound endodomains have been constructed: fusion of the intracellular part of a T-cell co-stimulatory molecule to that of CD3ζ results in second generation receptors which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. Some receptors have also been described which include TNF receptor family endodomains, such as the closely related OX40 and 41BB which transmit survival signals. Even more potent third generation CARs have now been described which have endodomains capable of transmitting activation, proliferation and survival signals.

CAR-encoding nucleic acids may be transferred to cells (such a T cells) using, for example, retroviral or lentiviral vectors. In this way, a large number of cancer-specific T cells can be generated for adoptive cell transfer. When the CAR binds the target-antigen, this results in the transmission of an activating signal to the T-cell it is expressed on. Thus the CAR directs the specificity and cytotoxicity of the T cell towards tumour cells expressing the targeted antigen.

The inventors have defined three distinct categories of cells which co-express a first CAR and a second CAR such that the cell can recognize a desired pattern of expression on target cells in the manner of a logic gate as detailed in the truth tables: Table 1, 2 and 3.

Both the first and second (and optionally subsequent) CARs comprise:
(i) an antigen-binding domain;
(ii) a spacer;
(iii) a transmembrane domain; and
(iii) an intracellular domain, which may comprise or associate with a T-cell signalling endodomain.

**Table 1: Truth Table for CAR OR GATE**

| *Antigen A* | *Antigen B* | *Response* |
|---|---|---|
| Absent | Absent | No activation |
| Absent | Present | Activation |
| Present | Absent | Activation |
| Present | Present | Activation |

**Table 2: Truth Table for CAR AND GATE**

| *Antigen A* | *Antigen B* | *Response* |
|---|---|---|
| Absent | Absent | No activation |
| Absent | Present | No Activation |
| Present | Absent | No Activation |
| Present | Present | Activation |

**Table 3: Truth Table for CAR AND NOT GATE**

| *Antigen A* | *Antigen B* | *Response* |
|---|---|---|
| Absent | Absent | No activation |
| Absent | Present | No Activation |
| Present | Absent | Activation |
| Present | Present | No Activation |

Using the nucleic acid construct of the present invention, the first and second CARs are produced as a polypeptide comprising both CARs, together with a cleavage site.

SEQ ID No. 4 is an example of an OR gate, which comprises two CARs. The nucleic acid construct of the invention may comprise one or other part of the following amino acid sequence, which corresponds to a single CAR. One or both CAR sequences may be modified to include one or more intracellular retention signals, and/or to alter their signal peptides, as defined below.

SEQ ID No 4 is a CAR OR gate which recognizes CD19 OR CD33

SEQ ID No. 4 breaks down as follows:
**Signal peptide derived from Human CD8a:**
   MSLPVTALLLPLALLLHAARP
**Linker:**
   SD
**Human CD8aSTK:**
   PTTTPAPRPPTPAPTIASQPLSLRPEACRPAAGGAVHTRGLDFACDI
**Human CD28TM:**
   FVWLVWGGVLACYSLLVTVAFIIFWV
**2A peptide:**
   RAEGRGSLLTCGDVEENPGP
**Signal peptide derived from mouse Ig kappa:**
   MAVPTQVLGLLLLWLTDA
**Linker:**
   DPA
**Linker:**
   KDPK
**Human CD28TM:**
   FVWLVWGGVLACYSLLVTVAFIIFWV
**Linker:**
   RS

The present invention relates to the modulation of the relative expression of the two or more CARs in an OR gate. This may be done, for example, by including an intracellular retention signal in one or other CAR.

In relation to SEQ ID NO. 4 given above, a suitable position for the intracellular retention signal may be readily determined based on the position of the retention signal, or signals of a similar type, in its native protein. The modulatory effect of the retention signal may also be fine tuned by choosing a certain position for the retention signal in the molecule.

For example, the nucleic acid construct may comprise a tyrp-1 retention protein sequence, such as:
RARRSMDEANQPLLTDQYQCYAEEYEKLQNPNQSVV

Such a sequence may be included, for example, in the CD19 CAR sequence in order for the relative expression level of CD19 CAR to be reduced with respect to CD33 CAR.

The position of the tyrp-1 retention signal in the aCD19 receptor will alter the amount of reduction: for low expression levels, the tyrp-1 retention signal may be placed between "Human CD28TM" and "Human CD3zeta intracellular domain"; for medium expression levels the tyrp-1 retention signal may be placed between "Human CD3zeta intracellular domain" and "2A peptide" in SEQ ID NO. 4.

Alternatively, the nucleic acid construct may comprise the Adenoviral E3/19K intracellular retention signal. The intracellular retention signal may comprise the E3/19K cytosolic domain KYKSRRSFIDEKKMP (SEQ ID No. 2) or a portion thereof, such as the sequence DEKKMP (SEQ ID No. 3).

The E3/19K retention signal may be positioned at the C-terminus of the CAR whose expression is to be reduced.

The nucleic acid construct of the invention may encode SEQ ID No. 4 or any of its components parts, such as the CD19 CAR or the CD33 CAR. The nucleic acid construct of the invention may encode a variant of the CAR-encoding part of the sequence shown as SEQ ID No. 4 having at least 80, 85, 90, 95, 98 or 99% sequence identity, provided that the variant sequence is a CAR having the required properties.

Methods of sequence alignment are well known in the art and are accomplished using suitable alignment programs. The % sequence identity refers to the percentage of amino acid or nucleotide residues that are identical in the two sequences when they are optimally aligned. Nucleotide and protein sequence homology or identity may be determined using standard algorithms such as a BLAST program (Basic Local Alignment Search Tool at the National Center for Biotechnology Information) using default parameters, which is publicly available at http://blast.ncbi.nlm.nih.gov. Other algorithms for determining sequence identity or homology include: LALIGN (http://www.ebi.ac.uk/Tools/psa/lalign/ and http://www.ebi.ac.uk/Tools/psa/lalign/nucleotide.html), AMAS (Analysis of Multiply Aligned Sequences, at http://www.compbio.dundee.ac.uk/Software/Amas/amas.html), FASTA (http://www.ebi.ac.uk/Tools/sss/fasta/), Clustal Omega (http://www.ebi.ac.uk/Tools/msa/clustalo/), SIM (http://web.expasy.org/sim/), and EMBOSS Needle (http://www.ebi.ac.uk/Tools/psa/emboss_needle/nucleotide.html).

In the CAR logical OR gate, the antigen binding domains of the first and second CARs bind to different antigens and both CARs comprise an activating endodomain. The spacer domains may be the same, or sufficiently different to prevent cross-pairing of the two different receptors. A cell can hence be engineered to activate upon recognition of either or both antigens. This is useful in the field of oncology as indicated by the Goldie-Coldman hypothesis: sole targeting of a single antigen may result in tumour escape by modulation of said antigen due to the high mutation rate inherent in most cancers. By simultaneously targeting two antigens, the probably of such escape is exponentially reduced.

Various tumour associated antigens are known as shown in the following Table 4. For a given disease, the first CAR and second CAR may bind to two different TAAs associated with that disease. In this way, tumour escape by modulating a single antigen is prevented, since a second antigen is also targeted. For example, when targeting a B-cell malignancy, both CD19 and CD20 can be simultaneously targeted. With a CAR logical OR gate, it is important that the two CARs do not heterodimerize.

**TABLE 4**

| **Cancer type** | **TAA** |
|---|---|
| Diffuse Large B-cell Lymphoma | CD19, CD20 |
| Breast cancer | ErbB2, MUC1 |
| AML | CD13, CD33 |
| Neuroblastoma | GD2, NCAM |
| B-CLL | CD19, CD52 |
| Colorectal cancer | Folate binding protein, CA-125 |

### DIFFERENTIAL EXPRESSION OF TARGET ANTIGENS

### CD19/CD22

The nucleic acid construct of the present invention may encode an OR gate which comprises a CAR recognising CD19 and a CAR recognising CD22. A CD19/CD22 OR gate is useful for the treatments of a number of B-cell malignancies, including B-cell acute lymphoblastic leukemia (B-ALL), B-cell lymphoma and B-cell chronic lymphocytic leukemia (B-CLL).

It has been reported in several studies that the levels of expression of CD19 and CD22 are very different in these conditions.

For example, Haso et al (2013, Blood 121:1165-1174) measured CD22 and CD19 surface expression on various B-cell precursor ALL cell lines and found that all cell lines expressed both CD22 and CD19, but CD19 had a significantly higher site density (CD22 expression ranged from 6485 to 54,878 molecules/cell versus a range of CD19 expression of 14,112 to 56,946 molecules/cell).

Similarly Du et al (2008 Cancer Res 68: 6300-6305) examined the surface expression levels of CD19 and CD22 on six B cell lymphoma lines and found that CD19 levels were 4-9 fold higher than CD22 levels on B cell lymphoma cells (CD19 levels ranged from 210,000-578,000 sites per cell, whereas CD22 levels ranged from 26,000-94,000 sites per cell).

In the OR gate of the present invention, the relative expression of the CD19 CAR and CD22 CAR may be modified in order to reflect the relative levels of CD19 and CD22 on the cancer cells. The level of expression of the CD22 CAR may be reduced by incorporation of an intracellular retention signal or by altering the signal peptide such that it has fewer hydrophobic amino acids than the signal peptide of the CD19 CAR.

### CD19/CD20

Berton et al (2014, Haematol & Oncol 7:33) investigated the mean antibody binding capacity values of CD19 and CD20 on primary B malignancies and B cell lines, as follows:
Raji, Ramos, Namalwa - human Burkitt's lymphoma cell lines
SU-DHL-6 - human diffuse histiocytic lymphoma cell line
Mec-1 - human B-CLL cell line

It was found that for the human Burkitt's lymphoma cell lines, the number of antigenic sites was 2-27 fold greater for CD20 than CD19. By contrast, the number of antigenic sites for CD19 was greater than CD20 for the human diffuse histiocytic lymphoma cell line (22-fold) and the B-CLL cell line (3-fold).

The OR gate of the present invention may comprise a CAR recognising CD19 and a CAR recognising CD20. The relative expression of the CD19 CAR and CD20 CAR may be modified in order to reflect the relative levels of CD19 and CD20 on the cancer cells. For some cancer types, the level of expression of the CD20 CAR may be reduced by incorporation of an intracellular retention signal or by altering the signal peptide such that it has fewer hydrophobic amino acids than the signal peptide of the CD19 CAR. For other cancer types, the level of expression of the CD19 CAR may be reduced by incorporation of an intracellular retention signal or by altering the signal peptide such that it has fewer hydrophobic amino acids than the signal peptide of the CD20 CAR.

### SIGNAL PEPTIDE

The polypeptides A and B (and optionally others, C, D etc) encoded by the nucleic acid construct of the invention each comprise may a signal sequence so that when the polypeptide is expressed inside a cell the nascent protein is directed to the endoplasmic reticulum (ER) (see Figure 13).

The term "signal peptide" is synonymous with "signal sequence".

A signal peptide is a short peptide, commonly 5-30 amino acids long, present at the N-terminus of the majority of newly synthesized proteins that are destined towards the secretory pathway. These proteins include those that reside either inside certain organelles (for example, the endoplasmic reticulum, golgi or endosomes), are secreted from the cell, and transmembrane proteins.

Signal peptides commonly contain a core sequence which is a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein. The free signal peptides are then digested by specific proteases.

The signal peptide is commonly positioned at the amino terminus of the molecule, although some carboxy-terminal signal peptides are known.

As mentioned above, signal sequences have a tripartite structure, consisting of a hydrophobic core region (h-region) flanked by an n- and c-region. The latter contains the signal peptidase (SPase) consensus cleavage site. Usually, signal sequences are cleaved off co-translationally, the resulting cleaved signal sequences are termed signal peptides.

In the signal peptide from the murine Ig kappa chain V-III region, which has the sequence: METD**TLILWVLLLLV**PGSTG: the n-region has the sequence METD; the h-region (shown in bold) has the sequence TLILWVLLLLV; and the c-region has the sequence PGSTG.

In the nucleic acid construct of the present invention, the signal peptides of the two CARs may differ in the number of hydrophobic amino acids, to modulate the relative levels of expression of the CARs at the cell surface.

In the nucleic acid construct of the present invention the signal sequence of the two (or more) polypeptides therefore may differ in their h-regions. One polypeptide (which has higher relative expression) may have a greater number of hydrophobic amino acids in the h-region that the other polypeptide (which has lower relative expression). The signal peptide of the polypeptide with lower relative expression may comprise one or more amino acid mutations, such as substitutions or deletions, of hydrophobic amino acids in the h-region than the signal peptide of the polypeptide with lower relative expression.

The first signal peptide and the second signal peptide may have substantially the same n- and c- regions, but differ in the h-region as explained above. "Substantially the same" indicates that the n- and c- regions may be identical between the first and second signal peptide or may differ by one, two or three amino acids in the n- or c-chain, without affecting the function of the signal peptide.

The hydrophobic amino acids in the core may, for example be: Alanine (A); Valine (V); Isoleucine (I); Leucine (L); Methionine (M); Phenylalanine (P); Tyrosine (Y); or Tryptophan (W).

The hydrophobic acids mutated in order to alter signal peptide efficiency may be any from the above list, in particular: Valine (V); Isoleucine (I); Leucine (L); and Tryptophan (W).

Of the residues in the h-region, one signal peptide (for example, the altered signal peptide) may comprise at least 10%, 20%, 30%, 40% or 50% fewer hydrophobic amino acids than the other signal peptide (for example, the unaltered signal peptide).

Where the h-region comprises 5-15 amino acids, one signal peptide may comprise 1, 2, 3, 4 or 5 more hydrophobic amino acids than the other signal peptide.

The altered signal peptide may comprise 1, 2, 3, 4 or 5 amino acid deletions or substitutions of hydrophobic amino acids. Hydrophobic amino acids may be replaced with non-hydrophobic amino acids, such as hydrophilic or neutral amino acids.

Signal sequences can be detected or predicted using software techniques (see for example, http://www.predisi.de/).

A very large number of signal sequences are known, and are available in databases. For example, http://www.sianalpeptide.de lists 2109 confirmed mammalian signal peptides in its database.

Table 5 provides a list of signal sequences purely for illustrative purposes. The hydrophobic core is highlighted in bold. This includes examples of amino acids which may be substituted or removed for the purposes of the present invention.

**Table 5**

| **Accession Number** | **Entry Name** | **Protein Name** | **Length** | **Signal Sequence (hydrophobic core)** |
|---|---|---|---|---|
| P01730 | CD4_HUMAN | T-cell surface glycoprotein CD4 | 25 | MNRGVPFRH**LLLVLQLALL**PAATQG |
| P08575 | CD45_HUMAN | Leukocyte common antigen | 23 | MYLWLK**LLAFGFAFL**DTE**VFV**TG |
| P01732 | CD8A_HUMAN | T-cell surface glycoprotein CD8 alpha chain | 21 | MAL**PVTALLLPLALLL**HAARP |
| P10966 | CD8B_HUMAN | T-cell surface glycoprotein CD8 beta chain | 21 | MRPRLW**LLLAAQLTVL**HGNSV |
| P06729 | CD2_HUMAN | T-cell surface antigen CD2 | 24 | MSFPCKF**VASFLLIF**N**V**SSKGAVS |
| P06127 | CD5_HUMAN | T-cell surface glycoprotein CD5 | 24 | MPMGSLQP**LATLYLLGMLVASCL**G |
| P09564 | CD7_HUMAN | T-cell antigen CD7 | 25 | MAGPPR**LLLLPLLLALA**RGLPGALA |
| P17643 | TYRP1_HUMAN | 5,6-dihydroxyindole-2-carboxylic acid oxidase | 24 | MSAPK**LLSLGOFFPLLLF**QQARA |
| P00709 | LALBA_HUMAN | Alpha-lactalbumin | 19 | MR**FFVPLFLVGILF**PAILA |
| P16278 | BGAL_HUMAN | Beta-galactosidase | 23 | MPG**FLVRILPLLLVLLLL**GPTRG |
| P31358 | CD52_HUMAN | CAMPATH-1 antigen | 24 | MKR**FLFLLLTISLLVM**VQIQTGLS |
| Q6YHK3 | CD109_tnjMAN | CD109 antigen | 21 | MQGFP**LLTAAHLLCVCTAAL**A |
| P01024 | CO3_HUMAN | Complement C3 | 22 | MGPTSGPS**LLLLLLT**HL**PLAL**G |
| P10144 | GRAB_HUMAN | Granzyme B | 18 | MQ**PILLLLAFLLL**PRADA |
| P04434 | KV310_HUMAN | Ig kappa chain V-III region VH | 20 | MEAPAQLLF**LLLL**WLPDTTR |
| P06312 | KV401_HUMAN | Ig kappa chain V-IV region | 20 | MVLQTQ**VFISLLL**WISGAYG |
| P06319 | LV605_HUMAN | Ig lambda chain V-VI region EB4 | 19 | MAWAP**LLLTLLA**HCIDCWA |
| P31785 | IL2RG_HUMAN | Cytokine receptor common gamma chain | 22 | MLKPSLPFTS**LLFLQLPLLGV**G |
| Q8N4F0 | BPIL1_HUMAN | Bactericidal/permeability-increasing protein-like 1 | 20 | MAWASR**LGLLLALLLP**WGA |
| P55899 | FCGRN_HUMAN | IgG receptor FcRn large subunit p51 | 23 | MGVPRPQPW**ALGLLLFLL**PGSLG |

The mutated signal peptide comprises one or more mutation(s) such that it has fewer hydrophobic amino acids than the wild-type signal peptide from which it is derived. The term "wild type" means the sequence of the signal peptide which occurs in the natural protein from which it is derived. For example, the signal peptide described in the examples is the signal peptide from the murine Ig kappa chain V-III region, which has the wild-type sequence: METDTLILWVLLLLVPGSTG.

The term "wild-type" also includes signal peptides derived from a naturally occurring protein which comprise one or more amino acid mutations in the n- or c- region. For example it is common to modify a natural signal peptide with a conserved amino acid substitution on the N-terminus to introduce a restriction site. Such modified signal peptide sequences (which do not comprise any mutations in the h-region) are considered "wild-type" for the purposes of the present invention.

The present invention also relates to synthetic signal peptide sequences, which cannot be defined with reference to a wild-type sequence. In this embodiment, the signal peptide of the one polypeptide comprises fewer hydrophobic amino acids than the signal sequence of the other polypeptide. The two signal sequences may be derived from the same synthetic signal peptide sequence, but differ in the number of hydrophobic amino acids in the core region.

### ANTIGEN BINDING DOMAIN

The antigen binding domain is the portion of the CAR which recognizes antigen. Numerous antigen-binding domains are known in the art, including those based on the antigen binding site of an antibody, antibody mimetics, and T-cell receptors. For example, the antigen-binding domain may comprise: a single-chain variable fragment (scFv) derived from a monoclonal antibody; a natural ligand of the target antigen; a peptide with sufficient affinity for the target; a single domain antibody; an artificial single binder such as a Darpin (designed ankyrin repeat protein); or a single-chain derived from a T-cell receptor.

The antigen binding domain may comprise a domain which is not based on the antigen binding site of an antibody. For example the antigen binding domain may comprise a domain based on a protein/peptide which is a soluble ligand for a tumour cell surface receptor (e.g. a soluble peptide such as a cytokine or a chemokine); or an extracellular domain of a membrane anchored ligand or a receptor for which the binding pair counterpart is expressed on the tumour cell.

The antigen binding domain may be based on a natural ligand of the antigen. For example, the antigen binding domain may comprise APRIL, the natural ligand of BCMA.

The antigen binding domain may comprise an affinity peptide from a combinatorial library or a *de novo* designed affinity protein/peptide.

### SPACER DOMAIN

CARs may comprise a spacer sequence to connect the antigen-binding domain with the transmembrane domain and spatially separate the antigen-binding domain from the endodomain. A flexible spacer allows the antigen-binding domain to orient in different directions to facilitate binding.

The first and second CARs encoded by the nucleic acid construct of the invention may comprise different spacer molecules. For example, the spacer sequence may, for example, comprise an IgG1 Fc region, an IgG1 hinge or a human CD8 stalk or the mouse CD8 stalk. The spacer may alternatively comprise an alternative linker sequence which has similar length and/or domain spacing properties as an IgG1 Fc region, an IgG1 hinge or a CD8 stalk. A human IgG1 spacer may be altered to remove Fc binding motifs.

Examples of amino acid sequences for these spacers are given below:
SEQ ID No. 6 (human CD8 stalk):
   TTTPAPRPPTPAPTIASQPLSLRPEACRPAAGGAVHTRGLDFACDI
SEQ ID No. 7 (human IgG1 hinge):
   AEPKSPDKTHTCPPCPKDPK

Since CARs are typically homodimers (see Figure 1a), cross-pairing may result in a heterodimeric chimeric antigen receptor. This is undesirable for various reasons, for example: (1) the epitope may not be at the same "level" on the target cell so that a cross-paired CAR may only be able to bind to one antigen; (2) the VH and VL from the two different scFv could swap over and either fail to recognize target or worse recognize an unexpected and unpredicted antigen. For the "OR" gate, the spacer of the first CAR may be sufficiently different from the spacer of the second CAR in order to avoid cross-pairing. The amino acid sequence of the first spacer may share less that 50%, 40%, 30% or 20% identity at the amino acid level with the second spacer.

All the spacer domains mentioned above form homodimers. However the mechanism is not limited to using homodimeric receptors and should work with monomeric receptors as long as the spacer is sufficiently rigid. An example of such a spacer is CD2 or truncated CD22.

### TRANSMEMBRANE DOMAIN

The transmembrane domain is the sequence of the CAR that spans the membrane.

A transmembrane domain may be any protein structure which is thermodynamically stable in a membrane. This is typically an alpha helix comprising of several hydrophobic residues. The transmembrane domain of any transmembrane protein can be used to supply the transmembrane portion of the invention. The presence and span of a transmembrane domain of a protein can be determined by those skilled in the art using the TMHMM algorithm (http://www.cbs.dtu.dk/services/TMHMM-2.0/). Further, given that the transmembrane domain of a protein is a relatively simple structure, i.e a polypeptide sequence predicted to form a hydrophobic alpha helix of sufficient length to span the membrane, an artificially designed TM domain may also be used (US 7052906 B1 describes synthetic transmembrane components).

The transmembrane domain may be derived from CD28, which gives good receptor stability.

### ACTIVATING ENDODOMAIN

The endodomain is the signal-transmission portion of a CAR. It may be part of the CAR, or as part of a separate molecule which associates with the CAR (antigen binding and transmembrane domains) intracellular to perform its signal transmission function. After antigen recognition, receptors cluster, native CD45 and CD148 are excluded from the synapse and a signal is transmitted to the cell. The most commonly used endodomain component is that of CD3-zeta which contains 3 ITAMs. This transmits an activation signal to the T cell after antigen is bound. CD3-zeta may not provide a fully competent activation signal and additional co-stimulatory signalling may be needed. For example, chimeric CD28 and OX40 can be used with CD3-Zeta to transmit a proliferative / survival signal, or all three can be used together.

In the "OR gate" of the present invention, the cell comprises two CARs, each which comprises or associates with an activating endodomain. The activating endodomain is capable of transmitting both immunological signal 1 and immunological signal 2. An endodomain is not considered "activating" if it just comprises CD3ζ, which is sufficient to trigger T-cell killing of cognate target cells does not fully activate the T-cell to proliferate and survive. An "activating endodomain" is a compound endodomain: in which the intracellular part of a T-cell co-stimulatory molecule is fused to that of CD3 ζ resulting in a "second generation" receptor which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. In addition to a co-stimulatory domain, the activating endodomain may also include TNF receptor family endodomains, such as the closely related OX40 and 41BB which transmit survival signals.

An "activating" endodomain may therefore not comprise the CD3-Zeta endodomain or the CD28 endodomain alone. An activating endodomain may, for example, comprise the CD3-Zeta endodomain with that of either CD28 or OX40 or the CD28 endodomain and OX40 and CD3-Zeta endodomain.

Each of the CARs in the OR gate is independently capable of activating the T cell. The T cell is thus activated by the presence of either antigen alone. The two CARs are not "complementary" in the sense that activation of both CARs is necessary to provide activation and co-stimulatory signals.

An endodomain which contains an ITAM motif can act as an activation endodomain in this invention. Several proteins are known to contain endodomains with one or more ITAM motifs. Examples of such proteins include the CD3 epsilon chain, the CD3 gamma chain and the CD3 delta chain to name a few. The ITAM motif can be easily recognized as a tyrosine separated from a leucine or isoleucine by any two other amino acids, giving the signature YxxL/I. Typically, but not always, two of these motifs are separated by between 6 and 8 amino acids in the tail of the molecule (YxxL/Ix(6-8)YxxL/I). Hence, one skilled in the art can readily find existing proteins which contain one or more ITAM to transmit an activation signal. Further, given the motif is simple and a complex secondary structure is not required, one skilled in the art can design polypeptides containing artificial ITAMs to transmit an activation signal (see WO 2000063372, which relates to synthetic signalling molecules).

The transmembrane and intracellular T-cell signalling domain (endodomain) of a CAR with an activating endodomain may comprise the sequence shown as SEQ ID No. 10, 11 or 12 or a variant thereof having at least 80% sequence identity.

A variant sequence may have at least 80%, 85%, 90%, 95%, 98% or 99% sequence identity to 10, 11 or 12, provided that the sequence provides an effective trans-membrane domain and an effective intracellular T cell signaling domain.

### NUCLEIC ACID CONSTRUCT

The present invention provides a nucleic acid construct comprising the following structure:

A-X-B

in which
A and B are nucleic acid sequences encoding a first chimeric antigen receptor (CAR) and a second CAR; and
X is a nucleic acid sequence which encodes a cleavage site,
wherein
the first CAR and second CAR recognise different antigens;
the first CAR and second CAR each comprise an activating endodomain; and the first and/or second CAR comprises an intracellular retention signal selected from the following group: an endocytosis signal; a Golgi retention signal; a *trans-*Golgi network (TGN) recycling signal; an endoplasmic reticulum (ER) retention signal; and a lysosomal sorting signal.

The present invention also provides nucleic acid constructs which comprise A-X-B but also express one or more further proteins of interest (POls). For example, the present invention provides a nucleic acid construct comprising the following structure:

A-X-B-Y-C

in which
A, B and C are nucleic acid sequences encoding a first, second and third polypeptides of interest (POls); and
X and Y are nucleic acid sequences which may be the same or different, each of which encodes a cleavage site,
wherein at least two of the POls are chimeric antigen receptors (CARs) which comprise an intracellular retention signal.

### NUCLEIC ACID SEQUENCES ENCODING CARS

The nucleic acid construct of the present invention comprises two or more nucleic acid sequences encoding CARs.

The nucleic acid sequence may comprise SEQ ID No. 13, or a variant thereof, with the addition of one or more sequences which encode intracellular retention sequence(s)

The nucleic acid sequence may encode the same amino acid sequence as that encoded by SEQ ID No. 13, but may have a different nucleic acid sequence, due to the degeneracy of the genetic code. The nucleic acid sequence may have at least 80, 85, 90, 95, 98 or 99% identity to the sequence shown as SEQ ID No. 13, provided that it encodes a first CAR and a second CAR.

### CLEAVAGE SITE

The present nucleic acid construct comprises a sequence encoding a cleavage site positioned between nucleic acid sequences which encode first and second CARs, such that the first and second CARs can be expressed as separate entities.

The cleavage site may be any sequence which enables the polypeptide comprising the first and second CARs to become separated.

The term "cleavage" is used herein for convenience, but the cleavage site may cause the first and second CARs to separate into individual entities by a mechanism other than classical cleavage. For example, for the Foot-and-Mouth disease virus (FMDV) 2A self-cleaving peptide (see below), various models have been proposed for to account for the "cleavage" activity: proteolysis by a host-cell proteinase, autoproteolysis or a translational effect (Donnelly et al (2001) J. Gen. Virol. 82:1027-1041). The exact mechanism of such "cleavage" is not important for the purposes of the present invention, as long as the cleavage site, when positioned between nucleic acid sequences which encode first and second CARs, causes the first and second CARs to be expressed as separate entities.

The cleavage site may be a furin cleavage site.

Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-beta-secretase, membrane type-1 matrix metalloproteinase, beta subunit of pro-nerve growth factor and von Willebrand factor. Furin cleaves proteins just downstream of a basic amino acid target sequence (canonically, Arg-X-(Arg/Lys)-Arg') and is enriched in the Golgi apparatus.

The cleavage site may be a Tobacco Etch Virus (TEV) cleavage site.

TEV protease is a highly sequence-specific cysteine protease which is chymotrypsin-like proteases. It is very specific for its target cleavage site and is therefore frequently used for the controlled cleavage of fusion proteins both *in vitro* and *in vivo.* The consensus TEV cleavage site is ENLYFQ\S (where '\' denotes the cleaved peptide bond). Mammalian cells, such as human cells, do not express TEV protease. Thus in embodiments in which the present nucleic acid construct comprises a TEV cleavage site and is expressed in a mammalian cell - exogenous TEV protease must also expressed in the mammalian cell.

The cleavage site may encode a self-cleaving peptide.

A 'self-cleaving peptide' refers to a peptide which functions such that when the polypeptide comprising the first and second CARs and the self-cleaving peptide is produced, it is immediately "cleaved" or separated into distinct and discrete first and second CARs without the need for any external cleavage activity.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus.

The C-terminal 19 amino acids of the longer cardiovirus protein, together with the N-terminal proline of 2B mediate "cleavage" with an efficiency approximately equal to the apthovirus FMDV 2a sequence. Cardioviruses include encephalomyocarditis virus (EMCV) and Theiler's murine encephalitis virus (TMEV).

Mutational analysis of EMCV and FMDV 2A has revealed that the motif DxExNPGP is intimately involved in "cleavage" activity (Donelly et al (2001) as above).

The cleavage site of the present invention may comprise the amino acid sequence: Dx₁Ex₂NPGP, where x₁ and x₂ are any amino acid. X₁ may be selected from the following group: I, V, M and S. X₂ may be selected from the following group: T, M, S, L, E, Q and F.

For example, the cleavage site may comprise one of the amino acid sequences shown in Table 6.

**Table 6**

| Motif | Present in: |
|---|---|
| DIETNPGP (SEQ ID No. 14) | Picornaviruses EMCB, EMCD, EMCPV21 |
| DVETNPGP (SEQ ID No. 15) | Picornaviruses MENGO and TMEBEAN; Insect virus DCV, ABPV |
| DVEMNPGP (SEQ ID No. 16) | Picornaviruses TMEGD7 and TMEBEAN |
| DVESNPGP (SEQ ID No. 17) | Picornaviruses FMDA10, FMDA12, FMDC1, FMD01K, FMDSAT3, FMDVSAT2, ERAV; Insect virus CrPV |
| DMESNPGP (SEQ ID No. 18) | Picornavirus FMDV01G |
| DVELNPGP (SEQ ID No. 19) | Picornavirus ERBV; Porcine rotavirus |
| DVEENPGP (SEQ ID No. 20) | Picornavirus PTV-1; Insect virus TaV; Trypanosoma TSR1 |
| DIELNPGP (SEQ ID No. 21) | Bovine Rotavirus, human rotavirus |
| DIEQNPGP (SEQ ID No. 22) | Trypanosoma AP endonuclease |
| DSEFNPGP (SEQ ID No. 23) | Bacterial sequence *T. maritima* |

The cleavage site, based on a 2A sequence may be, for example 15-22 amino acids in length. The sequence may comprise the C-terminus of a 2A protein, followed by a proline residue (which corresponds to the N-terminal proline of 2B).

Mutational studies have also shown that, in addition to the naturally occurring 2A sequences, some variants are also active. The cleavage site may correspond to a variant sequence from a naturally occurring 2A polypeptide, have one, two or three amino acid substitutions, which retains the capacity to induce the "cleavage" of a polyprotein sequence into two or more separate proteins.

The cleavage sequence may be selected from the following which have all been shown to be active to a certain extent (Donnelly *et al* (2001) as above):
LLNFDLLKLAGDVESNPGP (SEQ ID No. 24)
LLNFDLLKLAGDVQSNPGP (SEQ ID No. 25)
LLNFDLLKLAGDVEINPGP (SEQ ID No. 26)
LLNFDLLKLAGDVEFNPGP (SEQ ID No. 27)
LLNFDLLKLAGDVESHPGP (SEQ ID No. 28)
LLNFDLLKLAGDVESEPGP (SEQ ID No. 29)
LLNFDLLKLAGDVESQPGP (SEQ ID No. 30)
LLNFDLLKLAGDVESNPGG (SEQ ID No. 31)

Based on the sequence of the DxExNPGP "a motif, "2A-like" sequences have been found in picornaviruses other than aptho- or cardioviruses, 'picornavirus-like' insect viruses, type C rotaviruses and repeated sequences within *Trypanosoma spp* and a bacterial sequence (Donnelly et al (2001) as above). The cleavage site may comprise one of these 2A-like sequences, such as:
YHADYYKQRLIHDVEMNPGP (SEQ ID No. 32)
HYAGYFADLLIHDIETNPGP (SEQ ID No. 33)
QCTNYALLKLAGDVESNPGP (SEQ ID No. 34)
ATNFSLLKQAGDVEENPGP (SEQ ID No. 35)
AARQMLLLLSGDVETNPGP (SEQ ID No. 36)
RAEGRGSLLTCGDVEENPGP (SEQ ID No. 37)
TRAEIEDELIRAGIESNPGP (SEQ ID No. 38)
TRAEIEDELIRADIESNPGP (SEQ ID No. 39)
AKFQIDKILISGDVELNPGP (SEQ ID No. 40)
SSIIRTKMLVSGDVEENPGP (SEQ ID No. 41)
CDAQRQKLLLSGDIEQNPGP (SEQ ID No. 42)
YPIDFGGFLVKADSEFNPGP (SEQ ID No. 43)

The cleavage site may comprise the 2A-like sequence shown as SEQ ID No. 37 (RAEGRGSLLTCGDVEENPGP).

It has been shown that including an N-terminal "extension" of between 5 and 39 amino acids can increase activity (Donnelly et al (2001) as above). In particular, the cleavage sequence may comprise one of the following sequences or a variant thereof having, for example, up to 5 amino acid changes which retains cleavage site activity:
VTELLYRMKRAETYCPRPLAIHPTEARHKQKIVAPVKQTLNFDLLKLAGDVESNPGP (SEQ ID No. 44)
LLAIHPTEARHKQKIVAPVKQTLNFDLLKLAGDVESNPGP (SEQ ID No. 45)
EARHKQKIVAPVKQTLNFDLLKLAGDVESNPGP (SEQ ID No. 46)
APVKQTLNFDLLKLAGDVESNPGP (SEQ ID No. 47)

### INTRACELLULAR RETENTION SIGNAL

The nucleic acid construct of the present invention may comprise a sequence which encodes a CAR comprising an intracellular retention signal.

Protein targeting or protein sorting is the biological mechanism by which proteins are transported to the appropriate destinations in the cell or outside of it. Proteins can be targeted to the inner space of an organelle, different intracellular membranes, plasma membrane, or to exterior of the cell via secretion. This delivery process is carried out based on sequence information contain in the protein itself.

Proteins synthesised in the rough endoplasmic reticulum (ER) of eukaryotic cells use the exocytic pathway for transport to their final destinations. Proteins lacking special sorting signals are vectorially transported from the ER via the Golgi and the trans-Golgi network (TGN) to the plasma membrane. Other proteins have targeting signals for incorporation into specific organelles of the exocytic pathway, such as endosomes and lysosomes.

Lysosomes are acidic organelles in which endogenous and internalised macromolecules are degraded by luminal hydolases. Endogenous macromolecules reach the lysosome by being sorted in the TGN from which they are transported to endosomes and then lysosomes.

The targeting signals used by a cell to sort proteins to the correct intracellular location may be exploited by the present invention. The signals may be broadly classed into the following **types:**
i) endocytosis signals
ii) Golgi retention signals
iii) TGN recycling signals
iv) ER retention signals
v) lysosomal sorting signals

'Intracellular retention signal' refers to an amino acid sequence which directs the protein in which it is encompassed to a cellular compartment other than the cell surface membrane or to the exterior of the cell.

The intracellular retention signal causes a reduction in the amount of the CAR expressed on the surface of a cell compared to an equivalent, control CAR or other transmembrane protein which does not comprise an intracellular retention signal.

In other words, the proportion of translated CAR comprising an intracellular retention signal which is expressed on at the cell surface is less than the proportion of an equivalent amount of an equivalent, translated control CAR or other transmembrane protein which does not comprise an intracellular retention signal.

For example, the amount of the CAR comprising an intracellular retention signal which is expressed on the surface of a cell may be less than 75%, less than 50%, less than 25% or less than 10% of the amount of an equivalent control CAR or other transmembrane protein which does not comprise an intracellular retention signal.

Constructs which express a polyprotein that is subsequently cleaved by a protease are generally limited by the fact the expression of the peptides from the polyprotein is limited to a 1:1 ratio. However, in the present invention, the inclusion of an intracellular retention signal in the CAR means that its expression on the cell surface can be modulated (e.g. reduced compared to an equivalent control CAR or other transmembrane protein which does not comprise an intracellular retention signal). As such the ratio of the CAR which comprises the intracellular retention signal expressed on the cell surface compared to the expression of the second CAR expressed in the polyprotein may be, for example about 1:1.5, of from 1:1.5-1:2, 1:2-1:3, 1:3-1:4, 1:4-1:5, or more than 1:5.

The amount of a CAR expressed on the surface of a cell may be determined using methods which are known in the art, for example flow cytometry or fluorescence microscopy.

The intracellular retention signal may direct the CAR away from the secretory pathway during translocation from the ER.

The intracellular retention signal may direct the CAR to an intracellular compartment or complex. The intracellular retention signal may direct the CAR to a membrane-bound intracellular compartment.

For example, the intracellular retention signal may direct the CAR to a lysosomal, endosomal or Golgi compartment (trans-Golgi Network, 'TGN').

Within a normal cell, proteins arising from biogenesis or the endocytic pathway are sorted into the appropriate intracellular compartment following a sequential set of sorting decisions. At the plasma membrane, proteins can either remain at the cell surface or be internalised into endosomes. At the TGN, the choice is between going to the plasma membrane or being diverted to endosomes. In endosomes, proteins can either recycle to the plasma membrane or go to lysosomes. These decisions are governed by sorting signals on the proteins themselves.

Lysosomes are cellular organelles that contain acid hydrolase enzymes that break down waste materials and cellular debris. The membrane around a lysosome allows the digestive enzymes to work at the pH they require. Lysosomes fuse with autophagic vacuoles (phagosomes) and dispense their enzymes into the autophagic vacuoles, digesting their contents.

An endosome is a membrane-bounded compartment inside eukaryotic cells. It is a compartment of the endocytic membrane transport pathway from the plasma membrane to the lysosome and provides an environment for material to be sorted before it reaches the degradative lysosome. Endosomes may be classified as early endosomes, late endosomes, or recycling endosomes depending on the time it takes for endocytosed material to reach them. The intracellular retention signal used in the present invention may direct the protein to a late endosomal compartment.

The Golgi apparatus is part of the cellular endomembrane system, the Golgi apparatus packages proteins inside the cell before they are sent to their destination; it is particularly important in the processing of proteins for secretion.

There is a considerable body of knowledge which has arisen from studies investigating the sorting signals present in known proteins, and the effect of altering their sequence and/or position within the molecule (Bonifacino and Traub (2003) Ann. Rev. Biochem. 72:395-447; Braulke and Bonifacino (2009) Biochimica and Biophysica Acta 1793:605-614; Griffith (2001) Current Biology 11:R226-R228; Mellman and Nelson (2008) Nat Rev Mol Cell Biol. 9:833-845; Dell'Angelica and Payne (2001) Cell 106:395-398; Schafer et al (1995) EMBO J. 14:2424-2435; Trejo (2005) Mol. Pharmacol. 67:1388-1390). Numerous studies have shown that it is possible to insert one or more sorting signals into a protein of interest in order to alter the intracellular location of a protein of interest (Pelham (2000) Meth. Enzymol. 327:279-283).

It is therefore perfectly possible to select a sorting signal having a desired localisation property and include it within a protein of interest in order to direct the intracellular location of that protein. In connection with the present application, it is therefore possible to select a sorting signal having the desired amount of reduction of expression at the plasma membrane.

The optimal position of the sorting signal in the nascent protein of interest may depend on the type of transmembrane protein (i.e. types I-IV) and whether the C-terminus is on the luminal or the cytoplasmic side of the membrane (Goder and Spiess (2001) FEBS Lett 504:87-93). This may readily be determined by considering the position of the sorting signal in its natural protein.

Examples of endocytosis signals include those from the transferrin receptor and the asialoglycoprotein receptor.

Examples of signals which cause TGN-endosome recycling include those form proteins such as the Cl- and CD-MPRs, sortilin, the LDL-receptor related proteins LRP3 and LRP10 and β-secretase, GGA1-3, LIMP-II, NCP1, mucolipn-1, sialin, GLUT8 and invariant chain.

Examples of TGN retention signals include those from the following proteins which are localized to the TGN: the prohormone processing enzymes furin, PC7, CPD and PAM; the glycoprotein E of herpes virus 3 and TGN38.

Examples of ER retention signals include C-terminal signals such as KDEL, KKXX or KXKXX and the RXR(R) motif of potassium channels. Known ER proteins include the adenovirus E19 protein and ERGIC53.

Examples of lysosomal sorting signals include those found in lysosomal membrane proteins, such as LAMP-1 and LAMP-2, CD63, CD68, endolyn, DC-LAMP, cystinosin, sugar phosphate exchanger 2 and acid phosphatase.

The intracellular retention signal may be from the adenovirus E19 protein. The intracellular setention signal may be from the protein E3/19K, which is also known as E3gp 19 kDa; E19 or GP19K. The intracellular retention signal may comprise the full cytosolic tail of E3/19K, which is shown as SEQ ID No. 2; or the last 6 amino acids of this tail, which is shown as SEQ ID No. 3. The present inventors have shown that the last 6 amino acids are particularly important for retention (Example 3 and Figure 5)
SEQ ID No. 2: KYKSRRSFIDEKKMP
SEQ ID No. 3: DEKKMP

### TUNABILITY

The relative expression of one or both CARs may be fine tuned by various methods, such as
a) altering the position of the intracellular retention signal in the protein molecule; and/or
b) selecting a particular intracellular retention signal.

Option a) is discussed in more detail below.

With regard to option b), a range of intracellular retention signals is available from the large number of naturally occurring proteins which are sorted to distinct cellular locations inside eukaryotic cells. It is also possible to use "synthetic" intracellular retention signals which comprise one or more of the motifs found in naturally occurring proteins (see next section) and have a similar sorting signal function.

A cascade of signal strength is available, depending on the intracellular location to which the sorting signal sends the relevant protein. Broadly speaking, the more "intracellular" the location directed by the sorting signal, the "stronger" the signal is in terms of lowering the relative expression of the protein.

When a sorting signal directs a protein to the lysosomal compartment, the protein is internalised and degraded by the cell, resulting in relatively little escape to the cell surface. The protein is degraded and lost from the system once it enters the lysosome. Therefore lysosomal sorting signals, such as LAMP1, are the "strongest" in terms of reducing relative expression at the cell surface.

When a sorting signal directs a protein to be retained in the ER, only a very small proportion of the protein gets to the cell surface. Hence ER retention or recycling signals, such as ERGIC-53 and KKFF signal are the next most strong, in terms of reducing relative expression at the cell surface.

When a sorting signal directs a protein to the endosomal, Golgi or TGN compartments, then the protein is likely to recycle to some extent between the TGN, the endosomal compartment, and the plasma membrane. These signals provide a more limited level of reduction of expression as a significant proportion of the protein will still reach the plasma membrane.

In general the reduction in expression seen with known sorting signals can be summarised as follows:
Lysosomal sorting signals>ER retention/recycling signals>TGN retention/recycling signals>endocytosis signals.

The tunability using different sorting signals and/or different positions of sorting signals within the protein is especially useful when one considers the expression of multiple proteins, each with their own relative expression. For example, consider a nucleic acid construct having the following structure:

A-X-B-Y-C

in which
A, B and C are nucleic acid sequences encoding polypeptides; and
X and Y are nucleic acid sequences encoding cleavage sites.

The nucleic acid construct will encode three proteins *A, B* and *C*, any or all of which may be CARs. For example, *B* and *C* may be CARs which comprise an intracellular retention signal. If it is desired for A, B and C to be expressed such that the relative levels are *A>B>C,* then the nucleic acid sequence A may have no intracellular retention signal, the nucleic acid sequence B may have an intracellular retention signal that causes a small proportion of protein *B* to be retained in the cell (i.e. not to be expressed at the cell surface), and the nucleic acid sequence C may have an intracellular retention signal that causes a large proportion of protein *C* to be retained in the cell.

As explained below, differential amounts of intracellular retention, leading to different amounts of cell surface expression may be achieved by:
(a) using different intracellular retention signals for the proteins; and/or
(b) having the intracellular retention signal located at a different position in the proteins.

### SIGNAL TYPES

Numerous proteins which include an intracellular retention signal and are directed to an intracellular compartment are known in the art.

The intracellular retention signal may be a retention signal from a protein which resides in the lysosomal, endosomal or Golgi compartment.

Intracellular retention signals are well known in the art (see, for example, Bonifacino & Traub; Annu. Rev. Biochem.; 2003; 72; 395-447).

The intracellular retention signal may be a tyrosine-based sorting signal, a dileucine-based sorting signal, an acidic cluster signal, a lysosomal avoidance signal, an NPFX'(1,2)D-Type signal, a KDEL, a KKX'X' or a KX'KX'X' signal (wherein X' is any amino acid).

Tyrosine-based sorting signals mediate rapid internalization of transmembrane proteins from the plasma membrane and the targeting of proteins to lysosomes (Bonifacino & Traub; as above). Two types of tyrosine-based sorting signals are represented by the NPX'Y and YX'X'Z' consensus motifs (wherein Z' is an amino acid with a bulky hydrophobic side chain).

NPX'Y signals have been shown to mediate rapid internalization of type I transmembrane proteins, they occur in families such as members of the LDL receptor, integrin β, and β-amyloid precursor protein families.

Examples of NPX'Y signals are provided in Table 7.

**Table 7 - NPX'Y signals**

| **Protein** | **Species** | **Sequence** |
|---|---|---|
| LDL receptor | Human | Tm-10-INFD**NP**V**Y**QKTT-29 |
| LRP1 (1) | Human | Tm-21-VEIG**NP**T**Y**KMYE-64 |
| LRP1 (2) | Human | Tm-55-TNFT**NP**V**Y**ATLY-33 |
| LRP1 | *Drosophila* | Tm-43-GNFA**NP**V**Y**ESMY-38 |
| LRP1 (1) | *C. elegans* | Tm-54-TTFT**NP**V**Y**ELED-91 |
| LRP1 (2) | *C. elegans* | Tm-140-LRVD**NP**L**Y**DPDS-4 |
| Megalin (1) | Human | Tm-70-IIFE**NP**M**Y**SARD-125 |
| Megalin (2) | Human | Tm-144-TNFE**NP**I**Y**AQME-53 |
| | | |
| Integrin 13-1 (1) | Human | Tm-18-DTGE**NP**I**Y**KSAV-11 |
| Integrin 13-1 (2) | Human | Tm-30-TTVV**NP**K**Y**EGK |
| Integrin 13 (1) | *Drosophila* | Tm-26-WDTE**NP**I**Y**KQAT-11 |
| Integrin 13 (2) | *Drosophila* | Tm-35-STFK**NP**M**Y**AGK |
| APLP1 | Human | Tm-33-HGYE**NP**T**Y**RFLE-3 |
| APP | Human | Tm-32-NGYE**NP**T**Y**KFFE-4 |
| APP-like | *Drosophila* | Tm-38-NGYE**NP**T**Y**KYFE-3 |
| Insulin receptor | Human | Tm-36-YASS**NP**E**Y**LSAS-379 |
| EGR receptor (1) | Human | Tm-434-GSVQ**NP**V**Y**HNQP-96 |
| EGR receptor (2) | Human | Tm-462-TAVG**NP**E**Y**LNTV-68 |
| EGR receptor (3) | Human | Tm-496-ISLD**NP**D**Y**QQDF-34 |

| | | |
|---|---|---|
| Numbers in parentheses indicate motifs that are present in more than one copy within the same protein. The signals in this and other tables should be considered examples. Key residues are indicated in bold type. Numbers of amino acids before (i.e., amino-terminal) and after (i.e., carboxy-terminal) the signals are indicated. Abbreviations: Tm, transmembrane; LDL, low density lipoprotein; LRP1, LDL receptor related protein 1; APP, 13-amyloid precursor protein; APLP1, APP-like protein 1. | | |

YX'X'Z'-type signals are found in endocytic receptors such as the transferrin receptor and the asialoglycoprotein receptor, intracellular sorting receptors such as the Cl- and CD-MPRs, lysosomal membrane proteins such as LAMP-1 and LAMP-2, and TGN proteins such as TGN38 and furin, as well as in proteins localized to specialized endosomal-lysosomal organelles such as antigen-processing compartments (e.g., HLA-DM) and cytotoxic granules (e.g., GMP-17). The YX'X'Z'-type signals are involved in the rapid internalization of proteins from the plasma membrane. However, their function is not limited to endocytosis, since the same motifs have been implicated in the targeting of transmembrane proteins to lysosomes and lysosome-related organelles.

Examples of YX'X'Z'-type signals are provided in Table 8.

**Table 8 - YX'X'Z'-type signals**

| **Protein** | **Species** | **Sequence** |
|---|---|---|
| LAMP-1 | Human | Tm-RKRSHAG**Y**QT**I** |
| LAMP-2a | Human | Tm-KHHHAG**Y**EQ**F** |
| LAMP-2a | Chicken | Tm-KKHHNTG**Y**EQ**F** |
| LAMP-2b | Chicken | Tm-RRKSRTG**Y**QS**V** |
| LAMP-2c | Chicken | Tm-RRKSYAG**Y**QT**L** |
| LAMP | *Drosophila* | Tm-RRRSTSRG**Y**MS**F** |
| LAMP | Earthworm | Tm-RKRSRRG**Y**ES**V** |
| CD63 | Human | Tm-KSIRSG**Y**EV**M** |
| GMP-17 | Human | Tm-HCGGPRPG**Y**ET**L** |
| GMP-17 | Mouse | Tm-HCRTRRAE**Y**ET**L** |
| CD68 | Human | Tm-RRRPSA**Y**QA**L** |
| CDlb | Human | Tm-RRRS**Y**QN**I**P |
| CDlc | Human | Tm-KKHCS**Y**QD**I**L |
| CDld | Mouse | Tm-RRRSA**Y**QD**I**R |
| CD1 | Rat | Tm-RKRRRS**Y**QD**I**M |
| Endolyn | Rat | Tm-KFCKSKERN**Y**HT**L** |
| Endolyn | *Drosophila* | Tm-KFYKARNERN**Y**HT**L** |
| TSC403 | Human | Tm-KIRLRCQSSG**Y**QR**I** |
| TSC403 | Mouse | Tm-KIRQRHQSSA**Y**QR**I** |
| Cystinosin | Human | Tm-HFCLYRKRPG**Y**DQ**L**N |
| Putative solute carrier | Human | Tm-12-SLSRGSG**Y**KE**I** |
| TRP-2 | Human | Tm-RRLRKG**Y**TP**L**MET-11 |
| HLA-DM ◆ | Human | Tm-RRAGHSS**Y**TP**L**PGS-9 |
| LmpA | Dictyostelium | Tm-KKLRQQKQQG**Y**QA**I**INNE |
| Putative lysosomal protein | Dictyostelium | Tm-RSKSNQNQS**Y**NL**I**QL |
| LIMP-II | Dictyostelium | Tm-RKTFYNNNQYNG**Y**NI**I**N |
| Transferrin receptor | Human | 16-PLS**Y**TR**F**SLA-35-Tm |
| Asialoglycoprotein receptor H1 | Human | MTKE**Y**QD**L**QHL-29-Tm |
| CI-MPR | Human | Tm-22-SYTC**Y**SK**V**NKE-132 |
| CD-MPR | Human | Tm-40-PAA**Y**RG**V**GDD-16 |
| CTLA-4 | Human | Tm-10-TGV**Y**VK**M**PPT-16 |
| Furin | Human | Tm-17-LIS**Y**KG**L**PPE-29 |
| TGN38 | Rat | Tm-2 3-ASD**Y**QR**L**NLKL |
| gp41 | HIV-1 | Tm-13-RQG**Y**SP**L**SFQT-144 |
| Acid phosphatase | Human | Tm-RMQAQPPG**Y**RH**V**ADGEDHA |

| | | |
|---|---|---|
| See legend to Table 7 for explanation of signal format | | |

Dileucine-based sorting signals ([DE]X'X'X'LL[LI]) play critical roles in the sorting of many type I, type II, and multispanning transmembrane proteins. Dileucine-based sorting signals are involved in rapid internalization and lysosomal degradation of transmembrane proteins and the targeting of proteins to the late endosomal-lysosomal compartments. Transmembrane proteins that contain constitutively active forms of this signal are mainly localised to the late endosomes and lysosomes.

Examples of [DE]X'X'X'LL[LI] sorting signals are provided in Table 9.

**Table 9 - [DE])X'X'X'LL[LI] sorting signals**

| **Protein** | **Species** | **Signal** |
|---|---|---|
| CD3-Y | Human | Tm-8-S**D**KQT**LL**PN-26 |
| LIMP-II | Rat | Tm-11-D**E**RAP**LI**RT |
| Nmb | Human | Tm-37-Q**E**KDP**LL**KN-7 |
| QNR-71 | Quail | Tm-37-T**E**RNP**LL**KS-5 |
| Pme117 | Human | Tm-33-G**E**NSP**LL**SG-3 |
| Tyrosinase | Human | Tm-8-E**E**KQP**LL**ME-12 |
| Tyrosinase | Medaka fish | Tm-16-G**E**RQP**LL**QS-13 |
| Tyrosinase | Chicken | Tm-8-P**E**IQP**LL**TE-13 |
| TRP-1 | Goldfish | Tm-7-E**G**RQP**LL**GD-15 |
| TRP-1 | Human | Tm-7-E**A**NQP**LL**TD-20 |
| TRP-1 | Chicken | Tm-7-E**L**HQP**LL**TD-20 |
| TRP-2 | Zebrafish | Tm-5-R**E**FEP**LL**NA-11 |
| VMAT2 | Human | Tm-6-E**E**KMA**IL**MD-29 |
| VMAT1 | Human | Tm-6-E**E**KLA**IL**SQ-32 |
| VAchT | Mouse | Tm-10-S**E**RDV**LL**DE-42 |
| VAMP4 | Human | 19-S**E**RRN**LL**ED-88-Tm |
| Neonatal FcR | Rat | Tm-16-D**D**SGD**LL**PG-19 |
| CD4 | Human | Tm-12-S**Q**IKR**LL**SE-17 |
| CD4 | Cat | Tm-12-S**H**TKR**LL**SE-17 |
| GLUT4 | Mouse | Tm-17-R**R**TPS**LL**EQ-17 |
| GLUT4 | Human | Tm-17-H**R**TPS**LL**EQ-17 |
| IRAP | Rat | 46-EP**R**GSR**LL**VR-53-Tm |
| Ii | Human | MD**D**QRD**LI**SNNEQLP**ML**GR-11-Tm |
| Ii | Mouse | MD**D**QRD**LI**SNHEQLP**IL**GN-10-Tm |
| Ii | Chicken | MAE**E**QRD**LI**SSDGSSG**VL**PI-12-Tm |
| Ii-1 | Zebrafish | MEPDH**Q**NES**LI**QRVPSAET**IL**GR-12-Tm |
| Ii-2 | Zebrafish | MSSEG**NE**TP**LI**SDQSSVNvAGPQP-8-Tm |
| Lamp | TRypanosome | Tm-RPRRRT**E**EDE**LL**PE**E**AEG**LI**DPQN |
| Menkes protein | Human | Tm-74-P**D**KHS**LL**VGDFREDDDTAL |
| NPC1 | Human | Tm-13-T**E**RER**LL**NF |
| AQP4 | Human | Tm-32-V**E**TDD**LI**L-29 |
| RME-2 | *C. elegans* | Tm-104-F**E**NDS**LL** |
| Vam3p | *S. cerevisiae* | 153-N**E**QSP**LL**HN-121-Tm |
| ALP | *S. cerevisiae* | 7-S**E**QTR**LV**P-18-Tm |
| Gap1p | *S. cerevisiae* | Tm-23-E**V**QLD**LL**K-24 |

| | | |
|---|---|---|
| See legend to Table 7 for explanation of signal format. | | |

DX'X'LL signals constitute a distinct type of dileucine-based sorting signals. These signals are present in several transmembrane receptors and other proteins that cycle between the TGN and endosomes, such as the Cl- and CD-MPRs, sortilin, the LDL-receptor-related proteins LRP3 and LRP10, and β-secretase.

Examples of DX'X'LL sorting signals are provided in Table 10.

**Table 10 - DX'X'LL sorting signals**

| **Protein** | **Species** | **Sequence** |
|---|---|---|
| CI-MPR | Human | Tm-151-SFHDDS**D**ED**LL**HI |
| CI-MPR | Bovine | Tm-150-TFHDDS**D**ED**LL**HV |
| CI-MPR | Rabbit | Tm-151-SFHDDS**D**ED**LL**NI |
| CI-MPR | Chicken | Tm-148-SFHDDS**D**ED**LL**NV |
| CD-MPR | Human | Tm-5 4-EESEER**D**DH**LL**PM |
| CD-MPR | Chicken | Tm-54-DESEER**D**DH**LL**PM |
| Sortilin | Human | Tm-41-GYHDDS**D**ED**LL**E |
| SorLA | Human | Tm-41-1TGFSD**D**VP**MV**IA |
| Head-activator BP | Hydra | Tm-41-INRFSD**D**EP**LV**VA |
| LRP3 | Human | Tm-237-MLEASD**D**EA**LL**VC |
| ST7 | Human | Tm-330-KNETSD**D**EA**LL**LC |
| LRP10 | Mouse | Tm-235-WWEAEDEP**LL**A |
| LRP10 | Human | Tm-237-WVAEAE**D**EP**LL**T |
| Beta-secretase | Human | Tm-9-HDDFA**D**DIS**LL**K |
| Mucolipin-1 | Mouse | Tm-4 3-GRDSPE**D**HS**LL**VN |
| Nonclassical MHC-I | Deer mouse | Tm-6-VRCHPE**D**DR**LL**G |
| FLJ30532 | Human | Tm-8 3-HRVSQD**D**LD**LL**TS |
| GGA1 | Human | 350-ASVSLLD**D**E**LM**SL-275 |
| GGA1 | Human | 415-ASSGLD**D**LD**LL**GK-211 |
| GGA2 | Human | 408-VQNPSA**D**RN**LL**DL-192 |
| GGA3 | Human | 384-NALSWL**D**EE**LL**CL-326 |
| GGA | *Drosophila* | 447-TVDSID**D**VP**LL**SD-116 |

| | | |
|---|---|---|
| See legend to Table 7 for explanation of signal format. Serine and threonine residues are underlined. | | |

Another family of sorting motifs is provided by clusters of acidic residues containing sites for phosphorylation by CKII. This type of motif is often found in transmembrane proteins that are localized to the TGN at steady state, including the prohormone-processing enzymes furin, PC6B, PC7, CPD, and PAM, and the glycoprotein E of herpes virus 3.

Examples of acidic cluster signals are provided in Table 11.

**Table 11 - Acidic cluster sorting signals**

| **Protein** | **Species** | **Sequence** |
|---|---|---|
| Furin | Mouse | Tm-31-Q**EE**CPS**D**S**EEDE**G-14 |
| PC6B (1)^{a} | Mouse | m-39-R**D**R**D**Y**DEDDEDD**I-36 |
| PC6B (2) | Mouse | Tm-69-L**DE**T**EDDE**L**E**Y**DDE**S-4 |
| PC7 | Human | Tm-38-K**D**P**DE**V**E**T**E**S-47 |
| CPD | Human | Tm-36-H**E**FQ**DE**T**D**T**EEET**-6 |
| PAM | Human | Tm-59-Q**E**K**EDD**GS**E**S**EEE**Y-12 |
| VMAT2 | Human | Tm-35G**EDEE**S**E**S**D** |
| VMAT1 | Human | Tm-3 5-G**ED**S**DEE**P**D**H**EE** |
| VAMP4 | Human | 25-L**EDD**S**DEEED**F-81-Tm |
| Glycoprotein B | HCMV | Tm-125-K**D**S**DEEE**NV |
| Glycoprotein E | Herpes virus 3 | Tm-28-F**ED**S**E**ST**D**T**EEE**F-21 |
| Nef | HIV-1 (AAL65476) | 55-L**E**AQ**EEEE**V-139 |
| Kex1p (1) | *S. cerevisiae* | Tm-29-A**DD**L**E**SGLGA**ED**DL**E**Q**DE**Q**LE**G-40 |
| Kex1p (2) | *S. cerevisiae* | Tm-79-T**E**I**DE**SF**E**MT**D**F |
| Kex2p | *S. cerevisiae* | Tm-36-T**E**P**EE**V**ED**F**D**F**D**LS**DED**H-61 |
| Vps10p | *S. cerevisiae* | Tm-112-F**E**I**EEDD**VPTL**EEE**H-37 |

| | | |
|---|---|---|
| See legend to Table 7 for explanation of signal format. Serine and threonine residues are underlined. ^{a}The number in parentheses is the motif number. | | |

The KDEL receptor binds protein in the ER-Golgi intermediate compartment, or in the early Golgi and returns them to the ER. Although the common mammalian signal is KDEL, it has been shown that the KDEL receptor binds the sequence HDEL more tightly (Scheel et al; J. Biol. Chem. 268; 7465 (1993)). The intracellular retention signal may be HDEL.

KKX'X' and KX'KX'X' signals are retrieval signals which can be placed on the cytoplasmic side of a type I membrane protein. Sequence requirements of these signals are provided in detail by Teasdale & Jackson (Annu. Rev. Cell Dev. Biol.; 12; 27 (1996)).

The intracellular retention signal may be selected from the group of: NPX'Y, YX'X'Z, [DE]X'X'X'L[LI], DX'X'LL, DP[FW], FX'DX'F, NPF, LZX'Z[DE], LLDLL, PWDLW, KDEL, HDEL, KKX'X' or KX'KX'X'; wherein X' is any amino acid and Z' is an amino acid with a bulky hydrophobic side chain.

The intracellular retention signal may be any sequence shown in Tables 7 to 11.

The intracellular retention signal may comprise the Tyrosinase-related protein (TYRP)-1 intracellular retention signal. The intracellular retention signal may comprise the TYRP-1 intracellular domain. The intracellular retention signal may comprise the sequence NQPLLTD (SEQ ID No. 1).

TYRP1 is a well-characterized melansomal protein which is retained in the melanosome (a specialized lysosome) at >99% efficiency. TYRP1 is a 537 amino acid transmembrane protein with a lumenal domain (1-477aa), a transmembrane domain (478-501), and a cytoplasmic domain (502-537). A di-leucine signal residing on the cytoplasmic domain causes retention of the protein. This di-leucine signal has the sequence shown as SEQ ID No. 1 (NQPLLTD).

The intracellular retention signal may be in the endodomain of the CAR. In other words, the intracellular retention signal may be in the domain of the transmembrane protein which would be on the intracellular side of the cell membrane if the protein was correctly expressed at the cell surface.

The intracellular retention signal may be proximal to the transmembrane domain, for instance being immediately connected to it. The intracellular retention signal may be distal to the transmembrane domain - for instance at the carboxy-terminus of the endodomain. The positioning of the retention signal modulates its activity allowing "tuning" of the relative expression of two proteins. For instance in the case of the TYRP1 di-leucine motif, proximal placement results in low-level surface expression, while distal placement results in intermediate surface expression, as shown in the Examples.

### POLYPEPTIDE OF INTEREST

Any or all of A or B; or A, B or C of the nucleic acid sequences in the constructs defined herein may encode a CAR which may or may not comprise an intracellular retention signal.

The nucleic acid construct may comprise one or more further nucleic acid sequence(s) which encode polypeptide of interest (POIs). For example, the POI(s) may be an intracellular protein such as a nucleic protein, a cytoplasmic protein or a protein localised to a membrane-bound compartment; a secretory protein or a transmembrane protein.

The POI may be a suicide gene and/or a marker gene.

### SUICIDE/MARKER GENE

A suicide gene is a gene encoding a polypeptide which, when expressed by a cell enables that cell to be deleted.

A marker gene is a gene encoding a polypeptide which enables selection of a cell expressing that polypeptide.

Various suicide and marker genes are known in the art. WO2013/153391 describes compact polypeptide which comprises both a marker moiety and a suicide moiety. The polypeptide may be co-expressed with a therapeutic transgene, such as a gene encoding a CAR.

The marker moiety comprises a minimal epitope of CD34 which allows efficient selection of transduced cells using, for example, the Miltenyi CD34 cliniMACS system.

The suicide moiety comprises a minimal epitope based on the epitope from CD20. Cells expressing a polypeptide comprising this sequence can be selectively killed using a lytic antibody such as Rituximab.

The combined marker and suicide polypeptide is stably expressed on the cell surface after, for example, retroviral transduction of its encoding sequence.

The marker/suicide polypeptide may have the formula:

St-R1-S1-Q-S2-R2

wherein
St is a stalk sequence which, when the polypeptide is expressed at the surface of a target cell, causes the R and Q epitopes to be projected from the cell surface;
R1 and R2 are a Rituximab-binding epitopes;
S1 and S2 are optional spacer sequences, which may be the same or different; and
Q is a QBEnd10-binding epitope.

The polypeptide may comprise the sequence shown as SEQ ID No.48, or a variant thereof which has at least 80% identity with the sequence shown as SEQ ID No. 48 and which (i) binds QBEND10; (ii) binds Rituximab and (iii) when expressed on the surface of a cell, induces complement-mediated killing of the cell in the presence of Rituximab.

The present invention provides a method for deleting a cell which expresses such a marker/suicide gene, which comprises the step of exposing the cells to rituximab.

### CELL

The present invention relates to a cell which co-expresses a first CAR and a second CAR at the cell surface. The cell expresses a nucleic acid construct according to the first aspect of the invention.

The cell may be any eukaryotic cell capable of expressing a CAR at the cell surface, such as an immunological cell.

In particular the cell may be an immune effector cell such as a T cell or a natural killer (NK) cell.

T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface. There are various types of T cell, as summarised below.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytotoxic T cells (TC cells, or CTLs) destroy virally infected cells and tumor cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T cells with both myeloid (CD11c+) and plasmacytoid (CD123+) dendritic cells that have been activated with TSLP. Naturally occurring Treg cells can be distinguished from other T cells by the presence of an intracellular molecule called FoxP3. Mutations of the FOXP3 gene can prevent regulatory T cell development, causing the fatal autoimmune disease IPEX.

Adaptive Treg cells (also known as Tr1 cells or Th3 cells) may originate during a normal immune response.

The T cell of the invention may be any of the T cell types mentioned above, in particular a CTL.

Natural killer (NK) cells are a type of cytolytic cell which forms part of the innate immune system. NK cells provide rapid responses to innate signals from virally infected cells in an MHC independent manner

NK cells (belonging to the group of innate lymphoid cells) are defined as large granular lymphocytes (LGL) and constitute the third kind of cells differentiated from the common lymphoid progenitor generating B and T lymphocytes. NK cells are known to differentiate and mature in the bone marrow, lymph node, spleen, tonsils and thymus where they then enter into the circulation.

The CAR cells of the invention may be any of the cell types mentioned above.

CAR- expressing cells, such as CAR-expressing T or NK cells may either be created *ex vivo* either from a patient's own peripheral blood (1st party), or in the setting of a haematopoietic stem cell transplant from donor peripheral blood (2nd party), or peripheral blood from an unconnected donor (3rd party).

The present invention also provide a cell composition comprising CAR-expressing cells, such as CAR-expressing T and/orNK cells, according to the present invention. The cell composition may be made by transducing a blood-sample *ex vivo* with a nucleic acid construct according to the present invention.

Alternatively, CAR-expressing cells may be derived from *ex vivo* differentiation of inducible progenitor cells or embryonic progenitor cells to the relevant cell type, such as T cells. Alternatively, an immortalized cell line such as a T-cell line which retains its lytic function and could act as a therapeutic may be used.

In all these embodiments, CAR cells may be generated by introducing DNA or RNA coding for the CARs by one of many means including transduction with a viral vector, transfection with DNA or RNA.

A CAR T cell of the invention may be an *ex vivo* T cell from a subject. The T cell may be from a peripheral blood mononuclear cell (PBMC) sample. T cells may be activated and/or expanded prior to being transduced with CAR-encoding nucleic acid, for example by treatment with an anti-CD3 monoclonal antibody.

A CAR T cell of the invention may be made by:
(i) isolation of a T cell-containing sample from a subject or other sources listed above; and
(ii) transduction or transfection of the T cells with a nucleic acid construct encoding the first and second CAR.

The T cells may then by purified, for example, selected on the basis of co-expression of the first and second CAR.

### VECTOR

The present invention also provides a vector which comprises a CAR-encoding nucleic acid construct as defined herein. Such a vector may be used to introduce the nucleic acid sequence(s) into a host cell so that it expresses the first and second CARs.

The vector may, for example, be a plasmid or a viral vector, such as a retroviral vector or a lentiviral vector, or a transposon based vector or synthetic mRNA.

The vector may be capable of transfecting or transducing a T cell.

### PHARMACEUTICAL COMPOSITION

The present invention also relates to a pharmaceutical composition containing a plurality of CAR-expressing cells, such as T cells or NK cells, of the invention. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### METHOD OF TREATMENT

The cells of the present invention may be capable of killing target cells, such as cancer cells. The target cell may be recognisable by a defined pattern of antigen expression, for example the expression of antigen A OR antigen B.

The cells of the present invention may be used for the treatment of an infection, such as a viral infection.

The cells of the invention may also be used for the control of pathogenic immune responses, for example in autoimmune diseases, allergies and graft-vs-host rejection.

The cells of the invention may be used for the treatment of a cancerous disease, such as bladder cancer, breast cancer, colon cancer, endometrial cancer, kidney cancer (renal cell), leukemia, lung cancer, melanoma, non-Hodgkin lymphoma, pancreatic cancer, prostate cancer and thyroid cancer.

It is particularly suited for treatment of solid tumours where the availability of good selective single targets is limited.

The cells of the invention may be used to treat: cancers of the oral cavity and pharynx which includes cancer of the tongue, mouth and pharynx; cancers of the digestive system which includes oesophageal, gastric and colorectal cancers; cancers of the liver and biliary tree which includes hepatocellular carcinomas and cholangiocarcinomas; cancers of the respiratory system which includes bronchogenic cancers and cancers of the larynx; cancers of bone and joints which includes osteosarcoma; cancers of the skin which includes melanoma; breast cancer; cancers of the genital tract which include uterine, ovarian and cervical cancer in women, prostate and testicular cancer in men; cancers of the renal tract which include renal cell carcinoma and transitional cell carcinomas of the utterers or bladder; brain cancers including gliomas, glioblastoma multiforme and medullobastomas; cancers of the endocrine system including thyroid cancer, adrenal carcinoma and cancers associated with multiple endocrine neoplasm syndromes; lymphomas including Hodgkin's lymphoma and non-Hodgkin lymphoma; Multiple Myeloma and plasmacytomas; leukaemias both acute and chronic, myeloid or lymphoid; and cancers of other and unspecified sites including neuroblastoma.

Treatment with the cells of the invention may help prevent the escape or release of tumour cells which often occurs with standard approaches.

### METHOD

In a further aspect the present invention relates to a method for modulating the relative cell surface expression of two CARs expressed from a single nucleic acid construct; by including an intracellular retention signal one or both the nucleic acid sequence(s) encoding the CAR(s).

The invention will now be further described by way of Examples, which are meant to serve to assist one of ordinary skill in the art in carrying out the invention and are not intended in any way to limit the scope of the invention.

### EXAMPLES

### Example 1 - Creation of target cell populations

For the purposes of designing and testing an OR gate, receptors based on anti-CD19 and anti-CD33 were arbitrarily chosen. Using retroviral vectors, CD19 and CD33 were cloned. These proteins were truncated so that they do not signal and could be stably expressed for prolonged periods. Next, these vectors were used to transduce the SupT1 cell line either singly or doubly to establish cells negative for both antigen (the wild-type), positive for either and positive for both. The expression data are shown in Figure 3.

### Example 2 - Design and function of the OR gate

To construct the OR gate, a pair of receptors recognizing CD19 and CD33 were co-expressed. Different spacers were used to prevent cross-pairing. Both receptors had a trans-membrane domain derived from CD28 to improve surface stability and an endodomain derived from that of CD3 Zeta to provide a simple activating signal. In this way, a pair of independent 1^{st} generation CARs were co-expressed. The retroviral vector cassette used to co-express the sequences utilizes a foot-and-mouth 2A self-cleaving peptide to allow co-expression 1:1 of both receptors. The cassette design is shown in Figure 4, and the protein structures in Figure 5. The nucleotide sequence of homologous regions was codon-wobbled to prevent recombination during retroviral vector reverse transcription.

### Example 3 - Testing the OR gate

Expression of both CARs was tested on the T-cell surface by staining with cognate antigen fused to Fc. By using different species of Fc domains (mouse for CD19 and rabbit for CD33), co-expression of both CARs was determined on the cell surface by staining with different secondary antibodies conjugated with different fluorophores. This is shown in Figure 6.

Functional testing was then carried out using the mouse T-cell line BW5147. This cell line releases IL2 upon activation allowing a simple quantitative readout. These T-cells were co-cultured with increasing amounts of the artificial target cells described above. T-cells responded to target cells expressing either antigen, as shown by IL2 release measured by ELISA. Both CARs were shown to be expressed on the cell surfaces and the T-cells were shown to respond to either or both antigens. These data are shown in Figure 7.

### Example 4 - Dissection of TYRP1 lysozomal retention signals

The ability of the Tyrosinase-related protein 1 (TYRP1) retention signal to cause retention of a polypeptide when in the context of a more complex endodomain was determined using a number of constructs (Figure 9). The wild-type construct was compared with constructs where enhanced Green Fluorescent Protein (eGFP) was added or replaced the TYRP1 endodomain. Where eGFP was added, it was placed either after or before the native endodomain so the retention signal was either in its native location (just under the membrane), or distal to it.

All constructs are co-expressed with IRES.CD34. Staining of transduced SupT1 cells is shown with intracellular and surface staining in Figure 9.

It was found that replacement of the endodomain resulted in very bright surface expression, introduction of eGFP after the retention signal to almost no surface expression and introduction before the retention signal to intermediate surface expression (Figure 9).

### Example 5 - Modulation of the relative expression of a transmembrane protein co-expressed from a single expression cassette with a separate protein

An expression cassette encoding two CAR transmembrane proteins was modified such that one of the CAR proteins had the lysozomal retention signal from TYRP1 introduced either proximal or distal to the membrane. Expression of each of these two new variants at the cell surface was compared with that of the original unmodified CAR protein.

PBMCs were isolated from blood and stimulated using PHA and IL-2. Two days later the cells were transduced on retronectin coated plates with retro virus containing the CD19:CD33 CAR construct. On day 5 the expression level of the two CARs translated by the construct was evaluated via flow cytometry and the cells were depleted of CD56+ cells (predominantly NK cells). On day 6 the PBMCs were placed in a co-culture with target cells at a 1:2 effector to target cell ratio. On day 8 the supernatant was collected and analysed for IFN-gamma secretion via ELISA.

The pattern observed with Tyrp1-eGFP fusions was observed with some reduction of expression of modified transmembrane protein with the distal retention signal and marked reduction in the case of proximal retention signal. As expected, expression of the second transmembrane protein from the cassette was not altered (Figure 10).

### Example 6 - Modulation of expression using a retention signal from the Adenoviral E3/19K protein

The human adenovirus E3/19K protein is a type I transmembrane glycoprotein of the Endoplasmic Reticulum/Golgi that abrogates cell surface transport of major histocompatibility complex class I (MHC-I) and MHC-I-related chain A and B (MICA/B) molecules. The retention motif was identified to be depended on the cytosolic tail of the adenovirus E3/19K protein. More specifically, the last 6aa DEKKMP was found to be the most important for retention. The optimal positioning was found to be at the c-terminus of the protein.

An expression cassette encoding two CAR transmembrane proteins, as described in Example 5, was modified such that one of the CAR proteins had the retention motif from adenovirus E3/19K protein. In this experiment, the retention motif on the second CAR in the expression cassette (the anti-CD33 inhibitory CAR).

Constructs were generated comprising either the entire cytosolic tail of adenovirus E3/19K protein or only the last 6aa from E3/19K (DEKKMP), which were found to be critical for its Golgi/ER retention ability (Figure 11). These constructs were transfected into 293T cells and stained primarily with a chimeric soluble CD19-Rabbit Fc and a chimeric soluble CD33-Mouse Fc proteins. These cells were then subsequently stained with an anti-Rabbit Fc-FITC and an anti-Mouse Fc-APC (Figure 12). These cells show a clear retention when the full length adenovirus E3/19K protein, or the DEKKMP motif, was placed on the anti-CD33 receptor but had no effect on anti-CD19 receptor expression levels.

### Example 7 - Modulation of expression by altering the signal peptide - swapping in the murine Ig kappa chain V-III signal sequence

PCT/GB2014/053452 describes a vector system encoding two chimeric antigen receptors (CARs), one against CD19 and one against CD33. The signal peptide used for the CARs in that study was the signal peptide from the human CD8a signal sequence. For the purposes of this study, this was substituted with the signal peptide from the murine Ig kappa chain V-III region, which has the sequence: METDTLILWVLLLLVPGSTG (hydrophobic residues hightlited in bold). In order to establish that the murine Ig kappa chain V-III signal sequence functioned as well as the signal sequence from human CD8a, a comparative study was performed. For both signal sequences, functional expression of the anti-CD33 CAR and the anti-CD19 CAR was observed. This substituted signal sequence and all subsequent mutations thereof were transiently transfected into 293T cells. Three days after transfection the 293T cells were stained with both soluble chimeric CD19 fused with rabbit Fc chain and soluble chimeric CD33 fused with mouse Fc chain. All cells were then stained with anti-Rabbit Fc-FITC and anti-mouse Fc-APC. Flow cytometry plots show the substituted signal sequence as a comparison with non-transfected (NT) and the construct with Cd8 signal sequences (Figure 15). The murine Ig kappa chain V-III signal sequence was found to function as well as the signal sequence from human CD8a.

### Example 8 - Altering relative expression by deleting hydrophobic residues in the signal peptide.

Hydrophobic residues were deleted in a stepwise fashion and the effect on the relative expression of the anti-CD33 CAR and the anti-CD19 CAR was observed. The effect of one, two, three and four amino acid deletions was investigated and the results are shown in Figures 16 to 19 respectively.

All mutant constructs showed a decrease in relative expression of the anti-CD19 CAR compared to the anti-CD33 CAR. The relative decrease of anti-CD19 CAR expression was greater with a greater number of amino acid deletions from 1 to 3, but then plateaued out (four deletions gave a similar decrease in expression as three deletions).

## Claims

1. A nucleic acid construct comprising the following structure:
A-X-B
in which
A and B are nucleic acid sequences encoding a first chimeric antigen receptor (CAR) and a second CAR; and
X is a nucleic acid sequence which encodes a cleavage site,
wherein
the first CAR and the second CAR recognise different antigens;
the first CAR and the second CAR each comprise an activating endodomain; and wherein
(a) the first CAR and/or second CAR comprises an intracellular retention signal selected from the following group: an endocytosis signal; a Golgi retention signal; a *trans*-Golgi network (TGN) recycling signal; an endoplasmic reticulum (ER) retention signal; and a lysosomal sorting signal; and/or
(b) the signal peptide of the first CAR or second CAR comprises one or more mutation(s) such that the signal peptide of the first CAR and second CAR differ in their number of hydrophobic amino acids and wherein one signal peptide comprises up to five more hydrophobic amino acids than the other signal peptide.

2. A nucleic acid construct according to claim 1, wherein the first CAR and/or second CAR comprise(s) an intracellular retention signal which directs the CAR to a membrane-bound intracellular compartment.

3. A nucleic acid construct according to claim 1 or 2, wherein the first CAR and/or second CAR comprise(s) an intracellular retention signal which directs the CAR to a lysozomal, endosomal or Golgi compartment.

4. A nucleic acid construct according to claim 1, wherein the signal peptide of the first CAR or second CAR comprises one or more mutation(s) such that it has fewer hydrophobic amino acids than a) its wild-type sequence or b) the signal peptide of the other CAR.

5. A nucleic acid construct according to claim 4, wherein the hydrophobic amino acid(s) removed or replaced by mutation is/are selected from the group: Alanine (A); Valine (V); Isoleucine (I); Leucine (L); Methionine (M); Phenylalanine (P); Tyrosine (Y); Tryptophan (W).

6. A nucleic acid construct according to any preceding claim comprising the following structure:
A-X-B-Y-C
in which
A, B and C are nucleic acid sequences encoding a first, second and third polypeptides of interest (POIs); and
X and Y are nucleic acid sequences which may be the same or different, each of which encodes a cleavage site,
wherein at least two of the POIs are chimeric antigen receptors (CARs) which
(a) comprise an intracellular retention signal; and/or
(b) differ in the number of hydrophobic amino acids in their signal peptides.

7. A nucleic acid construct according to claim 6, encoding two CARs which comprise an intracellular retention signal, wherein the two CARs:
(a) comprise different intracellular retention signals; and/or
(b) have the intracellular retention signal located at a different position in the CAR, such that when the nucleic acid is expressed in a cell, there is differential relative expression of the two CARs at the cell surface.

8. A nucleic acid construct according to claim 6 or 7, wherein the third POI is a polypeptide which enables selection of transduced cells and/or enables cells expressing the polypeptide to be deleted.

9. A vector comprising a nucleic acid construct according to any preceding claim.

10. A cell comprising a nucleic acid construct according to any one of claims 1 to 8 or a vector according to claim 9.

11. A method for making a cell according to claim 10, which comprises the step of introducing a nucleic acid construct according to any of claims 1 to 8 or a vector according to claim 9 into a cell *ex vivo.*

12. A pharmaceutical composition comprising a plurality of cells according to claim 10.

13. A pharmaceutical composition according to claim 12 for use in treating and/or preventing a disease.

## Patentansprüche

1. Nukleinsäurekonstrukt, umfassend die folgende Struktur:
A - X - B,
in der
A und B für Nukleinsäuresequenzen stehen, die einen ersten chimären Antigenrezeptor (CAR) und einen zweiten CAR codieren; und
X für eine Nukleinsäuresequenz steht, die eine Spaltungsstelle codiert;
wobei
der erste und der zweite CAR unterschiedliche Antigene erkennen;
der erste und der zweite CAR jeweils eine aktivierende Endodomäne umfassen; und wobei
(a) der erste und/oder zweite CAR ein Intrazelluläre-Retention-Signal umfasst, das aus der folgenden Gruppe ausgewählt ist: einem Endozytosesignal; einem Golgi-Retention-Signal; einem TGN(*trans*-Golgi Network)-Recycling-Signal; einem Endoplasmatisches-Retikulum(ER)-Retention-Signal; und einem Lysosomensortierungssignal; und/oder
(b) das Signalpeptid des ersten oder zweiten CAR eine oder mehrere Mutation(en) umfasst, so dass sich das Signalpeptid des ersten und des zweiten CAR in ihrer Anzahl hydrophober Aminosäuren unterscheiden und wobei ein Signalpeptid bis zu fünf hydrophobe Aminosäuren mehr als das andere Signalpeptid umfasst.

2. Nukleinsäurekonstrukt nach Anspruch 1, wobei der erste CAR und/oder der zweite CAR ein Intrazelluläre-Retention-Signal umfassen bzw. umfasst, das den CAR zu einem membrangebundenen intrazellullären Kompartiment leitet.

3. Nukleinsäurekonstrukt nach Anspruch 1 oder 2, wobei der erste CAR und/oder der zweite CAR ein Intrazelluläre-Retention-Signal umfassen bzw. umfasst, das den CAR zu einem Lysosomen-, Endosomen- oder Golgi-Kompartiment leitet.

4. Nukleinsäurekonstrukt nach Anspruch 1, wobei das Signalpeptid des ersten CAR oder zweiten CAR eine oder mehrere Mutation(en) umfasst, so dass es weniger hydrophobe Aminosäuren als a) seine Wildtypsequenz oder b) das Signalpeptid des anderen CAR aufweist.

5. Nukleinsäurekonstrukt nach Anspruch 4, wobei die durch Mutation entfernte(n) oder ersetzte(n) hydrophobe(n) Aminosäure(n) aus der folgenden Gruppe ausgewählt ist/sind: Alanin (A); Valin (V); Isoleucin (I); Leucin (L); Methionin (M); Phenylalanin (P); Tyrosin (Y); Tryptophan (W).

6. Nukleinsäurekonstrukt nach einem vorhergehenden Anspruch, umfassend die folgende Struktur:
A - X - B - Y - C,
in der
A, B und C für Nukleinsäuresequenzen stehen, die ein erstes, ein zweites und ein drittes Polypeptid von Interesse (POI) codieren; und
X und Y für eine Nukleinsäuresequenz stehen, die gleich oder verschieden sein können und jeweils eine Spaltungsstelle codieren;
wobei es sich bei wenigstens zwei der POI um chimäre Antigenrezeptoren (CAR) handelt, die
(a) ein Intrazelluläre-Retention-Signal umfassen; und/oder
(b) sich in der Anzahl hydrophober Aminosäuren in ihren Signalpeptiden unterscheiden.

7. Nukleinsäurekonstrukt nach Anspruch 6, codierend zwei CAR, die ein Intrazelluläre-Retention-Signal umfassen, wobei die beiden CAR:
(a) unterschiedliche Intrazelluläre-Retention-Signale umfassen; und/oder
(b) das Intrazelluläre-Retention-Signal an einer unterschiedlichen Position im CAR vorliegen haben, so dass bei Expression der Nukleinsäure in einer Zelle differentielle relative Expression der beiden CAR an der Zelloberfläche vorliegt.

8. Nukleinsäurekonstrukt nach Anspruch 6 oder 7, wobei es sich bei dem dritten POI um ein Polypeptid handelt, das eine Selektion transduzierter Zellen und/oder die Deletion von das Polypeptid exprimierenden Zellen ermöglicht.

9. Vektor, umfassend ein Nukleinsäurekonstrukt nach einem vorhergehenden Anspruch.

10. Zelle, umfassend ein Nukleinsäurekonstrukt nach einem der Ansprüche 1 bis 8 oder einen Vektor nach Anspruch 9.

11. Verfahren zur Erzeugung einer Zelle nach Anspruch 10, das den Schritt umfasst, bei dem ein Nukleinsäurekonstrukt nach einem der Ansprüche 1 bis 8 oder ein Vektor nach Anspruch 9 in eine Zelle ex vivo eingeführt wird.

12. Pharmazeutische Zusammensetzung, umfassend mehrere Zellen nach Anspruch 10.

13. Pharmazeutische Zusammensetzung nach Anspruch 12 zur Behandlung und/oder Vorbeugung einer Krankheit.

## Revendications

1. Construction d'acide nucléique comprenant la structure suivante :
A-X-B
dans laquelle
A et B sont des séquences d'acide nucléique codant pour un premier récepteur antigénique chimérique (CAR) et un second CAR ; et
X est une séquence d'acide nucléique qui code pour un site de coupure,
dans laquelle
le premier CAR et le second CAR reconnaissent des antigènes différents ;
le premier CAR et le second CAR comprennent chacun un endodomaine d'activation ; et
dans laquelle
(a) le premier CAR et/ou le second CAR comprennent un signal de rétention intracellulaire choisi dans le groupe suivant : un signal d'endocytose ; un signal de rétention dans l'appareil de Golgi ; un signal de recyclage vers le réseau trans-golgien (RTG) ; un signal de rétention dans le réticulum endoplasmique (RE) ; et un signal de tri lysosomal ; et/ou
(b) le peptide signal du premier CAR ou du second CAR comprend une ou plusieurs mutations telles que le peptide signal du premier CAR et celui du second CAR diffèrent en ce qui concerne leur nombre d'acides aminés hydrophobes et dans laquelle un peptide signal comprend jusqu'à cinq acides aminés hydrophobes de plus que l'autre peptide signal.

2. Construction d'acide nucléique selon la revendication 1, dans laquelle le premier CAR et/ou le second CAR comprennent un signal de rétention intracellulaire qui dirige le CAR vers un compartiment intracellulaire délimité par une membrane.

3. Construction d'acide nucléique selon la revendication 1 ou 2, dans laquelle le premier CAR et/ou le second CAR comprennent un signal de rétention intracellulaire qui dirige le CAR vers un compartiment lysosomal, endosomal ou de Golgi.

4. Construction d'acide nucléique selon la revendication 1, dans laquelle le peptide signal du premier CAR ou du second CAR comprend une ou plusieurs mutations telles qu'il a moins d'acides aminés hydrophobes que a) sa séquence de type sauvage ou b) le peptide signal de l'autre CAR.

5. Construction d'acide nucléique selon la revendication 4, dans laquelle le ou les acides aminés hydrophobes enlevés ou remplacés par mutation sont choisis dans le groupe : alanine (A) ; valine (V) ; isoleucine (I) ; leucine (L) ; méthionine (M) ; phénylalanine (P) ; tyrosine (Y) ; tryptophane (W).

6. Construction d'acide nucléique selon une quelconque revendication précédente comprenant la structure suivante :
A-X-B-Y-C
dans laquelle
A, B et C sont des séquences d'acide nucléique codant pour des premier, deuxième et troisième polypeptides présentant de l'intérêt (POI) ; et
X et Y sont des séquences d'acide nucléique qui peuvent être identiques ou différentes, chacune d'entre elles codant pour un site de coupure,
dans laquelle au moins deux des POI sont des récepteurs antigéniques chimériques (CAR) qui
(a) comprennent un signal de rétention intracellulaire ; et/ou
(b) diffèrent en ce qui concerne le nombre d'acides aminés hydrophobes dans leurs peptides signaux.

7. Construction d'acide nucléique selon la revendication 6, codant pour deux CAR qui comprennent un signal de rétention intracellulaire, dans laquelle les deux CAR :
(a) comprennent des signaux de rétention intracellulaire différents ; et/ou
(b) ont le signal de rétention intracellulaire situé en une position différente dans le CAR,
de façon telle que lorsque l'acide nucléique est exprimé dans une cellule, il y a une expression relative différentielle des deux CAR à la surface cellulaire.

8. Construction d'acide nucléique selon la revendication 6 ou 7, dans laquelle le troisième POI est un polypeptide qui permet la sélection de cellules transduites et/ou qui permet de supprimer des cellules exprimant le polypeptide.

9. Vecteur comprenant une construction d'acide nucléique selon une quelconque revendication précédente.

10. Cellule comprenant une construction d'acide nucléique selon l'une quelconque des revendications 1 à 8 ou un vecteur selon la revendication 9.

11. Procédé pour la production d'une cellule selon la revendication 10, qui comprend l'étape consistant à introduire une construction d'acide nucléique selon l'une quelconque des revendications 1 à 8 ou un vecteur selon la revendication 9 dans une cellule *ex vivo.*

12. Composition pharmaceutique comprenant une pluralité de cellules selon la revendication 10.

13. Composition pharmaceutique selon la revendication 12 destinée à être utilisée en traitement et/ou prévention d'une maladie.


### SEQUENCE LISTING

<110> UCL Business PLC
<120> NUCLEIC ACID CONSTRUCT
<130> P106291PCT
<150> GB1507111.1
   <151> 2015-04-27
<160> 233
<170> PatentIn version 3.5
<210> 1
   <211> 7
   <212> PRT
   <213> Artificial Sequence
<220>
   <223> Tyrosinase-related protein (TYRP)-1 intracellular retention signal
<400> 1
   Asn Gln Pro Leu Leu Thr Asp
   1               5
<210> 2
   <211> 15
   <212> PRT
   <213> Artificial Sequence
<220>
   <223> Adenoviral E3/19K intracellular retention signal
<400> 2
   Lys Tyr Lys Ser Arg Arg Ser Phe Ile Asp Glu Lys Lys Met Pro
   1               5                   10                  15
<210> 3
   <211> 6
   <212> PRT
   <213> Artificial Sequence
<220>
   <223> Adenoviral E3/19K intracellular retention signal
<400> 3
   Asp Glu Lys Lys Met Pro
   1               5
<210> 4
   <211> 1129
   <212> PRT
   <213> Artificial Sequence
<220>
   <223> CAR OR gate which recognizes CD19 OR CD33
<400> 4
<210> 5
   <211> 234
   <212> PRT
   <213> Artificial Sequence
<220>
   <223> spacer sequence, hinge-CH2CH3 of human IgG1
<400> 5
   Ala Glu Pro Lys Ser Pro Asp Lys Thr His Thr Cys Pro Pro Cys Pro
   1 5 10 15
   Ala Pro Pro Val Ala Gly Pro Ser Val Phe Leu Phe Pro Pro Lys Pro
   20 25 30
   Lys Asp Thr Leu Met Ile Ala Arg Thr Pro Glu Val Thr Cys Val Val
   35 40 45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys Lys Asp
   225                 230
<210> 6
   <211> 46
   <212> PRT
   <213> Artificial Sequence
<220>
   <223> spacer sequence, human CD8 stalk
<400> 6
   Thr Thr Thr Pro Ala Pro Arg Pro Pro Thr Pro Ala Pro Thr Ile Ala
   1               5                   10                  15
   Ser Gln Pro Leu Ser Leu Arg Pro Glu Ala Cys Arg Pro Ala Ala Gly
                   20                  25                  30
   Gly Ala Val His Thr Arg Gly Leu Asp Phe Ala Cys Asp Ile
           35                  40                  45
<210> 7
   <211> 20
   <212> PRT
   <213> Artificial Sequence
<220>
   <223> spacer sequence, human IgG1 hinge
<400> 7
   Ala Glu Pro Lys Ser Pro Asp Lys Thr His Thr Cys Pro Pro Cys Pro
   1               5                   10                  15
   Lys Asp Pro Lys
               20
<210> 8
   <211> 185
   <212> PRT
   <213> Artificial Sequence
<220>
   <223> spacer sequence, CD2 ectodomain
<400> 8
   Lys Glu Ile Thr Asn Ala Leu Glu Thr Trp Gly Ala Leu Gly Gln Asp
   1               5                   10                  15
   Ile Asn Leu Asp Ile Pro Ser Phe Gln Met Ser Asp Asp Ile Asp Asp
               20                  25                  30
   Ile Lys Trp Glu Lys Thr Ser Asp Lys Lys Lys Ile Ala Gln Phe Arg
           35                  40                  45
   Lys Glu Lys Glu Thr Phe Lys Glu Lys Asp Thr Tyr Lys Leu Phe Lys
       50                  55                  60
   Asn Gly Thr Leu Lys Ile Lys His Leu Lys Thr Asp Asp Gln Asp Ile
   65                  70                  75                  80
   Tyr Lys Val Ser Ile Tyr Asp Thr Lys Gly Lys Asn Val Leu Glu Lys
                   85                  90                  95
   Ile Phe Asp Leu Lys Ile Gln Glu Arg Val Ser Lys Pro Lys Ile Ser
               100                 105                 110
   Trp Thr Cys Ile Asn Thr Thr Leu Thr Cys Glu Val Met Asn Gly Thr
           115                 120                 125
   Asp Pro Glu Leu Asn Leu Tyr Gln Asp Gly Lys His Leu Lys Leu Ser
       130                 135                 140
   Gln Arg Val Ile Thr His Lys Trp Thr Thr Ser Leu Ser Ala Lys Phe
   145                 150                 155                 160
   Lys Cys Thr Ala Gly Asn Lys Val Ser Lys Glu Ser Ser Val Glu Pro
                   165                 170                 175
   Val Ser Cys Pro Glu Lys Gly Leu Asp
               180                 185
<210> 9
   <211> 259
   <212> PRT
   <213> Artificial Sequence
<220>
   <223> spacer sequence, CD34 ectodomain
<400> 9
   Ser Leu Asp Asn Asn Gly Thr Ala Thr Pro Glu Leu Pro Thr Gln Gly
   1               5                   10                  15
   Thr Phe Ser Asn Val Ser Thr Asn Val Ser Tyr Gln Glu Thr Thr Thr
               20                  25                  30
   Pro Ser Thr Leu Gly Ser Thr Ser Leu His Pro Val Ser Gln His Gly
           35                  40                  45
   Asn Glu Ala Thr Thr Asn Ile Thr Glu Thr Thr Val Lys Phe Thr Ser
       50                  55                  60
   Thr Ser Val Ile Thr Ser Val Tyr Gly Asn Thr Asn Ser Ser Val Gln
   65                  70                  75                  80
   Ser Gln Thr Ser Val Ile Ser Thr Val Phe Thr Thr Pro Ala Asn Val
                   85                  90                  95
   Ser Thr Pro Glu Thr Thr Leu Lys Pro Ser Leu Ser Pro Gly Asn Val
               100                 105                 110
   Ser Asp Leu Ser Thr Thr Ser Thr Ser Leu Ala Thr Ser Pro Thr Lys
           115                 120                 125
   Pro Tyr Thr Ser Ser Ser Pro Ile Leu Ser Asp Ile Lys Ala Glu Ile
   130 135 140
   Lys Cys Ser Gly Ile Arg Glu Val Lys Leu Thr Gln Gly Ile Cys Leu
   145 150 155 160
   Glu Gln Asn Lys Thr Ser Ser Cys Ala Glu Phe Lys Lys Asp Arg Gly
   165 170 175
   Glu Gly Leu Ala Arg Val Leu Cys Gly Glu Glu Gln Ala Asp Ala Asp
   180 185 190
   Ala Gly Ala Gln Val Cys Ser Leu Leu Leu Ala Gln Ser Glu Val Arg
   195 200 205
   Pro Gln Cys Leu Leu Leu Val Leu Ala Asn Arg Thr Glu Ile Ser Ser
   210 215 220
   Lys Leu Gln Leu Met Lys Lys His Gln Ser Asp Leu Lys Lys Leu Gly
   225 230 235 240
   Ile Leu Asp Phe Thr Glu Gln Asp Val Ala Ser His Gln Ser Tyr Ser
   245 250 255
   Gln Lys Thr
<210> 10
   <211> 140
   <212> PRT
   <213> Artificial Sequence
<220>
   <223> comprising CD28 transmembrane domain and CD3 Z endodomain
<400> 10
   Phe Trp Val Leu Val Val Val Gly Gly Val Leu Ala Cys Tyr Ser Leu
   1               5                   10                  15
   Leu Val Thr Val Ala Phe Ile Ile Phe Trp Val Arg Arg Val Lys Phe
               20                  25                  30
   Ser Arg Ser Ala Asp Ala Pro Ala Tyr Gln Gln Gly Gln Asn Gln Leu
           35                  40                  45
   Tyr Asn Glu Leu Asn Leu Gly Arg Arg Glu Glu Tyr Asp Val Leu Asp
       50                  55                  60
   Lys Arg Arg Gly Arg Asp Pro Glu Met Gly Gly Lys Pro Arg Arg Lys
   65                  70                  75                  80
   Asn Pro Gln Glu Gly Leu Tyr Asn Glu Leu Gln Lys Asp Lys Met Ala
                   85                  90                  95
   Glu Ala Tyr Ser Glu Ile Gly Met Lys Gly Glu Arg Arg Arg Gly Lys
               100                 105                 110
   Gly His Asp Gly Leu Tyr Gln Gly Leu Ser Thr Ala Thr Lys Asp Thr
           115                 120                 125
   Tyr Asp Ala Leu His Met Gln Ala Leu Pro Pro Arg
       130                 135                 140
<210> 11
   <211> 180
   <212> PRT
   <213> Artificial Sequence
<220>
   <223> comprising CD28 transmembrane domain and CD28 and CD3 Zeta endodomains
<400> 11
   Phe Trp Val Leu Val Val Val Gly Gly Val Leu Ala Cys Tyr Ser Leu
   1               5                   10                  15
   Leu Val Thr Val Ala Phe Ile Ile Phe Trp Val Arg Ser Lys Arg Ser
               20                  25                  30
   Arg Leu Leu His Ser Asp Tyr Met Asn Met Thr Pro Arg Arg Pro Gly
           35                  40                  45
   Pro Thr Arg Lys His Tyr Gln Pro Tyr Ala Pro Pro Arg Asp Phe Ala
       50                  55                  60
   Ala Tyr Arg Ser Arg Val Lys Phe Ser Arg Ser Ala Asp Ala Pro Ala
   65                  70                  75                  80
   Tyr Gln Gln Gly Gln Asn Gln Leu Tyr Asn Glu Leu Asn Leu Gly Arg
                   85                  90                  95
   Arg Glu Glu Tyr Asp Val Leu Asp Lys Arg Arg Gly Arg Asp Pro Glu
               100                 105                 110
   Met Gly Gly Lys Pro Arg Arg Lys Asn Pro Gln Glu Gly Leu Tyr Asn
           115                 120                 125
   Glu Leu Gln Lys Asp Lys Met Ala Glu Ala Tyr Ser Glu Ile Gly Met
       130                 135                 140
   Lys Gly Glu Arg Arg Arg Gly Lys Gly His Asp Gly Leu Tyr Gln Gly
   145                 150                 155                 160
   Leu Ser Thr Ala Thr Lys Asp Thr Tyr Asp Ala Leu His Met Gln Ala
                   165                 170                 175
   Leu Pro Pro Arg
               180
<210> 12
   <211> 216
   <212> PRT
   <213> Artificial Sequence
<220>
   <223> comprising CD28 transmembrane domain and CD28, OX40 and CD3 Zeta endodomains
<400> 12
   Phe Trp Val Leu Val Val Val Gly Gly Val Leu Ala Cys Tyr Ser Leu
   1               5                   10                  15
   Leu Val Thr Val Ala Phe Ile Ile Phe Trp Val Arg Ser Lys Arg Ser
               20                  25                  30
   Arg Leu Leu His Ser Asp Tyr Met Asn Met Thr Pro Arg Arg Pro Gly
           35                  40                  45
   Pro Thr Arg Lys His Tyr Gln Pro Tyr Ala Pro Pro Arg Asp Phe Ala
       50                  55                  60
   Ala Tyr Arg Ser Arg Asp Gln Arg Leu Pro Pro Asp Ala His Lys Pro
   65                  70                  75                  80
   Pro Gly Gly Gly Ser Phe Arg Thr Pro Ile Gln Glu Glu Gln Ala Asp
                   85                  90                  95
   Ala His Ser Thr Leu Ala Lys Ile Arg Val Lys Phe Ser Arg Ser Ala
               100                 105                 110
   Asp Ala Pro Ala Tyr Gln Gln Gly Gln Asn Gln Leu Tyr Asn Glu Leu
           115                 120                 125
   Asn Leu Gly Arg Arg Glu Glu Tyr Asp Val Leu Asp Lys Arg Arg Gly
       130                 135                 140
   Arg Asp Pro Glu Met Gly Gly Lys Pro Arg Arg Lys Asn Pro Gln Glu
   145                 150                 155                 160
   Gly Leu Tyr Asn Glu Leu Gln Lys Asp Lys Met Ala Glu Ala Tyr Ser
                   165                 170                 175
   Glu Ile Gly Met Lys Gly Glu Arg Arg Arg Gly Lys Gly His Asp Gly
               180                 185                 190
   Leu Tyr Gln Gly Leu Ser Thr Ala Thr Lys Asp Thr Tyr Asp Ala Leu
           195                 200                 205
   His Met Gln Ala Leu Pro Pro Arg
       210                 215
<210> 13
   <211> 3390
   <212> DNA
   <213> Artificial Sequence
<220>
   <223> MP13974.SFG.aCD19fmc63-CD8STK-CD28tmZ-2A-aCD33glx-HCH2CH3pvaa-CD2 8tmZw
<400> 13
   atgagcctgc ccgtgaccgc cctgctgctg cccctggccc tgctgctgca cgccgccaga      60
   ccagacatcc agatgaccca gaccaccagc agcctgagcg ccagcctggg cgaccgggtg     120
   accatcagct gcagagccag ccaggacatc agcaagtacc tgaactggta ccagcagaag     180
   cccgacggca ccgtgaagct gctgatctac cacaccagcc ggctgcacag cggcgtgccc     240
   agccggttca gcggcagcgg cagcggcacc gactacagcc tgaccatcag caacctggag     300
   caggaggaca tcgccaccta cttctgccag cagggcaaca ccctgcccta caccttcgga     360
   ggcggcacca agctggagat caccaaggcc ggaggcggag gctctggcgg aggcggctct     420
   ggcggaggcg gctctggcgg aggcggcagc gaggtgaagc tgcaggagtc tggcccaggc     480
   ctggtggccc caagccagag cctgagcgtg acctgcaccg tgagcggcgt gagcctgccc     540
   gactacggcg tgagctggat caggcagccc ccacggaagg gcctggagtg gctgggcgtg     600
   atctggggca gcgagaccac ctactacaac agcgccctga agagccggct gaccatcatc     660
   aaggacaaca gcaagagcca ggtgttcctg aagatgaaca gcctgcagac cgacgacacc     720
   gccatctact actgcgccaa gcactactac tatggcggca gctacgctat ggactactgg     780
   ggccagggca ccagcgtgac cgtgagctca gatcccacca cgacgccagc gccgcgacca     840
   ccaacaccgg cgcccaccat cgcgtcgcag cccctgtccc tgcgcccaga ggcgtgccgg     900
   ccagcggcgg ggggcgcagt gcacacgagg gggctggact tcgcctgtga tatcttttgg     960
   gtgctggtgg tggttggtgg agtcctggct tgctatagct tgctagtaac agtggccttt    1020
   attattttct gggtgaggag agtgaagttc agcaggagcg cagacgcccc cgcgtaccag    1080
   cagggccaga accagctcta taacgagctc aatctaggac gaagagagga gtacgatgtt 1140
   ttggacaaga gacgtggccg ggaccctgag atggggggaa agccgagaag gaagaaccct 1200
   caggaaggcc tgtacaatga actgcagaaa gataagatgg cggaggccta cagtgagatt 1260
   gggatgaaag gcgagcgccg gaggggcaag gggcacgatg gcctttacca gggtctcagt 1320
   acagccacca aggacaccta cgacgccctt cacatgcagg ccctgcctcc tcgcagagcc 1380
   gagggcaggg gaagtcttct aacatgcggg gacgtggagg aaaatcccgg gcccatggcc 1440
   gtgcccactc aggtcctggg gttgttgcta ctgtggctta cagatgccag atgtgacatc 1500
   cagatgacac agtctccatc ttccctgtct gcatctgtcg gagatcgcgt caccatcacc 1560
   tgtcgagcaa gtgaggacat ttattttaat ttagtgtggt atcagcagaa accaggaaag 1620
   gcccctaagc tcctgatcta tgatacaaat cgcttggcag atggggtccc atcacggttc 1680
   agtggctctg gatctggcac acagtatact ctaaccataa gtagcctgca acccgaagat 1740
   ttcgcaacct attattgtca acactataag aattatccgc tcacgttcgg tcaggggacc 1800
   aagctggaaa tcaaaagatc tggtggcgga gggtcaggag gcggaggcag cggaggcggt 1860
   ggctcgggag gcggaggctc gagatctgag gtgcagttgg tggagtctgg gggcggcttg 1920
   gtgcagcctg gagggtccct gaggctctcc tgtgcagcct caggattcac tctcagtaat 1980
   tatggcatgc actggatcag gcaggctcca gggaagggtc tggagtgggt ctcgtctatt 2040
   agtcttaatg gtggtagcac ttactatcga gactccgtga agggccgatt cactatctcc 2100
   agggacaatg caaaaagcac cctctacctt caaatgaata gtctgagggc cgaggacacg 2160
   gccgtctatt actgtgcagc acaggacgct tatacgggag gttactttga ttactggggc 2220
   caaggaacgc tggtcacagt ctcgtctatg gatcccgccg agcccaaatc tcctgacaaa 2280
   actcacacat gcccaccgtg cccagcacct cccgtggccg gcccgtcagt cttcctcttc 2340
   cccccaaaac ccaaggacac cctcatgatc gcccggaccc ctgaggtcac atgcgtggtg 2400
   gtggacgtga gccacgaaga ccctgaggtc aagttcaact ggtacgtgga cggcgtggag 2460
   gtgcataatg ccaagacaaa gccgcgggag gagcagtaca acagcacgta ccgtgtggtc 2520
   agcgtcctca ccgtcctgca ccaggactgg ctgaatggca aggagtacaa gtgcaaggtc 2580
   tccaacaaag ccctcccagc ccccatcgag aaaaccatct ccaaagccaa agggcagccc 2640
   cgagaaccac aggtgtacac cctgccccca tcccgggatg agctgaccaa gaaccaggtc 2700
   agcctgacct gcctggtcaa aggcttctat cccagcgaca tcgccgtgga gtgggagagc 2760
   aatgggcaac cggagaacaa ctacaagacc acgcctcccg tgctggactc cgacggctcc 2820
   ttcttcctct acagcaagct caccgtggac aagagcaggt ggcagcaggg gaacgtcttc 2880
   tcatgctccg tgatgcatga ggccctgcac aatcactata cccagaaatc tctgagtctg 2940
   agcccaggca agaaggaccc caagttctgg gtcctggtgg tggtgggagg cgtgctggcc 3000
   tgttactctc tcctggtgac cgtggccttc atcatctttt gggtgcgctc ccgggtgaag 3060
   ttttctcgct ctgccgatgc cccagcctat cagcagggcc agaatcagct gtacaatgaa 3120
   ctgaacctgg gcaggcggga ggagtacgac gtgctggata agcggagagg cagagacccc 3180
   gagatgggcg gcaaaccacg gcgcaaaaat ccccaggagg gactctataa cgagctgcag 3240
   aaggacaaaa tggccgaggc ctattccgag atcggcatga agggagagag aagacgcgga 3300
   aagggccacg acggcctgta tcagggattg tccaccgcta caaaagatac atatgatgcc 3360
   ctgcacatgc aggccctgcc acccagatga 3390
<210> 14
   <211> 8
   <212> PRT
   <213> Artificial Sequence
<220>
   <223> cleavage site
<400> 14
   Asp Ile Glu Thr Asn Pro Gly Pro
   1               5
<210> 15
   <211> 8
   <212> PRT
   <213> Artificial Sequence
<220>
   <223> cleavage site
<400> 15
   Asp Val Glu Thr Asn Pro Gly Pro
   1               5
<210> 16
   <211> 8
   <212> PRT
   <213> Artificial Sequence
<220>
   <223> cleavage site
<400> 16
   Asp Val Glu Met Asn Pro Gly Pro
   1               5
<210> 17
   <211> 8
   <212> PRT
   <213> Artificial Sequence
<220>
   <223> cleavage site
<400> 17
   Asp Val Glu Ser Asn Pro Gly Pro
   1 5
<210> 18
   <211> 8
   <212> PRT
   <213> Artificial Sequence
<220>
   <223> cleavage site
<400> 18
   Asp Met Glu Ser Asn Pro Gly Pro
   1 5
<210> 19
   <211> 8
   <212> PRT
   <213> Artificial Sequence
<220>
   <223> cleavage site
<400> 19
   Asp Val Glu Leu Asn Pro Gly Pro
   1 5
<210> 20
   <211> 8
   <212> PRT
   <213> Artificial Sequence
<220>
   <223> cleavage site
<400> 20
   Asp Val Glu Glu Asn Pro Gly Pro
   1 5
<210> 21
   <211> 8
   <212> PRT
   <213> Artificial Sequence
<220>
   <223> cleavage site
<400> 21
   Asp Ile Glu Leu Asn Pro Gly Pro
   1 5
<210> 22
   <211> 8
   <212> PRT
   <213> Artificial Sequence
<220>
   <223> cleavage site
<400> 22
   Asp Ile Glu Gln Asn Pro Gly Pro
   1 5
<210> 23
   <211> 8
   <212> PRT
   <213> Artificial Sequence
<220>
   <223> cleavage site
<400> 23
   Asp Ser Glu Phe Asn Pro Gly Pro
   1 5
<210> 24
   <211> 19
   <212> PRT
   <213> Artificial Sequence
<220>
   <223> cleavage sequence
<400> 24
   Leu Leu Asn Phe Asp Leu Leu Lys Leu Ala Gly Asp Val Glu Ser Asn
   1 5 10 15
   Pro Gly Pro
<210> 25
   <211> 19
   <212> PRT
   <213> Artificial Sequence
<220>
   <223> cleavage sequence
<400> 25
   Leu Leu Asn Phe Asp Leu Leu Lys Leu Ala Gly Asp Val Gln Ser Asn
   1 5 10 15
   Pro Gly Pro
<210> 26
   <211> 19
   <212> PRT
   <213> Artificial Sequence
<220>
   <223> cleavage sequence
<400> 26
   Leu Leu Asn Phe Asp Leu Leu Lys Leu Ala Gly Asp Val Glu Ile Asn
   1 5 10 15
   Pro Gly Pro
<210> 27
   <211> 19
   <212> PRT
   <213> Artificial Sequence
<220>
   <223> cleavage sequence
<400> 27
   Leu Leu Asn Phe Asp Leu Leu Lys Leu Ala Gly Asp Val Glu Phe Asn
   1 5 10 15
   Pro Gly Pro
<210> 28
   <211> 19
   <212> PRT
   <213> Artificial Sequence
<220>
   <223> cleavage sequence
<400> 28
   Leu Leu Asn Phe Asp Leu Leu Lys Leu Ala Gly Asp Val Glu Ser His
   1 5 10 15
   Pro Gly Pro
<210> 29
   <211> 19
   <212> PRT
   <213> Artificial Sequence
<220>
   <223> cleavage sequence
<400> 29
   Leu Leu Asn Phe Asp Leu Leu Lys Leu Ala Gly Asp Val Glu Ser Glu
   1               5                   10                  15
   Pro Gly Pro
<210> 30
   <211> 19
   <212> PRT
   <213> Artificial Sequence
<220>
   <223> cleavage sequence
<400> 30
   Leu Leu Asn Phe Asp Leu Leu Lys Leu Ala Gly Asp Val Glu Ser Gln
   1               5                   10                  15
   Pro Gly Pro
<210> 31
   <211> 19
   <212> PRT
   <213> Artificial Sequence
<220>
   <223> cleavage sequence
<400> 31
   Leu Leu Asn Phe Asp Leu Leu Lys Leu Ala Gly Asp Val Glu Ser Asn
   1               5                   10                  15
   Pro Gly Gly
<210> 32
   <211> 20
   <212> PRT
   <213> Artificial Sequence
<220>
   <223> 2A-like sequence
<400> 32
   Tyr His Ala Asp Tyr Tyr Lys Gln Arg Leu Ile His Asp Val Glu Met
   1               5                   10                  15
   Asn Pro Gly Pro
               20
<210> 33
   <211> 20
   <212> PRT
   <213> Artificial Sequence
<220>
   <223> 2A-like sequence
<400> 33
   His Tyr Ala Gly Tyr Phe Ala Asp Leu Leu Ile His Asp Ile Glu Thr
   1               5                   10                  15
   Asn Pro Gly Pro
               20
<210> 34
   <211> 20
   <212> PRT
   <213> Artificial Sequence
<220>
   <223> 2A-like sequence
<400> 34
   Gln Cys Thr Asn Tyr Ala Leu Leu Lys Leu Ala Gly Asp Val Glu Ser
   1               5                   10                  15
   Asn Pro Gly Pro
               20
<210> 35
   <211> 19
   <212> PRT
   <213> Artificial Sequence
<220>
   <223> 2A-like sequence
<400> 35
   Ala Thr Asn Phe Ser Leu Leu Lys Gln Ala Gly Asp Val Glu Glu Asn
   1               5                   10                  15
   Pro Gly Pro
<210> 36
   <211> 19
   <212> PRT
   <213> Artificial Sequence
<220>
   <223> 2A-like sequence
<400> 36
   Ala Ala Arg Gln Met Leu Leu Leu Leu Ser Gly Asp Val Glu Thr Asn
   1               5                   10                  15
   Pro Gly Pro
<210> 37
   <211> 20
   <212> PRT
   <213> Artificial Sequence
<220>
   <223> 2A-like sequence
<400> 37
   Arg Ala Glu Gly Arg Gly Ser Leu Leu Thr Cys Gly Asp Val Glu Glu
   1               5                   10                  15
   Asn Pro Gly Pro
               20
<210> 38
   <211> 20
   <212> PRT
   <213> Artificial Sequence
<220>
   <223> 2A-like sequence
<400> 38
   Thr Arg Ala Glu Ile Glu Asp Glu Leu Ile Arg Ala Gly Ile Glu Ser
   1               5                   10                  15
   Asn Pro Gly Pro
               20
<210> 39
   <211> 20
   <212> PRT
   <213> Artificial Sequence
<220>
   <223> 2A-like sequence
<400> 39
   Thr Arg Ala Glu Ile Glu Asp Glu Leu Ile Arg Ala Asp Ile Glu Ser
   1               5                   10                  15
   Asn Pro Gly Pro
               20
<210> 40
   <211> 20
   <212> PRT
   <213> Artificial Sequence
<220>
   <223> 2A-like sequence
<400> 40
   Ala Lys Phe Gln Ile Asp Lys Ile Leu Ile Ser Gly Asp Val Glu Leu
   1               5                   10                  15
   Asn Pro Gly Pro
               20
<210> 41
   <211> 20
   <212> PRT
   <213> Artificial Sequence
<220>
   <223> 2A-like sequence
<400> 41
   Ser Ser Ile Ile Arg Thr Lys Met Leu Val Ser Gly Asp Val Glu Glu
   1               5                   10                  15
   Asn Pro Gly Pro
               20
<210> 42
   <211> 20
   <212> PRT
   <213> Artificial Sequence
<220>
   <223> 2A-like sequence
<400> 42
   Cys Asp Ala Gln Arg Gln Lys Leu Leu Leu Ser Gly Asp Ile Glu Gln
   1               5                   10                  15
   Asn Pro Gly Pro
               20
<210> 43
   <211> 20
   <212> PRT
   <213> Artificial Sequence
<220>
   <223> 2A-like sequence
<400> 43
   Tyr Pro Ile Asp Phe Gly Gly Phe Leu Val Lys Ala Asp Ser Glu Phe
   1               5                   10                  15
   Asn Pro Gly Pro
               20
<210> 44
   <211> 57
   <212> PRT
   <213> Artificial Sequence
<220>
   <223> cleavage sequence
<400> 44
   Val Thr Glu Leu Leu Tyr Arg Met Lys Arg Ala Glu Thr Tyr Cys Pro
   1               5                   10                  15
   Arg Pro Leu Ala Ile His Pro Thr Glu Ala Arg His Lys Gln Lys Ile
               20                  25                  30
   Val Ala Pro Val Lys Gln Thr Leu Asn Phe Asp Leu Leu Lys Leu Ala
           35                  40                  45
   Gly Asp Val Glu Ser Asn Pro Gly Pro
       50                  55
<210> 45
   <211> 40
   <212> PRT
   <213> Artificial Sequence
<220>
   <223> cleavage sequence
<400> 45
   Leu Leu Ala Ile His Pro Thr Glu Ala Arg His Lys Gln Lys Ile Val
   1               5                   10                  15
   Ala Pro Val Lys Gln Thr Leu Asn Phe Asp Leu Leu Lys Leu Ala Gly
               20                  25                  30
   Asp Val Glu Ser Asn Pro Gly Pro
           35                  40
<210> 46
   <211> 33
   <212> PRT
   <213> Artificial Sequence
<220>
   <223> cleavage sequence
<400> 46
   Glu Ala Arg His Lys Gln Lys Ile Val Ala Pro Val Lys Gln Thr Leu
   1               5                   10                  15
   Asn Phe Asp Leu Leu Lys Leu Ala Gly Asp Val Glu Ser Asn Pro Gly
               20                  25                  30
   Pro
<210> 47
   <211> 24
   <212> PRT
   <213> Artificial Sequence
<220>
   <223> cleavage sequence
<400> 47
   Ala Pro Val Lys Gln Thr Leu Asn Phe Asp Leu Leu Lys Leu Ala Gly
   1               5                   10                  15
   Asp Val Glu Ser Asn Pro Gly Pro
               20
<210> 48
   <211> 136
   <212> PRT
   <213> Artificial Sequence
<220>
   <223> marker/suicide polypeptide
<400> 48
   Cys Pro Tyr Ser Asn Pro Ser Leu Cys Ser Gly Gly Gly Gly Ser Glu
   1               5                   10                  15
   Leu Pro Thr Gln Gly Thr Phe Ser Asn Val Ser Thr Asn Val Ser Pro
               20                  25                  30
   Ala Lys Pro Thr Thr Thr Ala Cys Pro Tyr Ser Asn Pro Ser Leu Cys
           35                  40                  45
   Ser Gly Gly Gly Gly Ser Pro Ala Pro Arg Pro Pro Thr Pro Ala Pro
       50                  55                  60
   Thr Ile Ala Ser Gln Pro Leu Ser Leu Arg Pro Glu Ala Cys Arg Pro
   65                  70                  75                  80
   Ala Ala Gly Gly Ala Val His Thr Arg Gly Leu Asp Phe Ala Cys Asp
                   85                  90                  95
   Ile Tyr Ile Trp Ala Pro Leu Ala Gly Thr Cys Gly Val Leu Leu Leu
               100                 105                 110
   Ser Leu Val Ile Thr Leu Tyr Cys Asn His Arg Asn Arg Arg Arg Val
           115                 120                 125
   Cys Lys Cys Pro Arg Pro Val Val
       130                 135
<210> 49
   <211> 4
   <212> PRT
   <213> Artificial Sequence
<220>
   <223> intracellular retention signal
<220>
   <221> misc_feature
   <222> (4)..(4)
   <223> Xaa can be any naturally occurring amino acid
<400> 49
   Asn Pro Phe Xaa
   1
<210> 50
   <211> 4
   <212> PRT
   <213> Artificial Sequence
<220>
   <223> intracellular retention signal
<400> 50
   Lys Asp Glu Leu
   1
<210> 51
   <211> 4
   <212> PRT
   <213> Artificial Sequence
<220>
   <223> intracellular retention signal
<220>
   <221> misc_feature
   <222> (3)..(4)
   <223> Xaa can be any naturally occurring amino acid
<400> 51
   Lys Lys Xaa Xaa
   1
<210> 52
   <211> 5
   <212> PRT
   <213> Artificial Sequence
<220>
   <223> intracellular retention signal
<220>
   <221> misc_feature
   <222> (2)..(2)
   <223> Xaa can be any naturally occurring amino acid
<220>
   <221> misc_feature
   <222> (4)..(5)
   <223> Xaa can be any naturally occurring amino acid
<400> 52
   Lys Xaa Lys Xaa Xaa
   1               5
<210> 53
   <211> 4
   <212> PRT
   <213> Artificial Sequence
<220>
   <223> intracellular retention signal
<220>
   <221> misc_feature
   <222> (3)..(3)
   <223> Xaa can be any naturally occurring amino acid
<400> 53
   Asn Pro Xaa Tyr
   1
<210> 54
   <211> 4
   <212> PRT
   <213> Artificial Sequence
<220>
   <223> intracellular retention signal
<220>
   <221> misc_feature
   <222> (2)..(3)
   <223> Xaa can be any naturally occurring amino acid
<220>
   <221> MISC_FEATURE
   <222> (4)..(4)
   <223> Xaa is an amino acid with a bulky hydrophobic side chain
<400> 54
   Tyr Xaa Xaa Xaa
   1
<210> 55
   <211> 8
   <212> PRT
   <213> Artificial Sequence
<220>
   <223> intracellular retention signal
<220>
   <221> misc_feature
   <222> (3)..(5)
   <223> Xaa can be any naturally occurring amino acid
<400> 55
   Asp Glu Xaa Xaa Xaa Leu Leu Ile
   1               5
<210> 56
   <211> 5
   <212> PRT
   <213> Artificial Sequence
<220>
   <223> intracellular retention signal
<220>
   <221> misc_feature
   <222> (2)..(3)
   <223> Xaa can be any naturally occurring amino acid
<400> 56
   Asp Xaa Xaa Leu Leu
   1               5
<210> 57
   <211> 4
   <212> PRT
   <213> Artificial Sequence
<220>
   <223> intracellular retention signal
<400> 57
   Asp Pro Phe Trp
   1
<210> 58
   <211> 5
   <212> PRT
   <213> Artificial Sequence
<220>
   <223> intracellular retention signal
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 58
   Phe Xaa Asp Xaa Phe
   1 5
<210> 59
   <211> 6
   <212> PRT
   <213> Artificial Sequence
<220>
   <223> intracellular retention signal
<220>
   <221> MISC_FEATURE
   <222> (2)..(2)
   <223> Xaa is an amino acid with a bulky hydrophobic side chain
<220>
   <221> misc_feature
   <222> (3)..(3)
   <223> Xaa can be any naturally occurring amino acid
<220>
   <221> MISC_FEATURE
   <222> (4)..(4)
   <223> Xaa is an amino acid with a bulky hydrophobic side chain
<400> 59
   Leu Xaa Xaa Xaa Asp Glu
   1 5
<210> 60
   <211> 5
   <212> PRT
   <213> Artificial Sequence
<220>
   <223> intracellular retention signal
<400> 60
   Leu Leu Asp Leu Leu
   1 5
<210> 61
   <211> 5
   <212> PRT
   <213> Artificial Sequence
<220>
   <223> intracellular retention signal
<400> 61
   Pro Trp Asp Leu Trp
   1 5
<210> 62
   <211> 20
   <212> PRT
   <213> Mus musculus
<400> 62
   Met Glu Thr Asp Thr Leu Ile Leu Trp Val Leu Leu Leu Leu Val Pro
   1 5 10 15
   Gly Ser Thr Gly
   20
<210> 63
   <211> 21
   <212> PRT
   <213> Artificial Sequence
<220>
   <223> signal peptide derived from Human CD8a
<400> 63
   Met Ser Leu Pro Val Thr Ala Leu Leu Leu Pro Leu Ala Leu Leu Leu
   1 5 10 15
   His Ala Ala Arg Pro
   20
<210> 64
   <211> 248
   <212> PRT
   <213> Artificial Sequence
<220>
   <223> scFv aCD19
<400> 64
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Lys Tyr
               20                  25                  30
   Leu Asn Trp Tyr Gln Gln Lys Pro Asp Gly Thr Val Lys Leu Leu Ile
           35                  40                  45
   Tyr His Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Tyr
                   85                  90                  95
   Thr Phe Gly Gly Gly Thr Lys Leu Glu Ile Thr Lys Ala Gly Gly Gly
               100                 105                 110
   Gly Ser Gly Gly Gly Gly Ser Gly Gly Gly Gly Ser Gly Gly Gly Gly
           115                 120                 125
   Ser Glu Val Lys Leu Gln Glu Ser Gly Pro Gly Leu Val Ala Pro Ser
       130                 135                 140
   Gln Ser Leu Ser Val Thr Cys Thr Val Ser Gly Val Ser Leu Pro Asp
   145                 150                 155                 160
   Tyr Gly Val Ser Trp Ile Arg Gln Pro Pro Arg Lys Gly Leu Glu Trp
                   165                 170                 175
   Leu Gly Val Ile Trp Gly Ser Glu Thr Thr Tyr Tyr Asn Ser Ala Leu
               180                 185                 190
   Lys Ser Arg Leu Thr Ile Ile Lys Asp Asn Ser Lys Ser Gln Val Phe
           195                 200                 205
   Leu Lys Met Asn Ser Leu Gln Thr Asp Asp Thr Ala Ile Tyr Tyr Cys
           210                 215                 220
   Ala Lys His Tyr Tyr Tyr Gly Gly Ser Tyr Ala Met Asp Tyr Trp Gly
   225                 230                 235                 240
   Gln Gly Thr Ser Val Thr Val Ser
                   245
<210> 65
   <211> 47
   <212> PRT
   <213> Homo sapiens
<400> 65
   Pro Thr Thr Thr Pro Ala Pro Arg Pro Pro Thr Pro Ala Pro Thr Ile
   1               5                   10                  15
   Ala Ser Gln Pro Leu Ser Leu Arg Pro Glu Ala Cys Arg Pro Ala Ala
               20                  25                  30
   Gly Gly Ala Val His Thr Arg Gly Leu Asp Phe Ala Cys Asp Ile
           35                  40                  45
<210> 66
   <211> 27
   <212> PRT
   <213> Homo sapiens
<400> 66
   Phe Trp Val Leu Val Val Val Gly Gly Val Leu Ala Cys Tyr Ser Leu
   1               5                   10                  15
   Leu Val Thr Val Ala Phe Ile Ile Phe Trp Val
               20                  25
<210> 67
   <211> 113
   <212> PRT
   <213> Homo sapiens
<400> 67
   Arg Arg Val Lys Phe Ser Arg Ser Ala Asp Ala Pro Ala Tyr Gln Gln
   1               5                   10                  15
   Gly Gln Asn Gln Leu Tyr Asn Glu Leu Asn Leu Gly Arg Arg Glu Glu
               20                  25                  30
   Tyr Asp Val Leu Asp Lys Arg Arg Gly Arg Asp Pro Glu Met Gly Gly
           35                  40                  45
   Lys Pro Arg Arg Lys Asn Pro Gln Glu Gly Leu Tyr Asn Glu Leu Gln
       50                  55                  60
   Lys Asp Lys Met Ala Glu Ala Tyr Ser Glu Ile Gly Met Lys Gly Glu
   65                  70                  75                  80
   Arg Arg Arg Gly Lys Gly His Asp Gly Leu Tyr Gln Gly Leu Ser Thr
                   85                  90                  95
   Ala Thr Lys Asp Thr Tyr Asp Ala Leu His Met Gln Ala Leu Pro Pro
               100                 105                 110
   Arg
<210> 68
   <211> 20
   <212> PRT
   <213> Artificial Sequence
<220>
   <223> 2A peptide
<400> 68
   Arg Ala Glu Gly Arg Gly Ser Leu Leu Thr Cys Gly Asp Val Glu Glu
   1               5                   10                  15
   Asn Pro Gly Pro
               20
<210> 69
   <211> 18
   <212> PRT
   <213> Artificial Sequence
<220>
   <223> signal peptide derived from mouse Ig kappa
<400> 69
   Met Ala Val Pro Thr Gln Val Leu Gly Leu Leu Leu Leu Trp Leu Thr
   1               5                   10                  15
   Asp Ala
<210> 70
   <211> 254
   <212> PRT
   <213> Artificial Sequence
<220>
   <223> scFv aCD33
<400> 70
   Arg Cys Asp Ile Gln Met Thr Gln Ser Pro Ser Ser Leu Ser Ala Ser
   1               5                   10                  15
   Val Gly Asp Arg Val Thr Ile Thr Cys Arg Ala Ser Glu Asp Ile Tyr
               20                  25                  30
   Phe Asn Leu Val Trp Tyr Gln Gln Lys Pro Gly Lys Ala Pro Lys Leu
           35                  40                  45
   Leu Ile Tyr Asp Thr Asn Arg Leu Ala Asp Gly Val Pro Ser Arg Phe
       50                  55                  60
   Ser Gly Ser Gly Ser Gly Thr Gln Tyr Thr Leu Thr Ile Ser Ser Leu
   65                  70                  75                  80
   Gln Pro Glu Asp Phe Ala Thr Tyr Tyr Cys Gln His Tyr Lys Asn Tyr
                   85                  90                  95
   Pro Leu Thr Phe Gly Gln Gly Thr Lys Leu Glu Ile Lys Arg Ser Gly
               100                 105                 110
   Gly Gly Gly Ser Gly Gly Gly Gly Ser Gly Gly Gly Gly Ser Gly Gly
           115                 120                 125
   Gly Gly Ser Arg Ser Glu Val Gln Leu Val Glu Ser Gly Gly Gly Leu
       130                 135                 140
   Val Gln Pro Gly Gly Ser Leu Arg Leu Ser Cys Ala Ala Ser Gly Phe
   145                 150                 155                 160
   Thr Leu Ser Asn Tyr Gly Met His Trp Ile Arg Gln Ala Pro Gly Lys
                   165                 170                 175
   Gly Leu Glu Trp Val Ser Ser Ile Ser Leu Asn Gly Gly Ser Thr Tyr
               180                 185                 190
   Tyr Arg Asp Ser Val Lys Gly Arg Phe Thr Ile Ser Arg Asp Asn Ala
           195                 200                 205
   Lys Ser Thr Leu Tyr Leu Gln Met Asn Ser Leu Arg Ala Glu Asp Thr
       210                 215                 220
   Ala Val Tyr Tyr Cys Ala Ala Gln Asp Ala Tyr Thr Gly Gly Tyr Phe
   225                 230                 235                 240
   Asp Tyr Trp Gly Gln Gly Thr Leu Val Thr Val Ser Ser Met
                   245                 250
<210> 71
   <211> 231
   <212> PRT
   <213> Artificial Sequence
<220>
   <223> hinge and Fc derived from human IgG1
<400> 71
   Glu Pro Lys Ser Pro Asp Lys Thr His Thr Cys Pro Pro Cys Pro Ala
   1               5                   10                  15
   Pro Pro Val Ala Gly Pro Ser Val Phe Leu Phe Pro Pro Lys Pro Lys
               20                  25                  30
   Asp Thr Leu Met Ile Ala Arg Thr Pro Glu Val Thr Cys Val Val Val
           35                  40                  45
   Asp Val Ser His Glu Asp Pro Glu Val Lys Phe Asn Trp Tyr Val Asp
       50                  55                  60
   Gly Val Glu Val His Asn Ala Lys Thr Lys Pro Arg Glu Glu Gln Tyr
   65                  70                  75                  80
   Asn Ser Thr Tyr Arg Val Val Ser Val Leu Thr Val Leu His Gln Asp
                   85                  90                  95
   Trp Leu Asn Gly Lys Glu Tyr Lys Cys Lys Val Ser Asn Lys Ala Leu
               100                 105                 110
   Pro Ala Pro Ile Glu Lys Thr Ile Ser Lys Ala Lys Gly Gln Pro Arg
           115                 120                 125
   Glu Pro Gln Val Tyr Thr Leu Pro Pro Ser Arg Asp Glu Leu Thr Lys
       130                 135                 140
   Asn Gln Val Ser Leu Thr Cys Leu Val Lys Gly Phe Tyr Pro Ser Asp
   145                 150                 155                 160
   Ile Ala Val Glu Trp Glu Ser Asn Gly Gln Pro Glu Asn Asn Tyr Lys
                   165                 170                 175
   Thr Thr Pro Pro Val Leu Asp Ser Asp Gly Ser Phe Phe Leu Tyr Ser
               180                 185                 190
   Lys Leu Thr Val Asp Lys Ser Arg Trp Gln Gln Gly Asn Val Phe Ser
           195                 200                 205
   Cys Ser Val Met His Glu Ala Leu His Asn His Tyr Thr Gln Lys Ser
       210                 215                 220
   Leu Ser Leu Ser Pro Gly Lys
   225                 230
<210> 72
   <211> 4
   <212> PRT
   <213> Artificial Sequence
<220>
   <223> linker
<400> 72
   Lys Asp Pro Lys
   1
<210> 73
   <211> 27
   <212> PRT
   <213> Homo sapiens
<400> 73
   Phe Trp Val Leu Val Val Val Gly Gly Val Leu Ala Cys Tyr Ser Leu
   1               5                   10                  15
   Leu Val Thr Val Ala Phe Ile Ile Phe Trp Val
               20                  25
<210> 74
   <211> 112
   <212> PRT
   <213> Homo sapiens
<400> 74
   Arg Val Lys Phe Ser Arg Ser Ala Asp Ala Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 75
   <211> 36
   <212> PRT
   <213> Artificial Sequence
<220>
   <223> nucleic acid construct comprising a tyrp-1 retention protein sequence
<400> 75
   Arg Ala Arg Arg Ser Met Asp Glu Ala Asn Gln Pro Leu Leu Thr Asp
   1               5                   10                  15
   Gln Tyr Gln Cys Tyr Ala Glu Glu Tyr Glu Lys Leu Gln Asn Pro Asn
               20                  25                  30
   Gln Ser Val Val
           35
<210> 76
   <211> 4
   <212> PRT
   <213> Mus musculus
<400> 76
   Met Glu Thr Asp
   1
<210> 77
   <211> 11
   <212> PRT
   <213> Mus musculus
<400> 77
   Thr Leu Ile Leu Trp Val Leu Leu Leu Leu Val
   1               5                   10
<210> 78
   <211> 5
   <212> PRT
   <213> Mus musculus
<400> 78
   Pro Gly Ser Thr Gly
   1               5
<210> 79
   <211> 25
   <212> PRT
   <213> Homo sapiens
<400> 79
   Met Asn Arg Gly Val Pro Phe Arg His Leu Leu Leu Val Leu Gln Leu
   1               5                   10                  15
   Ala Leu Leu Pro Ala Ala Thr Gln Gly
               20                  25
<210> 80
   <211> 23
   <212> PRT
   <213> Homo sapiens
<400> 80
   Met Tyr Leu Trp Leu Lys Leu Leu Ala Phe Gly Phe Ala Phe Leu Asp
   1               5                   10                  15
   Thr Glu Val Phe Val Thr Gly
               20
<210> 81
   <211> 21
   <212> PRT
   <213> Homo sapiens
<400> 81
   Met Ala Leu Pro Val Thr Ala Leu Leu Leu Pro Leu Ala Leu Leu Leu
   1               5                   10                  15
   His Ala Ala Arg Pro
               20
<210> 82
   <211> 21
   <212> PRT
   <213> Homo sapiens
<400> 82
   Met Arg Pro Arg Leu Trp Leu Leu Leu Ala Ala Gln Leu Thr Val Leu
   1               5                   10                  15
   His Gly Asn Ser Val
        20
<210> 83
   <211> 24
   <212> PRT
   <213> Homo sapiens
<400> 83
   Met Ser Phe Pro Cys Lys Phe Val Ala Ser Phe Leu Leu Ile Phe Asn
   1               5                   10                  15
   Val Ser Ser Lys Gly Ala Val Ser
               20
<210> 84
   <211> 24
   <212> PRT
   <213> Homo sapiens
<400> 84
   Met Pro Met Gly Ser Leu Gln Pro Leu Ala Thr Leu Tyr Leu Leu Gly
   1               5                   10                  15
   Met Leu Val Ala Ser Cys Leu Gly
               20
<210> 85
   <211> 25
   <212> PRT
   <213> Homo sapiens
<400> 85
   Met Ala Gly Pro Pro Arg Leu Leu Leu Leu Pro Leu Leu Leu Ala Leu
   1               5                   10                  15
   Ala Arg Gly Leu Pro Gly Ala Leu Ala
               20                  25
<210> 86
   <211> 24
   <212> PRT
   <213> Homo sapiens
<400> 86
   Met Ser Ala Pro Lys Leu Leu Ser Leu Gly Cys Ile Phe Phe Pro Leu
   1               5                   10                  15
   Leu Leu Phe Gln Gln Ala Arg Ala
               20
<210> 87
   <211> 19
   <212> PRT
   <213> Homo sapiens
<400> 87
   Met Arg Phe Phe Val Pro Leu Phe Leu Val Gly Ile Leu Phe Pro Ala
   1               5                   10                  15
   Ile Leu Ala
<210> 88
   <211> 23
   <212> PRT
   <213> Homo sapiens
<400> 88
   Met Pro Gly Phe Leu Val Arg Ile Leu Pro Leu Leu Leu Val Leu Leu
   1               5                   10                  15
   Leu Leu Gly Pro Thr Arg Gly
               20
<210> 89
   <211> 24
   <212> PRT
   <213> Homo sapiens
<400> 89
   Met Lys Arg Phe Leu Phe Leu Leu Leu Thr Ile Ser Leu Leu Val Met
   1 5 10 15
   Val Gln Ile Gln Thr Gly Leu Ser
   20
<210> 90
   <211> 21
   <212> PRT
   <213> Homo sapiens
<400> 90
   Met Gln Gly Pro Pro Leu Leu Thr Ala Ala His Leu Leu Cys Val Cys
   1 5 10 15
   Thr Ala Ala Leu Ala
   20
<210> 91
   <211> 22
   <212> PRT
   <213> Homo sapiens
<400> 91
   Met Gly Pro Thr Ser Gly Pro Ser Leu Leu Leu Leu Leu Leu Thr His
   1 5 10 15
   Leu Pro Leu Ala Leu Gly
   20
<210> 92
   <211> 18
   <212> PRT
   <213> Homo sapiens
<400> 92
   Met Gln Pro Ile Leu Leu Leu Leu Ala Phe Leu Leu Leu Pro Arg Ala
   1 5 10 15
   Asp Ala
<210> 93
   <211> 20
   <212> PRT
   <213> Homo sapiens
<400> 93
   Met Glu Ala Pro Ala Gln Leu Leu Phe Leu Leu Leu Leu Trp Leu Pro
   1               5                   10                  15
   Asp Thr Thr Arg
               20
<210> 94
   <211> 20
   <212> PRT
   <213> Homo sapiens
<400> 94
   Met Val Leu Gln Thr Gln Val Phe Ile Ser Leu Leu Leu Trp Ile Ser
   1               5                   10                  15
   Gly Ala Tyr Gly
               20
<210> 95
   <211> 19
   <212> PRT
   <213> Homo sapiens
<400> 95
   Met Ala Trp Ala Pro Leu Leu Leu Thr Leu Leu Ala His Cys Thr Asp
   1               5                   10                  15
   Cys Trp Ala
<210> 96
   <211> 22
   <212> PRT
   <213> Homo sapiens
<400> 96
   Met Leu Lys Pro Ser Leu Pro Phe Thr Ser Leu Leu Phe Leu Gln Leu
   1               5                   10                  15
   Pro Leu Leu Gly Val Gly
               20
<210> 97
   <211> 20
   <212> PRT
   <213> Homo sapiens
<400> 97
   Met Ala Trp Ala Ser Arg Leu Gly Leu Leu Leu Ala Leu Leu Leu Pro
   1               5                   10                  15
   Val Val Gly Ala
               20
<210> 98
   <211> 23
   <212> PRT
   <213> Homo sapiens
<400> 98
   Met Gly Val Pro Arg Pro Gln Pro Trp Ala Leu Gly Leu Leu Leu Phe
   1               5                   10                  15
   Leu Leu Pro Gly Ser Leu Gly
               20
<210> 99
   <211> 4
   <212> PRT
   <213> Artificial Sequence
<220>
   <223> ITAM motif
<220>
   <221> misc_feature
   <222> (2)..(3)
   <223> Xaa can be any naturally occurring amino acid
<220>
   <221> MISC_FEATURE
   <222> (4)..(4)
   <223> Xaa may be Leu or Ile
<400> 99
   Tyr Xaa Xaa Xaa
   1
<210> 100
   <211> 4
   <212> PRT
   <213> Artificial Sequence
<220>
   <223> basic amino acid furin target sequence
<220>
   <221> misc_feature
   <222> (2)..(2)
   <223> Xaa can be any naturally occurring amino acid
<220>
   <221> MISC_FEATURE
   <222> (3)..(3)
   <223> Xaa may be Arg or Lys
<400> 100
   Arg Xaa Xaa Arg
   1
<210> 101
   <211> 7
   <212> PRT
   <213> Artificial Sequence
<220>
   <223> consensus Tobacco Etch Virus (TEV) cleavage site
<400> 101
   Glu Asn Leu Tyr Phe Gln Ser
   1 5
<210> 102
   <211> 8
   <212> PRT
   <213> Artificial Sequence
<220>
   <223> motif involved in cleavage activity
<220>
   <221> misc_feature
   <222> (2)..(2)
   <223> Xaa can be any naturally occurring amino acid
<220>
   <221> misc_feature
   <222> (4)..(4)
   <223> Xaa can be any naturally occurring amino acid
<400> 102
   Asp Xaa Glu Xaa Asn Pro Gly Pro
   1 5
<210> 103
   <211> 8
   <212> PRT
   <213> Artificial Sequence
<220>
   <223> cleavage site
<220>
   <221> MISC_FEATURE
   <222> (2)..(2)
   <223> Xaa may be Ile, Val, Met or Ser
<220>
   <221> MISC_FEATURE
   <222> (4)..(4)
   <223> Xaa may be Thr, Met, Ser, Leu, Glu, Gln or Phe
<400> 103
   Asp Xaa Glu Xaa Asn Pro Gly Pro
   1               5
<210> 104
   <211> 4
   <212> PRT
   <213> Artificial Sequence
<220>
   <223> C-terminal signal, motif of postassium channels
<220>
   <221> misc_feature
   <222> (2)..(2)
   <223> Xaa can be any naturally occurring amino acid
<400> 104
   Arg Xaa Arg Arg
   1
<210> 105
   <211> 12
   <212> PRT
   <213> Homo sapiens
<400> 105
   Ile Asn Phe Asp Asn Pro Val Tyr Gln Lys Thr Thr
   1               5                   10
<210> 106
   <211> 12
   <212> PRT
   <213> Homo sapiens
<400> 106
   Val Glu Ile Gly Asn Pro Thr Tyr Lys Met Tyr Glu
   1               5                   10
<210> 107
   <211> 12
   <212> PRT
   <213> Homo sapiens
<400> 107
   Thr Asn Phe Thr Asn Pro Val Tyr Ala Thr Leu Tyr
   1               5                   10
<210> 108
   <211> 12
   <212> PRT
   <213> Drosophila sp.
<400> 108
   Gly Asn Phe Ala Asn Pro Val Tyr Glu Ser Met Tyr
   1 5 10
<210> 109
   <211> 12
   <212> PRT
   <213> Caenorhabditis elegans
<400> 109
   Thr Thr Phe Thr Asn Pro Val Tyr Glu Leu Glu Asp
   1 5 10
<210> 110
   <211> 12
   <212> PRT
   <213> Caenorhabditis elegans
<400> 110
   Leu Arg Val Asp Asn Pro Leu Tyr Asp Pro Asp Ser
   1 5 10
<210> 111
   <211> 12
   <212> PRT
   <213> Homo sapiens
<400> 111
   Ile Ile Phe Glu Asn Pro Met Tyr Ser Ala Arg Asp
   1 5 10
<210> 112
   <211> 12
   <212> PRT
   <213> Homo sapiens
<400> 112
   Thr Asn Phe Glu Asn Pro Ile Tyr Ala Gln Met Glu
   1 5 10
<210> 113
   <211> 12
   <212> PRT
   <213> Homo sapiens
<400> 113
   Asp Thr Gly Glu Asn Pro Ile Tyr Lys Ser Ala Val
   1 5 10
<210> 114
   <211> 11
   <212> PRT
   <213> Homo sapiens
<400> 114
   Thr Thr Val Val Asn Pro Lys Tyr Glu Gly Lys
   1 5 10
<210> 115
   <211> 12
   <212> PRT
   <213> Drosophila sp.
<400> 115
   Trp Asp Thr Glu Asn Pro Ile Tyr Lys Gln Ala Thr
   1 5 10
<210> 116
   <211> 11
   <212> PRT
   <213> Drosophila sp.
<400> 116
   Ser Thr Phe Lys Asn Pro Met Tyr Ala Gly Lys
   1 5 10
<210> 117
   <211> 12
   <212> PRT
   <213> Homo sapiens
<400> 117
   His Gly Tyr Glu Asn Pro Thr Tyr Arg Phe Leu Glu
   1 5 10
<210> 118
   <211> 12
   <212> PRT
   <213> Homo sapiens
<400> 118
   Asn Gly Tyr Glu Asn Pro Thr Tyr Lys Phe Phe Glu
   1 5 10
<210> 119
   <211> 12
   <212> PRT
   <213> Drosophila sp.
<400> 119
   Asn Gly Tyr Glu Asn Pro Thr Tyr Lys Tyr Phe Glu
   1               5                   10
<210> 120
   <211> 12
   <212> PRT
   <213> Homo sapiens
<400> 120
   Tyr Ala Ser Ser Asn Pro Glu Tyr Leu Ser Ala Ser
   1               5                   10
<210> 121
   <211> 12
   <212> PRT
   <213> Homo sapiens
<400> 121
   Gly Ser Val Gln Asn Pro Val Tyr His Asn Gln Pro
   1               5                   10
<210> 122
   <211> 12
   <212> PRT
   <213> Homo sapiens
<400> 122
   Thr Ala Val Gly Asn Pro Glu Tyr Leu Asn Thr Val
   1               5                   10
<210> 123
   <211> 12
   <212> PRT
   <213> Homo sapiens
<400> 123
   Ile Ser Leu Asp Asn Pro Asp Tyr Gln Gln Asp Phe
   1               5                   10
<210> 124
   <211> 11
   <212> PRT
   <213> Homo sapiens
<400> 124
   Arg Lys Arg Ser His Ala Gly Tyr Gln Thr Ile
   1               5                   10
<210> 125
   <211> 10
   <212> PRT
   <213> Homo sapiens
<400> 125
   Lys His His His Ala Gly Tyr Glu Gln Phe
   1 5 10
<210> 126
   <211> 11
   <212> PRT
   <213> Gallus gallus
<400> 126
   Lys Lys His His Asn Thr Gly Tyr Glu Gln Phe
   1 5 10
<210> 127
   <211> 11
   <212> PRT
   <213> Gallus gallus
<400> 127
   Arg Arg Lys Ser Arg Thr Gly Tyr Gln Ser Val
   1 5 10
<210> 128
   <211> 11
   <212> PRT
   <213> Gallus gallus
<400> 128
   Arg Arg Lys Ser Tyr Ala Gly Tyr Gln Thr Leu
   1 5 10
<210> 129
   <211> 12
   <212> PRT
   <213> Drosophila sp.
<400> 129
   Arg Arg Arg Ser Thr Ser Arg Gly Tyr Met Ser Phe
   1 5 10
<210> 130
   <211> 11
   <212> PRT
   <213> Lumbricus terrestris
<400> 130
   Arg Lys Arg Ser Arg Arg Gly Tyr Glu Ser Val
   1 5 10
<210> 131
   <211> 10
   <212> PRT
   <213> Homo sapiens
<400> 131
   Lys Ser Ile Arg Ser Gly Tyr Glu Val Met
   1 5 10
<210> 132
   <211> 12
   <212> PRT
   <213> Homo sapiens
<400> 132
   His Cys Gly Gly Pro Arg Pro Gly Tyr Glu Thr Leu
   1 5 10
<210> 133
   <211> 12
   <212> PRT
   <213> Mus musculus
<400> 133
   His Cys Arg Thr Arg Arg Ala Glu Tyr Glu Thr Leu
   1 5 10
<210> 134
   <211> 10
   <212> PRT
   <213> Homo sapiens
<400> 134
   Arg Arg Arg Pro Ser Ala Tyr Gln Ala Leu
   1 5 10
<210> 135
   <211> 9
   <212> PRT
   <213> Homo sapiens
<400> 135
   Arg Arg Arg Ser Tyr Gln Asn Ile Pro
   1 5
<210> 136
   <211> 10
   <212> PRT
   <213> Homo sapiens
<400> 136
   Lys Lys His Cys Ser Tyr Gln Asp Ile Leu
   1 5 10
<210> 137
   <211> 10
   <212> PRT
   <213> Mus musculus
<400> 137
   Arg Arg Arg Ser Ala Tyr Gln Asp Ile Arg
   1 5 10
<210> 138
   <211> 11
   <212> PRT
   <213> Rattus norvegicus
<400> 138
   Arg Lys Arg Arg Arg Ser Tyr Gln Asp Ile Met
   1 5 10
<210> 139
   <211> 13
   <212> PRT
   <213> Rattus norvegicus
<400> 139
   Lys Phe Cys Lys Ser Lys Glu Arg Asn Tyr His Thr Leu
   1 5 10
<210> 140
   <211> 14
   <212> PRT
   <213> Drosophila sp.
<400> 140
   Lys Phe Tyr Lys Ala Arg Asn Glu Arg Asn Tyr His Thr Leu
   1 5 10
<210> 141
   <211> 14
   <212> PRT
   <213> Homo sapiens
<400> 141
   Lys Ile Arg Leu Arg Cys Gln Ser Ser Gly Tyr Gln Arg Ile
   1 5 10
<210> 142
   <211> 14
   <212> PRT
   <213> Mus musculus
<400> 142
   Lys Ile Arg Gln Arg His Gln Ser Ser Ala Tyr Gln Arg Ile
   1               5                   10
<210> 143
   <211> 15
   <212> PRT
   <213> Homo sapiens
<400> 143
   His Phe Cys Leu Tyr Arg Lys Arg Pro Gly Tyr Asp Gln Leu Asn
   1               5                   10                  15
<210> 144
   <211> 11
   <212> PRT
   <213> Homo sapiens
<400> 144
   Ser Leu Ser Arg Gly Ser Gly Tyr Lys Glu Ile
   1               5                   10
<210> 145
   <211> 13
   <212> PRT
   <213> Homo sapiens
<400> 145
   Arg Arg Leu Arg Lys Gly Tyr Thr Pro Leu Met Glu Thr
   1               5                   10
<210> 146
   <211> 14
   <212> PRT
   <213> Homo sapiens
<400> 146
   Arg Arg Ala Gly His Ser Ser Tyr Thr Pro Leu Pro Gly Ser
   1               5                   10
<210> 147
   <211> 18
   <212> PRT
   <213> Dictyostelium sp.
<400> 147
   Lys Lys Leu Arg Gln Gln Lys Gln Gln Gly Tyr Gln Ala Ile Ile Asn
   1               5                   10                  15
   Asn Glu
<210> 148
   <211> 15
   <212> PRT
   <213> Dictyostelium sp.
<400> 148
   Arg Ser Lys Ser Asn Gln Asn Gln Ser Tyr Asn Leu Ile Gln Leu
   1 5 10 15
<210> 149
   <211> 17
   <212> PRT
   <213> Dictyostelium sp.
<400> 149
   Arg Lys Thr Phe Tyr Asn Asn Asn Gln Tyr Asn Gly Tyr Asn Ile Ile
   1 5 10 15
   Asn
<210> 150
   <211> 10
   <212> PRT
   <213> Homo sapiens
<400> 150
   Pro Leu Ser Tyr Thr Arg Phe Ser Leu Ala
   1 5 10
<210> 151
   <211> 11
   <212> PRT
   <213> Homo sapiens
<400> 151
   Met Thr Lys Glu Tyr Gln Asp Leu Gln His Leu
   1 5 10
<210> 152
   <211> 10
   <212> PRT
   <213> Homo sapiens
<400> 152
   Ser Tyr Lys Tyr Ser Lys Val Asn Lys Glu
   1 5 10
<210> 153
   <211> 10
   <212> PRT
   <213> Homo sapiens
<400> 153
   Pro Ala Ala Tyr Arg Gly Val Gly Asp Asp
   1 5 10
<210> 154
   <211> 10
   <212> PRT
   <213> Homo sapiens
<400> 154
   Thr Gly Val Tyr Val Lys Met Pro Pro Thr
   1 5 10
<210> 155
   <211> 10
   <212> PRT
   <213> Homo sapiens
<400> 155
   Leu Ile Ser Tyr Lys Gly Leu Pro Pro Glu
   1 5 10
<210> 156
   <211> 11
   <212> PRT
   <213> Rattus norvegicus
<400> 156
   Ala Ser Asp Tyr Gln Arg Leu Asn Leu Lys Leu
   1 5 10
<210> 157
   <211> 11
   <212> PRT
   <213> Human immunodeficiency virus type 1
<400> 157
   Arg Gln Gly Tyr Ser Pro Leu Ser Phe Gln Thr
   1 5 10
<210> 158
   <211> 19
   <212> PRT
   <213> Homo sapiens
<400> 158
   Arg Met Gln Ala Gln Pro Pro Gly Tyr Arg His Val Ala Asp Gly Glu
   1 5 10 15
   Asp His Ala
<210> 159
   <211> 7
   <212> PRT
   <213> Artificial Sequence
<220>
   <223> Dileucine-based sorting signal
<220>
   <221> MISC_FEATURE
   <222> (1)..(1)
   <223> Xaa may be Asp or Glu
<220>
   <221> misc_feature
   <222> (2)..(4)
   <223> Xaa can be any naturally occurring amino acid
<220>
   <221> MISC_FEATURE
   <222> (7)..(7)
   <223> Xaa may be Leu or Ile
<400> 159
   Xaa Xaa Xaa Xaa Leu Leu Xaa
   1 5
<210> 160
   <211> 9
   <212> PRT
   <213> Homo sapiens
<400> 160
   Ser Asp Lys Gln Thr Leu Leu Pro Asn
   1 5
<210> 161
   <211> 9
   <212> PRT
   <213> Rattus norvegicus
<400> 161
   Asp Glu Arg Ala Pro Leu Ile Arg Thr
   1 5
<210> 162
   <211> 9
   <212> PRT
   <213> Homo sapiens
<400> 162
   Gln Glu Lys Asp Pro Leu Leu Lys Asn
   1 5
<210> 163
   <211> 9
   <212> PRT
   <213> Coturnix sp.
<400> 163
   Thr Glu Arg Asn Pro Leu Leu Lys Ser
   1 5
<210> 164
   <211> 9
   <212> PRT
   <213> Homo sapiens
<400> 164
   Gly Glu Asn Ser Pro Leu Leu Ser Gly
   1 5
<210> 165
   <211> 9
   <212> PRT
   <213> Homo sapiens
<400> 165
   Glu Glu Lys Gln Pro Leu Leu Met Glu
   1 5
<210> 166
   <211> 9
   <212> PRT
   <213> Oryzias latipes
<400> 166
   Gly Glu Arg Gln Pro Leu Leu Gln Ser
   1 5
<210> 167
   <211> 9
   <212> PRT
   <213> Gallus gallus
<400> 167
   Pro Glu Ile Gln Pro Leu Leu Thr Glu
   1 5
<210> 168
   <211> 9
   <212> PRT
   <213> Carassius auratus
<400> 168
   Glu Gly Arg Gln Pro Leu Leu Gly Asp
   1 5
<210> 169
   <211> 9
   <212> PRT
   <213> Homo sapiens
<400> 169
   Glu Ala Asn Gln Pro Leu Leu Thr Asp
   1 5
<210> 170
   <211> 9
   <212> PRT
   <213> Gallus gallus
<400> 170
   Glu Leu His Gln Pro Leu Leu Thr Asp
   1 5
<210> 171
   <211> 9
   <212> PRT
   <213> Danio rerio
<400> 171
   Arg Glu Phe Glu Pro Leu Leu Asn Ala
   1 5
<210> 172
   <211> 9
   <212> PRT
   <213> Homo sapiens
<400> 172
   Glu Glu Lys Met Ala Ile Leu Met Asp
   1 5
<210> 173
   <211> 9
   <212> PRT
   <213> Homo sapiens
<400> 173
   Glu Glu Lys Leu Ala Ile Leu Ser Gln
   1 5
<210> 174
   <211> 9
   <212> PRT
   <213> Mus musculus
<400> 174
   Ser Glu Arg Asp Val Leu Leu Asp Glu
   1 5
<210> 175
   <211> 9
   <212> PRT
   <213> Homo sapiens
<400> 175
   Ser Glu Arg Arg Asn Leu Leu Glu Asp
   1 5
<210> 176
   <211> 9
   <212> PRT
   <213> Rattus norvegicus
<400> 176
   Asp Asp Ser Gly Asp Leu Leu Pro Gly
   1 5
<210> 177
   <211> 9
   <212> PRT
   <213> Homo sapiens
<400> 177
   Ser Gln Ile Lys Arg Leu Leu Ser Glu
   1 5
<210> 178
   <211> 9
   <212> PRT
   <213> Felis catus
<400> 178
   Ser His Ile Lys Arg Leu Leu Ser Glu
   1 5
<210> 179
   <211> 9
   <212> PRT
   <213> Mus musculus
<400> 179
   Arg Arg Thr Pro Ser Leu Leu Glu Gln
   1 5
<210> 180
   <211> 9
   <212> PRT
   <213> Homo sapiens
<400> 180
   His Arg Thr Pro Ser Leu Leu Glu Gln
   1               5
<210> 181
   <211> 10
   <212> PRT
   <213> Rattus norvegicus
<400> 181
   Glu Pro Arg Gly Ser Arg Leu Leu Val Arg
   1               5                   10
<210> 182
   <211> 19
   <212> PRT
   <213> Homo sapiens
<400> 182
   Met Asp Asp Gln Arg Asp Leu Ile Ser Asn Asn Glu Gln Leu Pro Met
   1               5                   10                  15
   Leu Gly Arg
<210> 183
   <211> 19
   <212> PRT
   <213> Mus musculus
<400> 183
   Met Asp Asp Gln Arg Asp Leu Ile Ser Asn His Glu Gln Leu Pro Ile
   1               5                   10                  15
   Leu Gly Asn
<210> 184
   <211> 20
   <212> PRT
   <213> Gallus gallus
<400> 184
   Met Ala Glu Glu Gln Arg Asp Leu Ile Ser Ser Asp Gly Ser Ser Gly
   1               5                   10                  15
   Val Leu Pro Ile
               20
<210> 185
   <211> 23
   <212> PRT
   <213> Danio rerio
<400> 185
   Met Glu Pro Asp His Gln Asn Glu Ser Leu Ile Gln Arg Val Pro Ser
   1               5                   10                  15
   Ala Glu Thr Ile Leu Gly Arg
               20
<210> 186
   <211> 23
   <212> PRT
   <213> Danio rerio
<400> 186
   Met Ser Ser Glu Gly Asn Glu Thr Pro Leu Ile Ser Asp Gln Ser Ser
   1               5                   10                  15
   Val Asn Met Gly Pro Gln Pro
               20
<210> 187
   <211> 24
   <212> PRT
   <213> Trypanosoma sp.
<400> 187
   Arg Pro Arg Arg Arg Thr Glu Glu Asp Glu Leu Leu Pro Glu Glu Ala
   1               5                   10                  15
   Glu Gly Leu Ile Asp Pro Gln Asn
               20
<210> 188
   <211> 19
   <212> PRT
   <213> Homo sapiens
<400> 188
   Pro Asp Lys His Ser Leu Leu Val Gly Asp Phe Arg Glu Asp Asp Asp
   1 5 10 15
   Thr Ala Leu
<210> 189
   <211> 9
   <212> PRT
   <213> Homo sapiens
<400> 189
   Thr Glu Arg Glu Arg Leu Leu Asn Phe
   1 5
<210> 190
   <211> 8
   <212> PRT
   <213> Homo sapiens
<400> 190
   Val Glu Thr Asp Asp Leu Ile Leu
   1 5
<210> 191
   <211> 7
   <212> PRT
   <213> Caenorhabditis elegans
<400> 191
   Phe Glu Asn Asp Ser Leu Leu
   1 5
<210> 192
   <211> 9
   <212> PRT
   <213> Saccharomyces cerevisiae
<400> 192
   Asn Glu Gln Ser Pro Leu Leu His Asn
   1 5
<210> 193
   <211> 8
   <212> PRT
   <213> Saccharomyces cerevisiae
<400> 193
   Ser Glu Gln Thr Arg Leu Val Pro
   1 5
<210> 194
   <211> 8
   <212> PRT
   <213> Saccharomyces cerevisiae
<400> 194
   Glu Val Asp Leu Asp Leu Leu Lys
   1 5
<210> 195
   <211> 13
   <212> PRT
   <213> Homo sapiens
<400> 195
   Ser Phe His Asp Asp Ser Asp Glu Asp Leu Leu His Ile
   1 5 10
<210> 196
   <211> 13
   <212> PRT
   <213> Bos taurus
<400> 196
   Thr Phe His Asp Asp Ser Asp Glu Asp Leu Leu His Val
   1 5 10
<210> 197
   <211> 13
   <212> PRT
   <213> Oryctolagus cuniculus
<400> 197
   Ser Phe His Asp Asp Ser Asp Glu Asp Leu Leu Asn Ile
   1 5 10
<210> 198
   <211> 13
   <212> PRT
   <213> Gallus gallus
<400> 198
   Ser Phe His Asp Asp Ser Asp Glu Asp Leu Leu Asn Val
   1 5 10
<210> 199
   <211> 13
   <212> PRT
   <213> Homo sapiens
<400> 199
   Glu Glu Ser Glu Glu Arg Asp Asp His Leu Leu Pro Met
   1 5 10
<210> 200
   <211> 13
   <212> PRT
   <213> Gallus gallus
<400> 200
   Asp Glu Ser Glu Glu Arg Asp Asp His Leu Leu Pro Met
   1               5                   10
<210> 201
   <211> 12
   <212> PRT
   <213> Homo sapiens
<400> 201
   Gly Tyr His Asp Asp Ser Asp Glu Asp Leu Leu Glu
   1               5                   10
<210> 202
   <211> 13
   <212> PRT
   <213> Homo sapiens
<400> 202
   Ile Thr Gly Phe Ser Asp Asp Val Pro Met Val Ile Ala
   1               5                   10
<210> 203
   <211> 13
   <212> PRT
   <213> Hydra sp.
<400> 203
   Ile Asn Arg Phe Ser Asp Asp Glu Pro Leu Val Val Ala
   1               5                   10
<210> 204
   <211> 13
   <212> PRT
   <213> Homo sapiens
<400> 204
   Met Leu Glu Ala Ser Asp Asp Glu Ala Leu Leu Val Cys
   1               5                   10
<210> 205
   <211> 13
   <212> PRT
   <213> Homo sapiens
<400> 205
   Lys Asn Glu Thr Ser Asp Asp Glu Ala Leu Leu Leu Cys
   1 5 10
<210> 206
   <211> 12
   <212> PRT
   <213> Mus musculus
<400> 206
   Trp Val Val Glu Ala Glu Asp Glu Pro Leu Leu Ala
   1 5 10
<210> 207
   <211> 12
   <212> PRT
   <213> Homo sapiens
<400> 207
   Trp Val Ala Glu Ala Glu Asp Glu Pro Leu Leu Thr
   1 5 10
<210> 208
   <211> 12
   <212> PRT
   <213> Homo sapiens
<400> 208
   His Asp Asp Phe Ala Asp Asp Ile Ser Leu Leu Lys
   1 5 10
<210> 209
   <211> 13
   <212> PRT
   <213> Mus musculus
<400> 209
   Gly Arg Asp Ser Pro Glu Asp His Ser Leu Leu Val Asn
   1 5 10
<210> 210
   <211> 12
   <212> PRT
   <213> Peromyscus maniculatus
<400> 210
   Val Arg Cys His Pro Glu Asp Asp Arg Leu Leu Gly
   1 5 10
<210> 211
   <211> 13
   <212> PRT
   <213> Homo sapiens
<400> 211
   His Arg Val Ser Gln Asp Asp Leu Asp Leu Leu Thr Ser
   1 5 10
<210> 212
   <211> 13
   <212> PRT
   <213> Homo sapiens
<400> 212
   Ala Ser Val Ser Leu Leu Asp Asp Glu Leu Met Ser Leu
   1 5 10
<210> 213
   <211> 13
   <212> PRT
   <213> Homo sapiens
<400> 213
   Ala Ser Ser Gly Leu Asp Asp Leu Asp Leu Leu Gly Lys
   1 5 10
<210> 214
   <211> 13
   <212> PRT
   <213> Homo sapiens
<400> 214
   Val Gln Asn Pro Ser Ala Asp Arg Asn Leu Leu Asp Leu
   1 5 10
<210> 215
   <211> 13
   <212> PRT
   <213> Homo sapiens
<400> 215
   Asn Ala Leu Ser Trp Leu Asp Glu Glu Leu Leu Cys Leu
   1 5 10
<210> 216
   <211> 13
   <212> PRT
   <213> Drosophila sp.
<400> 216
   Thr Val Asp Ser Ile Asp Asp Val Pro Leu Leu Ser Asp
   1 5 10
<210> 217
   <211> 13
   <212> PRT
   <213> Mus musculus
<400> 217
   Gln Glu Glu Cys Pro Ser Asp Ser Glu Glu Asp Glu Gly
   1 5 10
<210> 218
   <211> 13
   <212> PRT
   <213> Mus musculus
<400> 218
   Arg Asp Arg Asp Tyr Asp Glu Asp Asp Glu Asp Asp Ile
   1 5 10
<210> 219
   <211> 15
   <212> PRT
   <213> Mus musculus
<400> 219
   Leu Asp Glu Thr Glu Asp Asp Glu Leu Glu Tyr Asp Asp Glu Ser
   1 5 10 15
<210> 220
   <211> 10
   <212> PRT
   <213> Homo sapiens
<400> 220
   Lys Asp Pro Asp Glu Val Glu Thr Glu Ser
   1 5 10
<210> 221
   <211> 13
   <212> PRT
   <213> Homo sapiens
<400> 221
   His Glu Phe Gln Asp Glu Thr Asp Thr Glu Glu Glu Thr
   1 5 10
<210> 222
   <211> 14
   <212> PRT
   <213> Homo sapiens
<400> 222
   Gln Glu Lys Glu Asp Asp Gly Ser Glu Ser Glu Glu Glu Tyr
   1 5 10
<210> 223
   <211> 9
   <212> PRT
   <213> Homo sapiens
<400> 223
   Gly Glu Asp Glu Glu Ser Glu Ser Asp
   1 5
<210> 224
   <211> 12
   <212> PRT
   <213> Homo sapiens
<400> 224
   Gly Glu Asp Ser Asp Glu Glu Pro Asp His Glu Glu
   1 5 10
<210> 225
   <211> 11
   <212> PRT
   <213> Homo sapiens
<400> 225
   Leu Glu Asp Asp Ser Asp Glu Glu Glu Asp Phe
   1 5 10
<210> 226
   <211> 9
   <212> PRT
   <213> Human cytomegalovirus
<400> 226
   Lys Asp Ser Asp Glu Glu Glu Asn Val
   1 5
<210> 227
   <211> 13
   <212> PRT
   <213> Herpesvirus 3
<400> 227
   Phe Glu Asp Ser Glu Ser Thr Asp Thr Glu Glu Glu Phe
   1 5 10
<210> 228
   <211> 9
   <212> PRT
   <213> Human immunodeficiency virus type 1
<400> 228
   Leu Glu Ala Gln Glu Glu Glu Glu Val
   1 5
<210> 229
   <211> 22
   <212> PRT
   <213> Saccharomyces cerevisiae
<400> 229
   Ala Asp Asp Leu Glu Ser Gly Leu Gly Ala Glu Asp Asp Leu Glu Gln
   1 5 10 15
   Asp Glu Gln Leu Glu Gly
   20
<210> 230
   <211> 12
   <212> PRT
   <213> Saccharomyces cerevisiae
<400> 230
   Thr Glu Ile Asp Glu Ser Phe Glu Met Thr Asp Phe
   1 5 10
<210> 231
   <211> 18
   <212> PRT
   <213> Saccharomyces cerevisiae
<400> 231
   Thr Glu Pro Glu Glu Val Glu Asp Phe Asp Phe Asp Leu Ser Asp Glu
   1 5 10 15
   Asp His
<210> 232
   <211> 15
   <212> PRT
   <213> Saccharomyces cerevisiae
<400> 232
   Phe Glu Ile Glu Glu Asp Asp Val Pro Thr Leu Glu Glu Glu His
   1 5 10 15
<210> 233
   <211> 4
   <212> PRT
   <213> Artificial Sequence
<220>
   <223> intracellular retention signal
<400> 233
   His Asp Glu Leu
   1